# EUROPÄISCHE PATENTSCHRIFT

(11) **EP 0 901 478 B1**
(45) Veröffentlichungstag und Bekanntmachung des Hinweises auf die Patenterteilung: **13.08.2003**
(21) Anmeldenummer: 97923858.1
(22) Anmeldetag: 09.05.1997
(51) Int. Cl.: C07D 249/12, C07D 401/06, C07D 405/06, A01N 43/653

(54) **THIOCYANO-TRIAZOLYL-DERIVATE UND IHRE VERWENDUNG ALS MIKROBIZIDE**
THIOCYANO-TRIAZOLYL DERIVATIVES AND THEIR USE AS MICROBICIDES
DERIVES DE THIOCYANO-TRIAZOLYLE ET LEUR UTILISATION COMME MICROBICIDES

(30) Priorität: 21.05.1996 DE 19620407
(43) Veröffentlichungstag der Anmeldung: 17.03.1999
(73) Patentinhaber: Bayer CropScience AG, 40789 Monheim (DE)
(72) Erfinder: JAUTELAT, Manfred, D-51399 Burscheid (DE); DUTZMANN, Stefan, D-40764 Langenfeld (DE); STENZEL, Klaus, D-40595 Düsseldorf (DE); HÄNSSLER, Gerd, D-51381 Leverkusen (DE)
(86) Internationale Anmeldenummer: EP9702373
(87) Internationale Veröffentlichungsnummer: WO97044331

(56) Entgegenhaltungen:
- EP-A- 0 251 086
- EP-A- 0 297 345
- WO-A-96/16048
- WO-A-96/38423
- WO-A-96/38424
- WO-A-96/38440
- WO-A-96/39395
- WO-A-96/41798
- WO-A-96/41804
- WO-A-97/05119
- WO-A-97/06151
- WO-A-97/06152

## Beschreibung

Die vorliegende Erfindung betrifft neue Thiocyano-triazolyl-Derivate, ein Verfahren zu deren Herstellung und deren Verwendung als Mikrobizide.

Es ist bereits bekannt geworden, daß zahlreiche Triazolyl-Derivate fungizide Eigenschaften besitzen (vgl. EP-A 0 015 756, EP-A 0 040 345, EP-A 0 052 424, EP-A 0 061 835, EP-A 0 297 345, EP-A 0 094 564, EP-A 0 196 038, EP-A 0 267 778, EP-A 0 378 953, EP-A 0 044 605, EP-A 0 069 442, EP-A 0 055 833, EP-A 0 301 393, DE-A 2 324 010, DE-A 2 737 489, DE-A 2 551 560, EP-A 0 065 485, DE-A 2 735 872, EP-A 0 234 242, DE-A 2 201 063, EP-A 0 145 294 und DE-A 3 721 786). Die Wirksamkeit dieser Stoffe ist gut, läßt aber bei niedrigen Aufwandmengen in manchen Fällen zu wünschen übrig.

Die EP-A 0 251 086 betrifft unter anderem fungizid wirksame Triazolyl-Derivate, in denen der Heterocyclus eine Thiocyano-Gruppe tragen kann. So werden Verbindungen dieses Typs offenbart, in denen einer der Substituenten am zentralen Kohlenstoffatom (dieses ist das Kohlenstoffatom, das mit den Resten-OR² und B verbunden ist) eine Alkenyl- oder Aralkenyl-Gruppe bedeutet.

Verbindungen, in denen diese Reste andere als die zuvor genannten Bedeutungen haben, werden aber nicht erwähnt.

Es wurden nun neue Thiocyano-triazolyl-Derivate der Formel 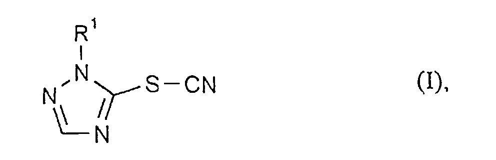 in welcher
- R¹: für einen Rest der Formel 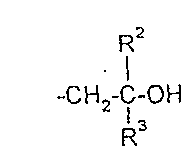 steht, in welcher
R² für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig
oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten, fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano, und
R³ für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten, fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,
oder
- R¹: für einen Rest der Formel 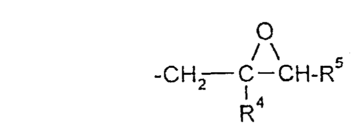 steht, in welcher
R⁴ für Methyl, Ethyl, n-Propyl, Isopropyl, n-Butyl, sek.- Butyl, iso-Butyl, tert.-Butyl, Fluor-tert.-butyl, Difluor-tert.-butyl, gegebenenfalls einfach bis dreifach, gleichartig oder verschieden durch Fluor, Chlor und/oder Brom substituiertes Cycloalkyl mit 3 bis 6 Kohlenstoffatomen, für Naphthyl oder für Phenyl steht, das einfach bis dreifach, gleichartig oder verschieden substituiert sein kann durch Fluor, Chlor, Brom, Nitro, Phenyl, Phenoxy, Methyl, Ethyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio, und
R⁵ für Phenyl steht, das einfach bis dreifach, gleichartig oder verschieden substituiert sein kann durch Fluor, Chlor, Brom, Methyl, Ethyl, Isopropyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio, oder

- R¹: für einen Rest der Formel 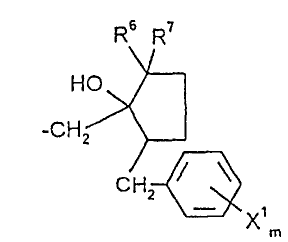 steht, in welcher
R⁶ für Wasserstoff, Methyl, Ethyl, n-Propyl, Isopropyl, n-Butyl, iso-Butyl, sek.-Butyl, tert.-Butyl oder n-Pentyl steht,
R⁷ für Wasserstoff, Methyl, Ethyl, n-Propyl, Isopropyl, n-Butyl, iso-Butyl, sek.-Butyl, tert.-Butyl oder n-Pentyl steht,
X¹ für Fluor, Chlor, Brom, Methyl, Ethyl, n-Propyl, Isopropyl, n-Butyl, iso-Butyl, sek.-Butyl, tert.-Butyl, Methoxy, Ethoxy, Phenyl, Phenoxy, Trichlormethyl, Trifluormethyl, Trifluormethoxy, Difluormethoxy oder Trifluormethylthio steht und
m für die Zahlen 0, 1 oder 2 steht, wobei X¹ für gleiche oder verschiedene Reste stehen kann, wenn m für 2 steht.
oder
- R¹: für einen Rest der Formel 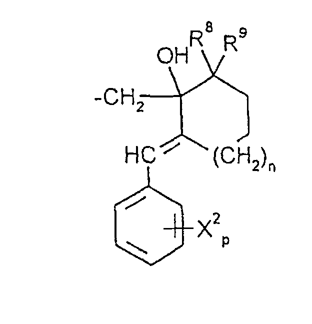 steht, in welcher
R⁸ für Wasserstoff, Methyl, Ethyl, n-Propyl, Isopropyl, n-Butyl, iso-Butyl, sek.-Butyl, tert.-Butyl oder n-Pentyl steht,
R⁹ für Wasserstoff, Methyl, Ethyl, n-Propyl, Isopropyl, n-Butyl, iso-Butyl, sek.-Butyl, tert.-Butyl oder n-Pentyl steht,
X² für Fluor, Chlor, Brom, Cyano, Nitro, Methyl, Ethyl, n-Propyl, Isopropyl, n-Butyl, iso-Butyl, sek.-Butyl, tert.-Butyl, Methoxy, Ethoxy, Trifluormethyl, Trichlormethyl, Difluormethyl, Trichlormethoxy, Trifluormethoxy, Difluormethoxy, Difluorchlormethoxy oder Phenyl steht,
n die Zahlen 0 oder 1 steht und
p für die Zahlen 0, 1 oder 2 steht,
wobei X² für gleiche oder verschiedene Reste stehen kann, wenn p für 2 steht,
oder
- R¹: für einen Rest der Formel 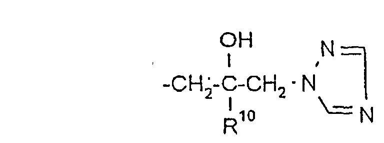 steht, in welcher
R¹⁰ für geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 5 Fluor-, Chlor- und/oder Brom-Atomen, für gegebenenfalls einfach bis dreifach, gleichartig oder verschieden durch Fluor, Chlor, Brom, Methyl und/oder Ethyl substituiertes Cycloalkyl mit 3 bis 6 Kohlenstoffatomen, für Phenyl, Benzyl oder Phenethyl steht, wobei jeder der drei zuletzt genannten Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen, Halogenalkylthio mit 1 bis 4 Kohlenstoffatomen und 1 bis 5 Halogenatomen, gegebenenfalls durch Halogen und/oder Alkyl mit 1 bis 4 Kohlenstoffatomen substituiertes Phenyl oder gegebenenfalls durch Halogen und/oder Alkyl mit 1 bis 4 Kohlenstoffatomen substituiertes Phenoxy,
oder
- R¹: für einen Rest der Formel 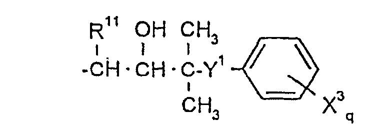 steht, in welcher
R¹¹ für Wasserstoff, geradkettiges oder verzweigtes Alkyl mit 1 bis 12 Kohlenstoffatomen oder für gegebenenfalls einfach bis dreifach, gleichartig oder verschieden durch Halogen und/oder Alkyl mit 1 bis 4 Kohlenstoffatomen substituiertes Cycloalkyl mit 3 bis 7 Kohlenstoffatomen steht,
X³ für Fluor, Chlor, Brom, Methyl, Ethyl, Methoxy, Methylthio, Trichlormethyl, Trifluormethyl, Trifluormethoxy, Difluormethoxy, Trifluormethylthio, Phenyl oder Phenoxy steht,
q für die Zahlen 0, 1, 2 oder 3 steht, wobei X³ für gleiche oder verschiedene Reste steht, wenn q für 2 oder 3 steht, und
Y¹ für ein Sauerstoffatom, eine CH₂-Gruppe oder eine direkte Bindung steht,
oder
- R¹: für einen Rest der Formel 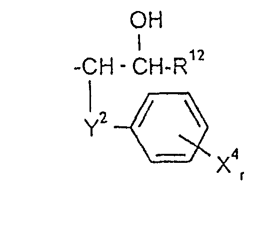 steht, in welcher
R¹² für geradkettiges oder verzweigtes Alkyl mit 1 bis 4 Kohlenstoffatomen, Fluoralkyl mit 1 bis 4 Kohlenstoffatomen und 1 bis 5 Fluoratomen, gegebenenfalls einfach bis dreifach, gleichartig oder verschieden durch Fluor, Chlor, Brom, Methyl und/oder Ethyl substituiertes Cycloalkyl mit 3 bis 6 Kohlenstoffatomen, Cycloalkylalkyl mit 3 bis 6 Kohlenstoffatomen im Cycloalkylteil und 1 bis 3 Kohlenstoffatomen im Alkylteil, gegebenenfalls einfach bis dreifach, gleichartig oder verschieden durch Fluor, Chlor und/oder Brom substituiertes Phenyl oder für gegebenenfalls einfach bis dreifach, gleichartig oder verschieden durch Fluor, Chlor und/oder Brom substituiertes Benzyl steht,
X⁴ für Fluor, Chlor, Brom, Nitro, Methyl, Ethyl, tert.-Butyl, Methoxy, Methylthio, Trichlormethyl, Trifluormethyl, Trifluormethoxy, Difluormethoxy, Trifluormethylthio, gegebenenfalls einfach oder zweifach, gleichartig oder verschieden durch Fluor, Chlor, Brom und/oder Methyl substituiertes Phenyl oder für gegebenenfalls einfach oder zweifach, gleichartig oder verschieden durch Fluor, Chlor, Brom und/oder Methyl substituiertes Phenoxy steht,
r für die Zahlen 0, 1, 2 oder 3 steht, wobei X⁴ für gleiche oder verschiedene Reste steht, wenn r für 2 oder 3 steht und
Y² für ein Sauerstoffatom oder für eine CH₂-Gruppe steht,
oder
- R¹: für einen Rest der Formel 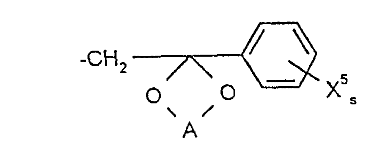 steht, in welcher
A für gegebenenfalls einfach bis dreifach, gleichartig oder verschieden durch Fluor, Chlor, Brom, Methyl, Ethyl, n-Propyl, Isopropyl, n-Butyl, i-Butyl, sek.-Butyl und/oder tert.-Butyl substituiertes Alkandiyl mit 2 oder 3 Kohlenstoffatomen steht,
X⁵ für Fluor, Chlor, Brom, Methyl, Ethyl, Methoxy, Methylthio, Trichlormethyl, Trifluormethyl, Trifluormethoxy, Trifluormethylthio, Difluormethoxy, gegebenenfalls einfach bis dreifach, gleichartig oder verschieden durch Fluor, Chlor und/oder Methyl substituiertes Phenyl und/oder für gegebenenfalls einfach bis dreifach, gleichartig oder verschieden durch Fluor, Chlor und/oder Methyl substituiertes Phenoxy steht und
s für die Zahlen 0, 1, 2 oder 3 steht, wobei X⁵ für gleiche oder verschiedene Reste steht, wenn s für 2 oder 3 steht,
oder
- R¹: für einen Rest der Formel 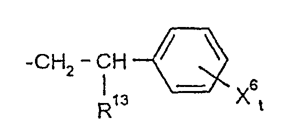 steht, in welcher
R¹³ für geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 5 Fluor-, Chlor- und/oder Bromatomen, Fluoralkoxyalkyl mit 1 bis 3 Kohlenstoffatomen und 1 bis 5 Fluoratomen im Fluoralkoxyteil und 1 bis 3 Kohlenstoffatomen im Alkylteil, gegebenenfalls einfach bis dreifach, gleichartig oder verschieden durch Fluor, Chlor, Brom, Methyl und/oder Ethyl substituiertes Cycloalkyl mit 3 bis 6 Kohlenstoffatomen, Cycloalkylalkyl mit 3 bis 6 Kohlenstoffatomen im Cycloalkylteil und 1 bis 3 Kohlenstoffatomen im Alkylteil, gegebenenfalls einfach bis dreifach, gleichartig oder verschieden durch Fluor, Chlor und/oder Brom substituiertes Phenyl oder für gegebenenfalls einfach bis dreifach, gleichartig oder verschieden durch Fluor, Chlor und/oder Brom substituiertes Phenylalkyl mit 1 oder 2 Kohlenstoffatomen im Alkylteil steht,
X⁶ für Fluor, Chlor, Brom, Methyl, Ethyl, tert.-Butyl, Methoxy, Methylthio, Trichlormethyl, Trifluormethyl, Trifluormethoxy, Difluormethoxy, Trifluormethylthio, gegebenenfalls einfach oder zweifach, gleichartig oder verschieden durch Fluor, Chlor, Brom und/oder Methyl substituiertes Phenyl oder für gegebenenfalls einfach oder zweifach, gleichartig oder verschieden durch Fluor, Chlor, Brom und/oder Methyl substituiertes Phenoxy steht und
t für die Zahlen 0, 1, 2 oder 3 steht, wobei X⁶ für gleiche oder verschiedene Reste steht, wenn t für 2 oder 3 steht,
oder
- R¹: für einen Rest der Formel 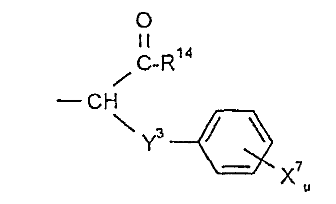 steht, in welcher
R¹⁴ für geradkettiges oder verzweigtes Alkyl mit 1 bis 4 Kohlenstoffatomen, Fluoralkyl mit 1 bis 4 kohlenstoffatomen und 1 bis 5 Fluoratomen, gegebenenfalls einfach bis dreifach, gleichartig oder verschieden durch Fluor, Chlor, Brom, Methyl und/oder Ethyl substituiertes Cycloalkyl mit 3 bis 6 Kohlenstoffatomen, Cycloalkylalkyl mit 3 bis 6 Kohlenstoffatomen im Cycloalkylteil und 1 bis 3 Kohlenstoffatomen im Alkylteil, gegebenenfalls einfach bis dreifach, gleichartig oder verschieden durch Fluor, Chlor und/oder Brom substituiertes Phenyl oder für gegebenenfalls einfach bis dreifach, gleichartig oder verschieden durch Fluor, Chlor und/oder Brom substituiertes Benzyl steht,
X⁷ für Fluor, Chlor, Brom, Methyl, Ethyl, tert.-Butyl, Methoxy, Methylthio, Trichlormethyl, Trifluormethyl, Trifluormethoxy, Difluormethoxy, Trifluormethylthio, gegebenenfalls einfach oder zweifach, gleichartig oder verschieden durch Fluor, Chlor, Brom und/oder Methyl substituiertes Phenyl oder für gegebenenfalls einfach oder zweifach, gleichartig oder verschieden durch Fluor, Chlor, Brom und/oder Methyl substituiertes Phenoxy steht,
u für die Zahlen 0, 1, 2 oder 3 steht, wobei X⁷ für gleiche oder verschiedene Reste steht, wenn u für 2 oder 3 steht, und
Y³ für ein Sauerstoffatom oder für eine CH₂-Gruppe steht,
oder
- R¹: für einen Rest der Formel 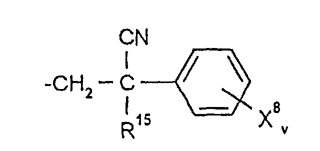 steht, in welcher
R¹⁵ für geradkettiges oder verzweigtes Alkyl mit 1 bis 4 Kohlenstoffatomen, Halogenalkyl mit 1 bis 4 Kohlenstoffatomen und 1 bis 5 Halogenatomen, gegebenenfalls einfach bis dreifach, gleichartig oder verschieden durch Halogen, Alkyl mit 1 bis 4 Kohlenstoffatomen, Halogenalkyl mit 1 oder 2 Kohlenstoffatomen mit 1 bis 5 Halogenatomen, Alkoxy mit 1 bis 4 Kohlenstoffatomen und/oder Halogenalkoxy mit 1 oder 2 Kohlenstoffatomen und 1 bis 5 Halogenatomen substituiertes Phenyl oder für gegebenenfalls einfach bis dreifach, gleichartig oder verschieden durch Halogen, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen und/oder Halogenalkoxy mit 1 oder 2 Kohlenstoffatomen und 1 bis 5 Halogenatomen substituiertes Phenylalkyl mit 1 bis 4 Kohlenstoffatomen im Alkylteil steht,
X⁸ für Fluor, Chlor, Brom, Methyl. Ethyl, tert.-Butyl, Methoxy, Methylthio, Trichlormethyl, Trifluormethyl, Trifluormethoxy, Difluormethoxy, Trifluormethylthio, gegebenenfalls einfach oder zweifach, gleichartig oder verschieden durch Fluor, Chlor, Brom und/oder Methyl substituiertes Phenyl oder für gegebenenfalls einfach oder zweifach, gleichartig oder verschieden durch Fluor, Chlor, Brom und/oder Methyl substituiertes Phenoxy steht und
v für die Zahlen 0, 1, 2 oder 3 steht, wobei X⁸ für gleiche oder verschiedene Reste steht, wenn v für 2 oder 3 steht, gefunden.

Zahlreiche der erfindungsgemäßen Stoffe enthalten eines oder mehrere asymmetrisch substituierte Kohlenstoffatome. Sie können daher in optischen Isomerenformen anfallen. Die vorliegende Erfindung betrifft sowohl die einzelnen Isomeren als auch deren Gemische.

Weiterhin wurde gefunden, daß man Thiocyano-triazolyl-Derivate der Formel (I) erhält, wenn man Mercapto-triazole der Formel 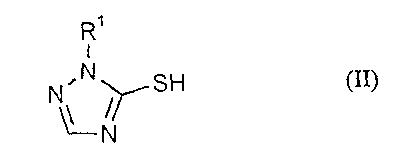 in welcher
- R¹: die oben angegebenen Bedeutungen hat,
mit Chlorcyan der Formel

Cl-CN (III)

in Gegenwart eines Verdünnungsmittels umsetzt.

Schließlich wurde gefunden, daß die neuen Thiocyano-triazolyl-Derivate der Formel (I) sehr gute mikrobizide Eigenschaften aufweisen und sich sowohl im Pflanzenschutz als auch im Materialschutz zur Bekämpfung unerwünschter Mikroorganismen verwenden lassen.

Überraschenderweise besitzen die erfindungsgemäßen Stoffe eine bessere mikrobizide Wirksamkeit, insbesondere fungizide Wirksamkeit, als die konstitutionell ähnlichsten, vorbekannten Verbindungen gleicher Wirkungsrichtung.

Die erfindungsgemäßen Thiocyano-triazolyl-Derivate sind durch die Formel (I) allgemein definiert.
- R¹: steht bevorzugt für einen Rest der Formel 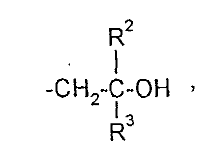 in welcher
R² bevorzugt für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl steht, wobei
jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl, und
R³ bevorzugt für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl.
- R¹: steht weiterhin bevorzugt für einen Rest der Formel 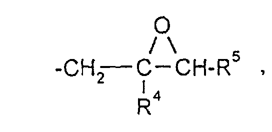 in welcher
R⁴ bevorzugt für Methyl, Ethyl, n-Propyl, Isopropyl, n-Butyl, sek.-Butyl, iso-Butyl, tert.-Butyl, Fluor-tert.-butyl, Difluor-tert.butyl, gegebenenfalls einfach bis dreifach, gleichartig oder verschieden durch Fluor, Chlor und/oder Brom substituiertes Cycloalkyl mit 3 bis 6 Kohlenstoffatomen, für Naphthyl oder für Phenyl steht, das einfach bis dreifach, gleichartig oder verschieden substituiert sein kann durch Fluor, Chlor, Brom, Nitro, Phenyl, Phenoxy, Methyl, Ethyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio, und
R⁵ bevorzugt für Phenyl steht, das einfach bis dreifach, gleichartig oder verschieden substituiert sein kann durch Fluor, Chlor, Brom, Methyl, Ethyl, Isopropyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio.
- R¹: steht weiterhin bevorzugt für einen Rest der Formel 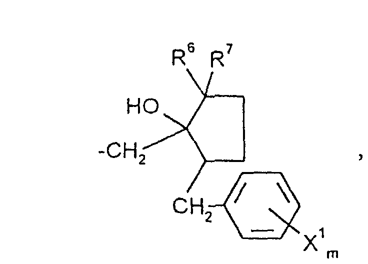 in welcher
R⁶ bevorzugt Wasserstoff, Methyl, Ethyl, n-Propyl, Isopropyl, n-Butyl, iso-Butyl, sek.-Butyl, tert.-Butyl oder n-Pentyl steht,
R⁷ bevorzugt für Wasserstoff, Methyl, Ethyl, n-Propyl, Isopropyl, n-Butyl, iso-Butyl, sek.-Butyl, tert.-Butyl oder n-Pentyl steht,
X¹ bevorzugt für Fluor, Chlor, Brom, Methyl, Ethyl, n-Propyl, Isopropyl, n-Butyl, iso-Butyl, sek.-Butyl, tert.-Butyl, Methoxy, Ethoxy, Phenyl, Phenoxy, Trichlormethyl, Trifluormethyl, Trifluormethoxy, Difluormethoxy oder Trifluormethylthio steht und
m auch bevorzugt für die Zahlen 0, 1 oder 2 steht,
wobei X¹ für gleiche oder verschiedene Reste stehen kann, wenn m für 2 steht.
- R¹: steht weiterhin bevorzugt für einen Rest der Formel 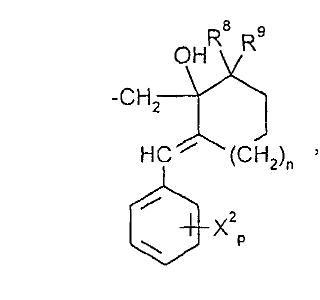 in welcher
R⁸ bevorzugt für Wasserstoff, Methyl, Ethyl, n-Propyl, Isopropyl, n-Butyl, iso-Butyl, sek.-Butyl, tert.-Butyl oder n-Pentyl steht,
R⁹ bevorzugt für Wasserstoff, Methyl, Ethyl, n-Propyl, Isopropyl, n-Butyl, iso-Butyl, sek.-Butyl, tert.-Butyl oder n-Pentyl steht,
X² bevorzugt für Fluor, Chlor, Brom, Cyano, Nitro, Methyl, Ethyl, n-Propyl, Isopropyl, n-Butyl, iso-Butyl, sek.-Butyl, tert.-Butyl, Methoxy, Ethoxy, Trifluormethyl, Trichlormethyl, Difluormethyl, Trichlormethoxy, Trifluormethoxy, Difluormethoxy, Difluorchlormethoxy oder Phenyl steht,
n auch bevorzugt für die Zahlen 0 oder 1 steht und
p auch bevorzugt für die Zahlen 0, 1 oder 2 steht,
wobei X² für gleiche oder verschiedene Reste stehen kann, wenn p für 2 steht.
- R¹: steht weiterhin bevorzugt für einen Rest der Formel 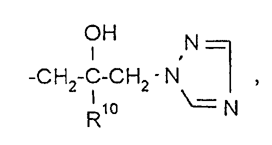 in welcher
R¹⁰ bevorzugt für Methyl, Ethyl, n-Propyl, Isopropyl, n-Butyl, sec.-Butyl, iso-Butyl, tert.-Butyl, Halogenalkyl mit 1 bis 4 Kohlenstoffatomen und 1 bis 3 Fluor-, Chlor- und/oder Brom-Atomen, gegebenenfalls einfach bis dreifach, gleichartig oder verschieden durch Fluor, Chlor, Brom, Methyl und/oder Ethyl substituiertes Cyclopropyl, Cyclopentyl oder Cyclohexyl, sowie für Phenyl, Benzyl oder Phenethyl steht, wobei jeder der drei zuletzt genannten Reste im Phenylteil einfach bis dreifach, gleichartig oder verschieden substituiert sein kann durch Fluor, Chlor, Brom, Methyl, Ethyl, Methoxy, Methylthio, Trichlormethyl, Trifluormethyl, Trifluormethoxy, Difluormethoxy, Trifluormethylthio, Phenyl und/oder Phenoxy.
- R¹: steht weiterhin bevorzugt für einen Rest der Formel 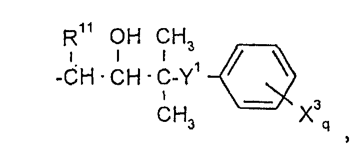 in welcher
R¹¹ bevorzugt für Wasserstoff, geradkettiges oder verzweigtes Alkyl mit 1 bis 6 Kohlenstoffatomen oder für gegebenenfalls einfach bis dreifach, gleichartig oder verschieden durch Fluor, Chlor, Brom, Methyl und/oder Ethyl substituiertes Cyclopropyl, Cyclopentyl oder Cyclohexyl steht,
X³ bevorzugt für Fluor, Chlor, Brom, Methyl, Methoxy, Methylthio, Trichlormethyl, Trifluormethyl, Trifluormethoxy, Difluormethoxy, Trifluormethylthio, Phenyl oder Phenoxy steht,
q auch bevorzugt für die Zahlen 0, 1, 2 oder 3 steht, wobei X³ für gleiche oder verschiedene Reste steht, wenn q für 2 oder 3 steht und
Y¹ auch bevorzugt für ein Sauerstoffatom, eine CH₂-Gruppe oder eine direkte Bindung steht.
- R¹: steht weiterhin bevorzugt für einen Rest der Formel 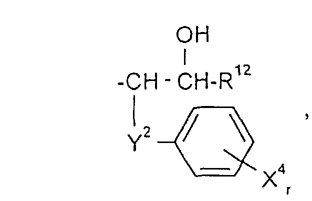 in welcher
R¹² bevorzugt für Methyl, Isopropyl, tert.-Butyl, Fluor-tert.-butyl, Difluor-tert.-butyl, gegebenenfalls einfach bis dreifach, gleichartig oder verschieden durch Fluor, Chlor, und/oder Methyl substituiertes Cyclopropyl, Cyclopentyl oder Cyclohexyl, für Cycloalkylalkyl mit 3 bis 6 Kohlenstoffatomen im Cycloalkylteil und 1 oder 2 Kohlenstoffatomen im Alkylteil, gegebenenfalls einfach oder zweifach, gleichartig oder verschieden durch Fluor, Chlor und/oder Brom substituiertes Phenyl oder für gegebenenfalls einfach oder zweifach, gleichartig oder verschieden durch Fluor, Chlor und/oder Brom substituiertes Benzyl steht,
X⁴ bevorzugt für Fluor, Chlor, Brom, Nitro, Methyl, Ethyl, tert.-Butyl, Methoxy, Methylthio, Trichlormethyl, Trifluormethyl, Trifluormethoxy, Difluormethoxy, Trifluormethylthio, Phenyl oder Phenoxy steht,
r auch bevorzugt für die Zahlen 0, 1, 2 oder 3 steht, wobei X⁴ für gleiche oder verschiedene Reste steht, wenn r für 2 oder 3 steht und
Y² auch bevorzugt für ein Sauerstoffatom oder für eine CH₂-Gruppe steht.
- R¹: steht weiterhin bevorzugt für einen Rest der Formel 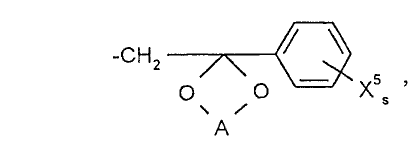 in welcher
A bevorzugt für gegebenenfalls einfach bis dreifach, gleichartig oder verschieden durch Fluor, Chlor, Brom, Methyl, Ethyl, n-Propyl, Isopropyl, n-Butyl, i-Butyl, sek.-Butyl und/oder tert.-Butyl substituiertes Alkandiyl mit 2 oder 3 Kohlenstoffatomen steht,
X⁵ bevorzugt für Fluor, Chlor, Brom, Methyl, Ethyl, Methoxy, Methylthio, Trichlormethyl, Trifluormethyl, Trifluormethoxy, Trifluormethylthio, Difluormethoxy, gegebenenfalls einfach bis dreifach, gleichartig oder verschieden durch Fluor, Chlor und/oder Methyl substituiertes Phenyl und/oder für gegebenenfalls einfach bis dreifach, gleichartig oder verschieden durch Fluor, Chlor und/oder Methyl substituiertes Phenoxy steht und
s auch bevorzugt für die Zahlen 0, 1, 2 oder 3 steht, wobei X⁵ für gleiche oder verschiedene Reste steht, wenn s für 2 oder 3 steht.
- R¹: steht weiterhin bevorzugt für einen Rest der Formel 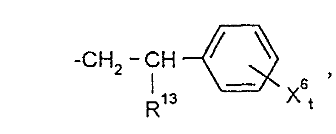 in welcher
R¹³ bevorzugt für Methyl, Ethyl, n-Propyl, Isopropyl, n-Butyl, iso-Butyl, sek.-Butyl, tert.-Butyl, Halogenalkyl mit 1 bis 4 Kohlenstoffatomen und 1 bis 5 Fluor-, Chlor- und/oder Bromatomen, Fluoralkoxyalkyl mit 1 oder 2 Kohlenstoffatomen und 1 bis 5 Fluoratomen im Fluoralkoxyteil und 1 oder 2 Kohlenstoffatomen im Alkylteil, gegebenenfalls einfach bis dreifach, gleichartig oder verschieden durch Fluor, Chlor, nd/oder Methyl substituiertes Cyclopropyl, Cyclopentyl oder Cyclohexyl, für Cycloalkylalkyl mit 3 bis 6 Kohlenstoffatomen im Cycloalkylteil und 1 oder 2 Kohlenstoffatomen im Alkylteil, gegebenenfalls einfach oder zweifach, gleichartig oder verschieden durch Fluor, Chlor und/oder Brom substituiertes Phenyl oder für gegebenenfalls einfach oder zweifach, gleichartig oder verschieden durch Fluor, Chlor und/oder Brom substituiertes Benzyl steht,
X⁶ bevorzugt für Fluor, Chlor, Brom, Methyl, Ethyl, tert.-Butyl, Methoxy, Methylthio, Trichlormethyl, Trifluormethyl, Trifluormethoxy, Difluormethoxy, Trifluormethylthio, Phenyl oder Phenoxy steht und
t auch bevorzugt für die Zahlen 0, 1, 2 oder 3 steht, wobei X⁶ für gleiche oder verschiedene Reste steht, wenn t für 2 oder 3 steht.
- R¹: steht weiterhin bevorzugt für einen Rest der Formel 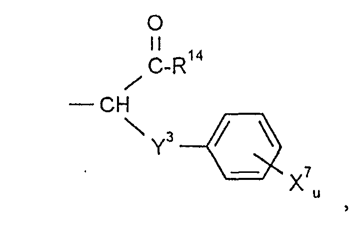 in welcher
R¹⁴ bevorzugt für Methyl, Isopropyl, tert.-Butyl, Fluor-tert.-butyl, Difluor-tert.-butyl, gegebenenfalls einfach bis dreifach, gleichartig oder verschieden durch Fluor, Chlor, und/oder Methyl substituiertes Cyclopropyl, Cyclopentyl oder Cyclohexyl, für Cycloalkylalkyl mit 3 bis 6 Kohlenstoffatomen im Cycloalkylteil und 1 oder 2 Kohlenstoffatomen im Alkylteil, gegebenenfalls einfach oder zweifach, gleichartig oder verschieden durch Fluor, Chlor und/oder Brom substituiertes Phenyl oder für gegebenenfalls einfach oder zweifach, gleichartig oder verschieden durch Fluor, Chlor und/oder Brom substituiertes Benzyl steht,
X⁷ bevorzugt für Fluor, Chlor, Brom, Methyl, Ethyl, tert.-Butyl, Methoxy, Methylthio, Trichlormethyl, Trifluormethyl, Trifluormethoxy, Difluormethoxy, Trifluormethylthio, Phenyl oder Phenoxy steht,
Y³ auch bevorzugt für ein Sauerstoffatom oder für eine CH₂-Gruppe steht, und
u auch bevorzugt für die Zahlen 0, 1, 2 oder 3 steht, wobei X⁷ für gleiche oder verschiedene Reste steht, wenn u für 2 oder 3 steht.
- R¹: steht außerdem auch vorzugsweise für einen Rest der Formel 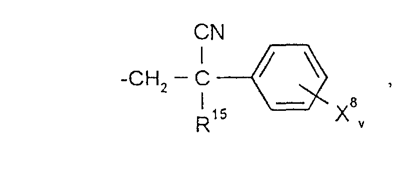 in welcher
R¹⁵ bevorzugt für Methyl, Ethyl, n-Propyl, Isopropyl, n-Butyl, sek.-Butyl, iso-Butyl, tert.-Butyl, Fluor-tert.-butyl, Difluor-tert.-butyl, gegebenenfalls einfach oder zweifach, gleichartig oder verschieden durch Fluor, Chlor, Methyl, Ethyl, Trifluormethyl, Trichlormethyl, Methoxy, Ethoxy, Trifluormethoxy und/oder Difluormethoxy substituiertes Phenyl oder für gegebenenfalls im Phenylteil einfach oder zweifach, gleichartig oder verschieden durch Fluor, Chlor, Brom, Methyl, Ethyl, Trifluomethyl, Trichlormethyl, Methoxy, Ethoxy, Trifluormethoxy und/oder Difluormethoxy substituiertes Phenylalkyl mit 1 oder 2 Kohlenstoffatomen im Alkylteil steht,
X⁸ bevorzugt für Fluor, Chlor, Brom, Methyl, Ethyl, tert.-Butyl, Methoxy, Methylthio, Trichlormethyl, Trifluormethyl, Trifluormethoxy, Difluormethoxy, Trifluormethylthio, Phenyl oder Phenoxy steht, und
v auch bevorzugt für die Zahlen 0, 1, 2 oder 3 steht, wobei X⁸ für gleiche oder verschiedene Reste steht, wenn v für 2 oder 3 steht.

Die bei der Herstellung der erfindungsgemäßen Stoffe als Ausgangssubstanzen benötigten Mercapto-triazole können in der "Mercapto"-Form der Formel 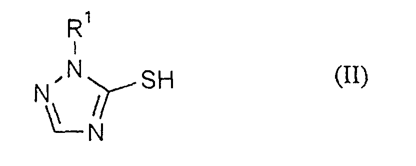 oder in der tautomeren "Thiono"-Form der Formel 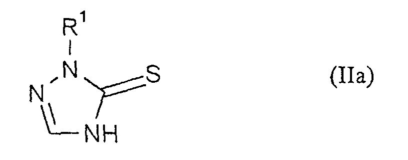 vorliegen. Es ist deshalb nicht auszuschließen, daß sich die erfindungsgemäßen Stoffe ganz oder teilweise von der "Thiono"-Form der Formel (IIa) ableiten. Das bedeutet, daß die erfindungsgemäßen Stoffe entweder als Substanzen der Formel 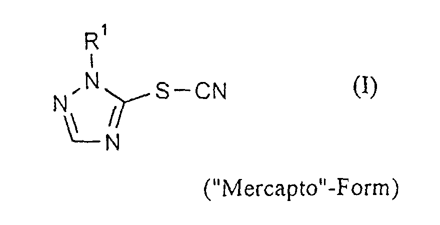 oder der Formel 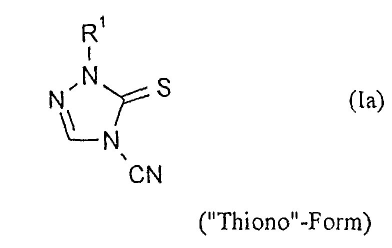 oder als Gemische von Substanzen der Formeln (I) und (Ia) vorliegen. Der Einfachheit halber wird jeweils nur die Struktur der "Mercapto"-Form angegeben.

Als Beispiele für erfindungsgemäße Stoffe seien die in den folgenden Tabellen aufgeführten Thiocyano-triazolyl-Derivate genannt.

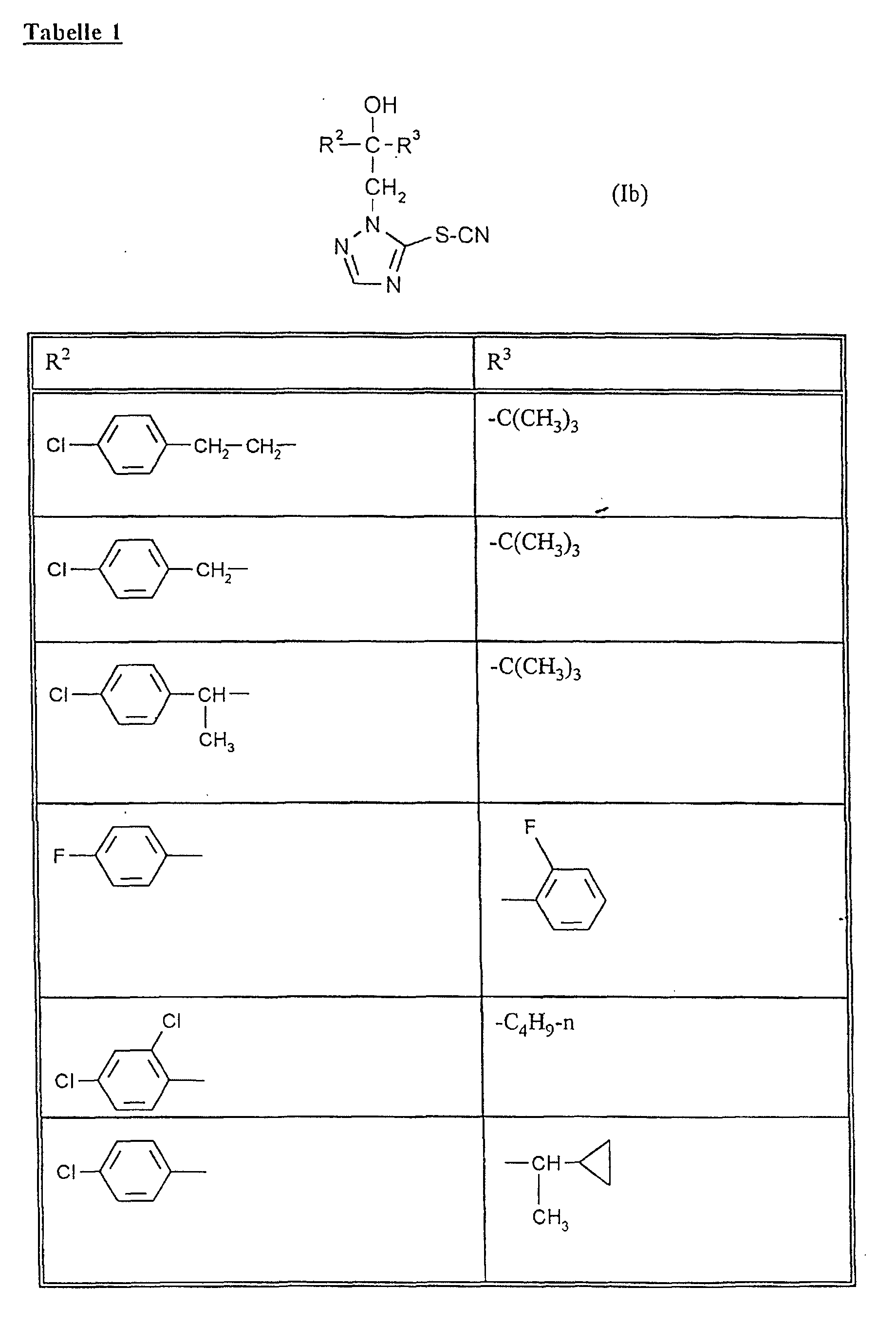

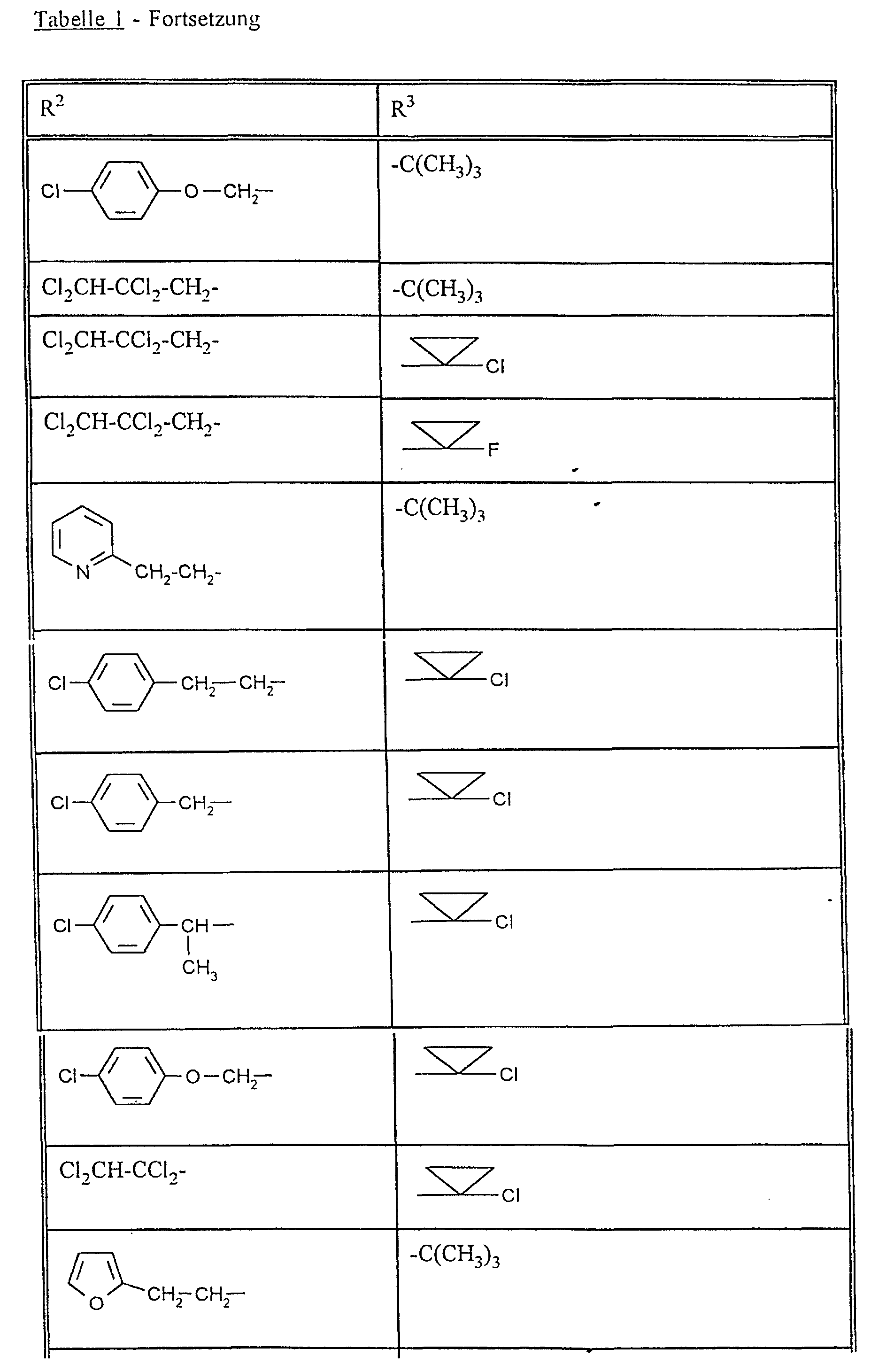

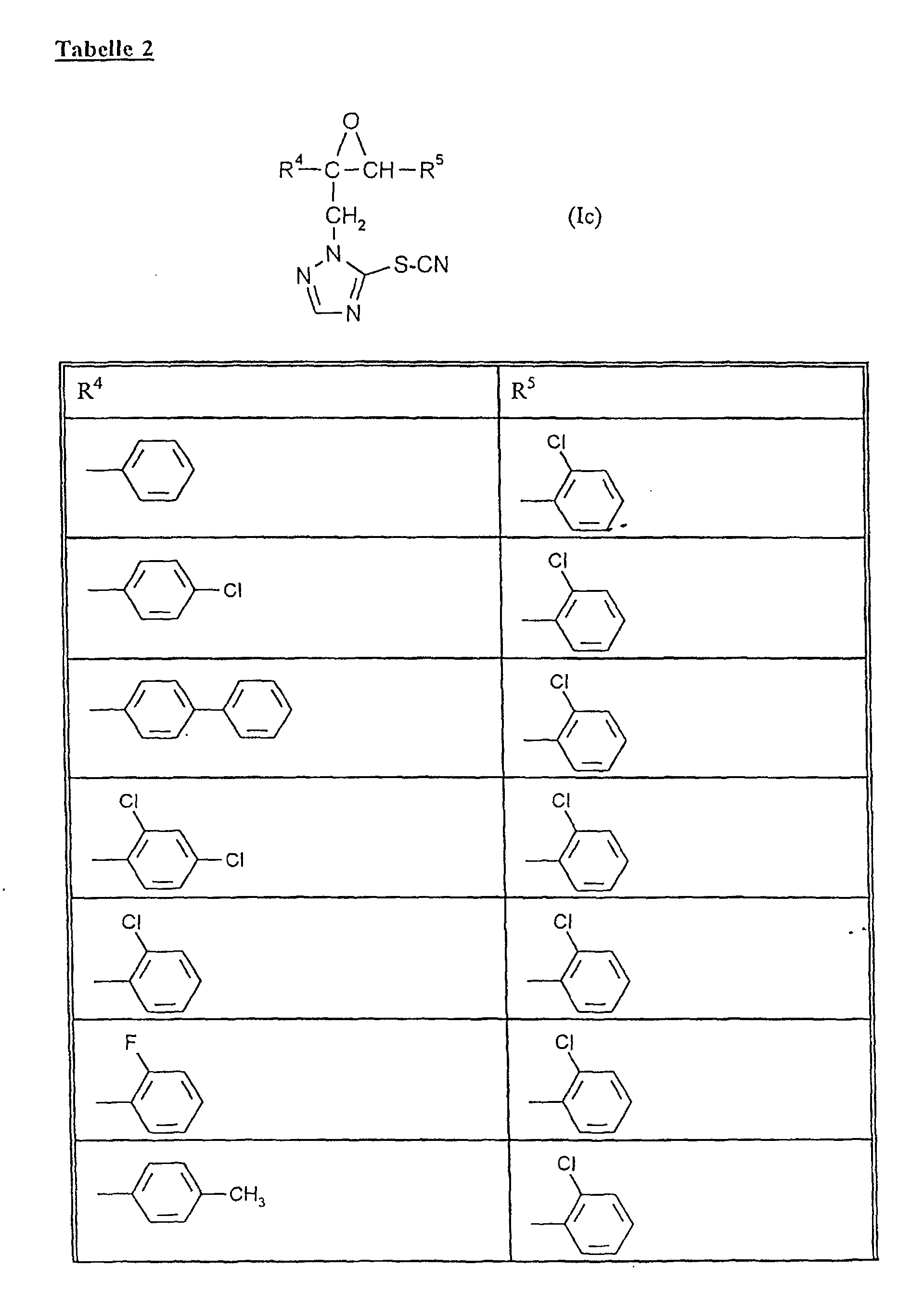

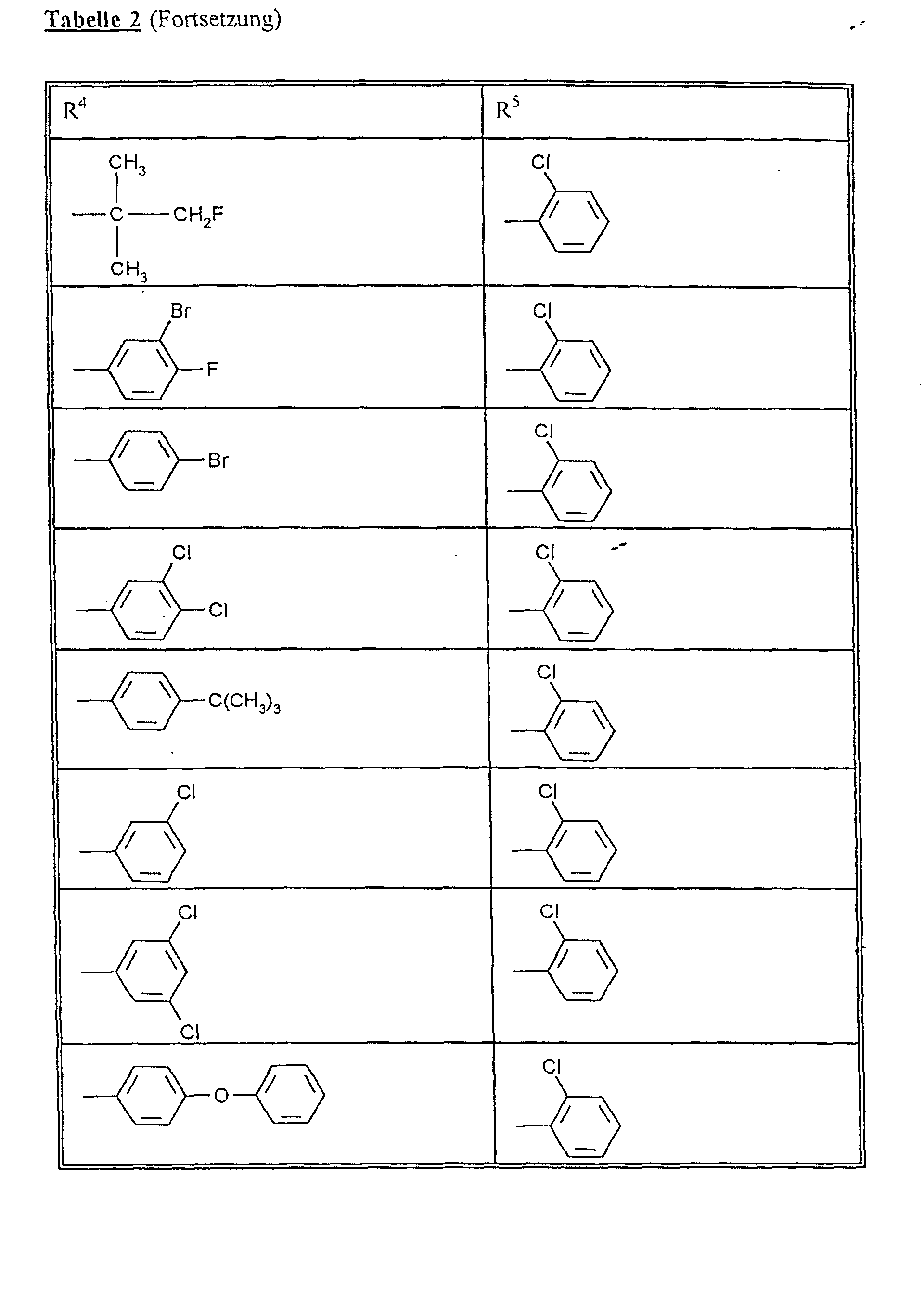

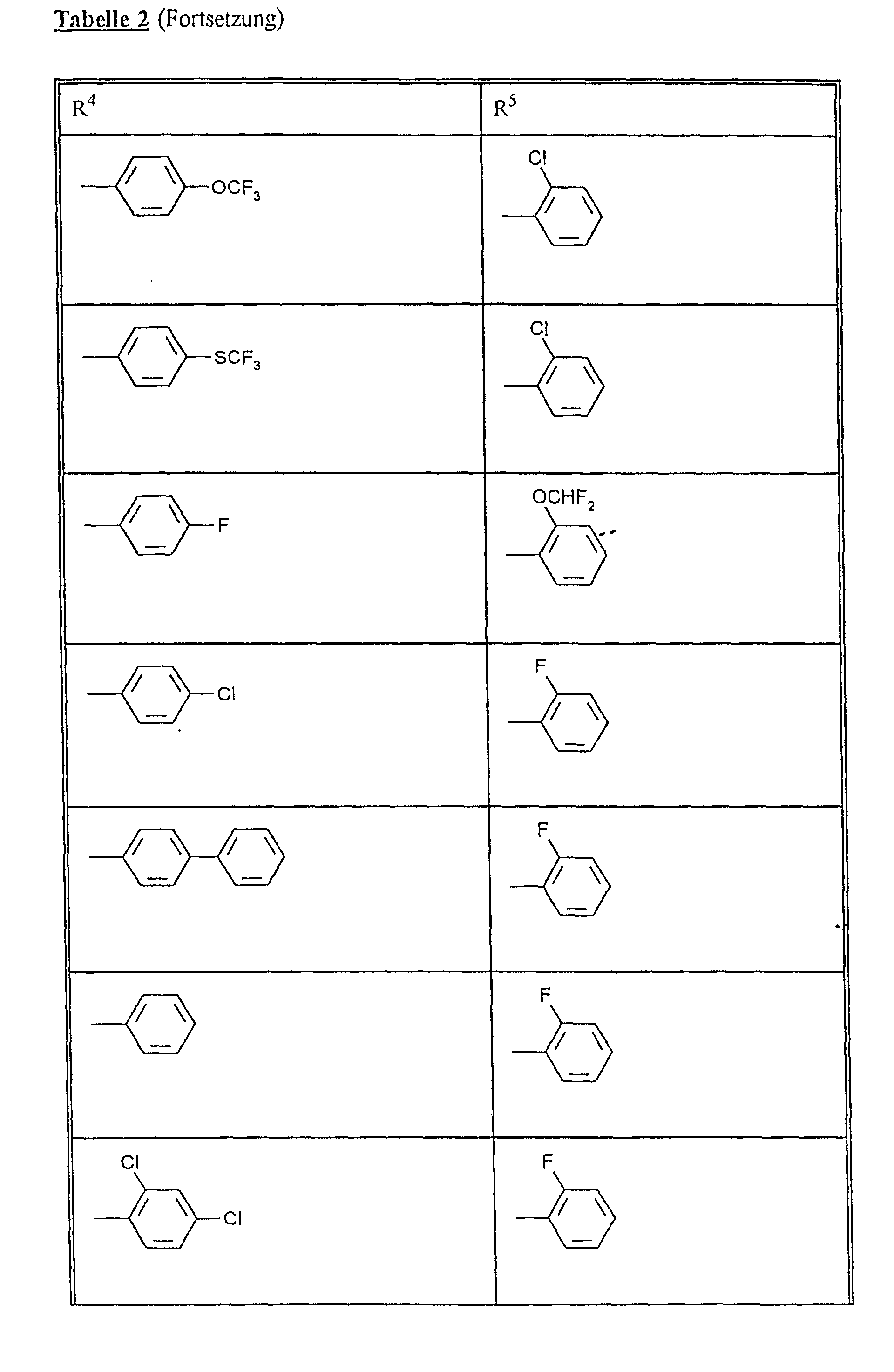

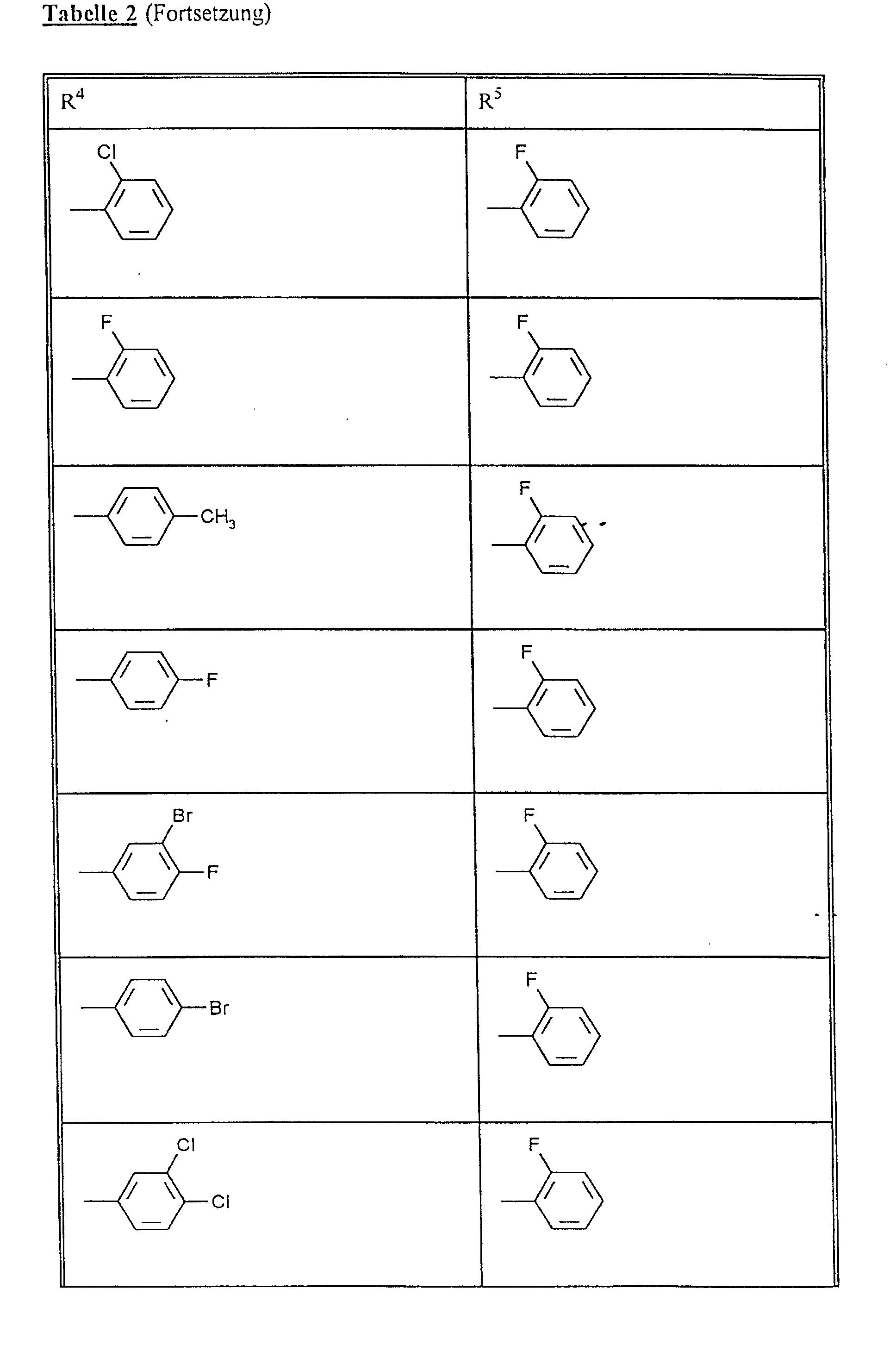

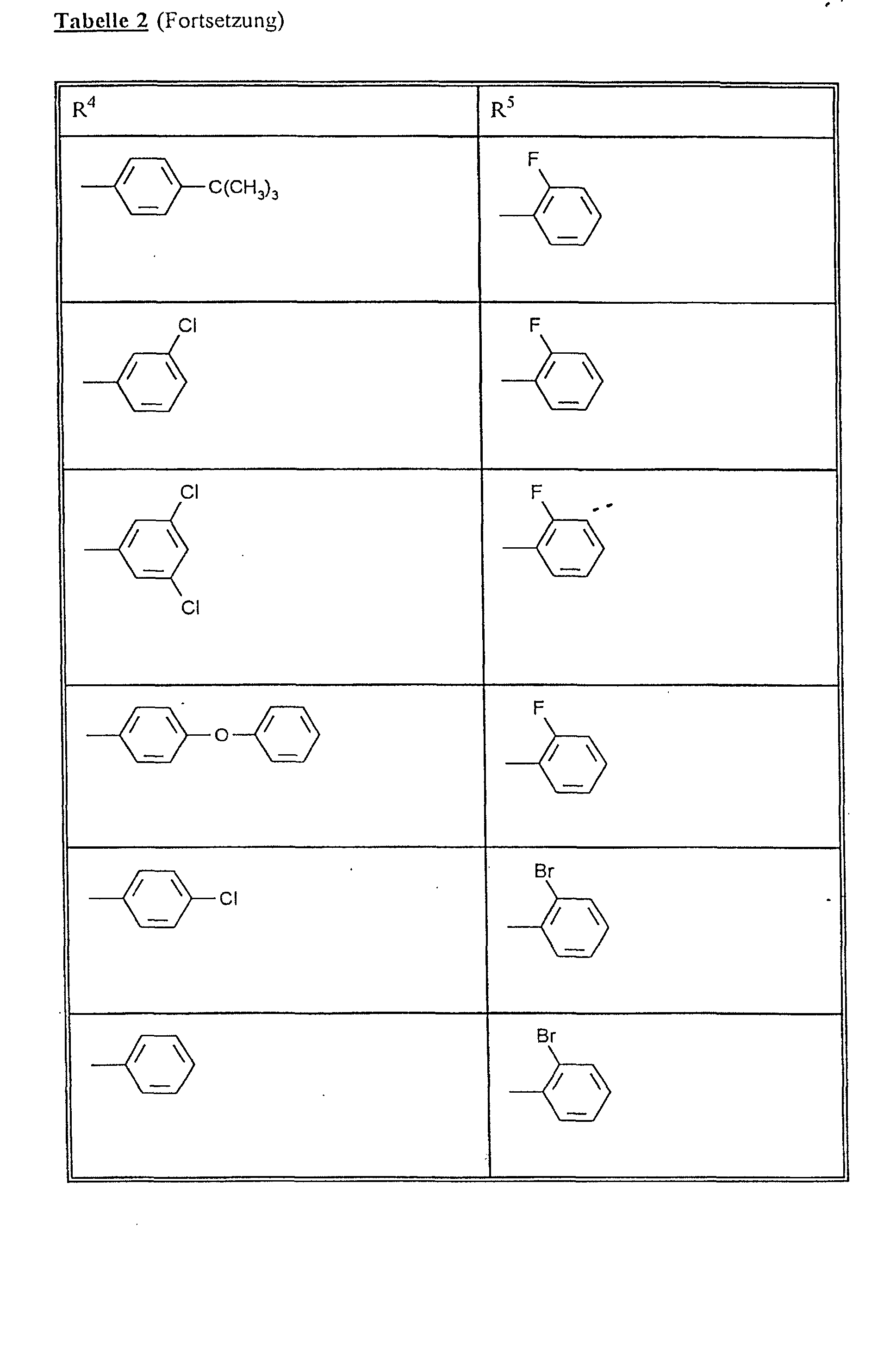

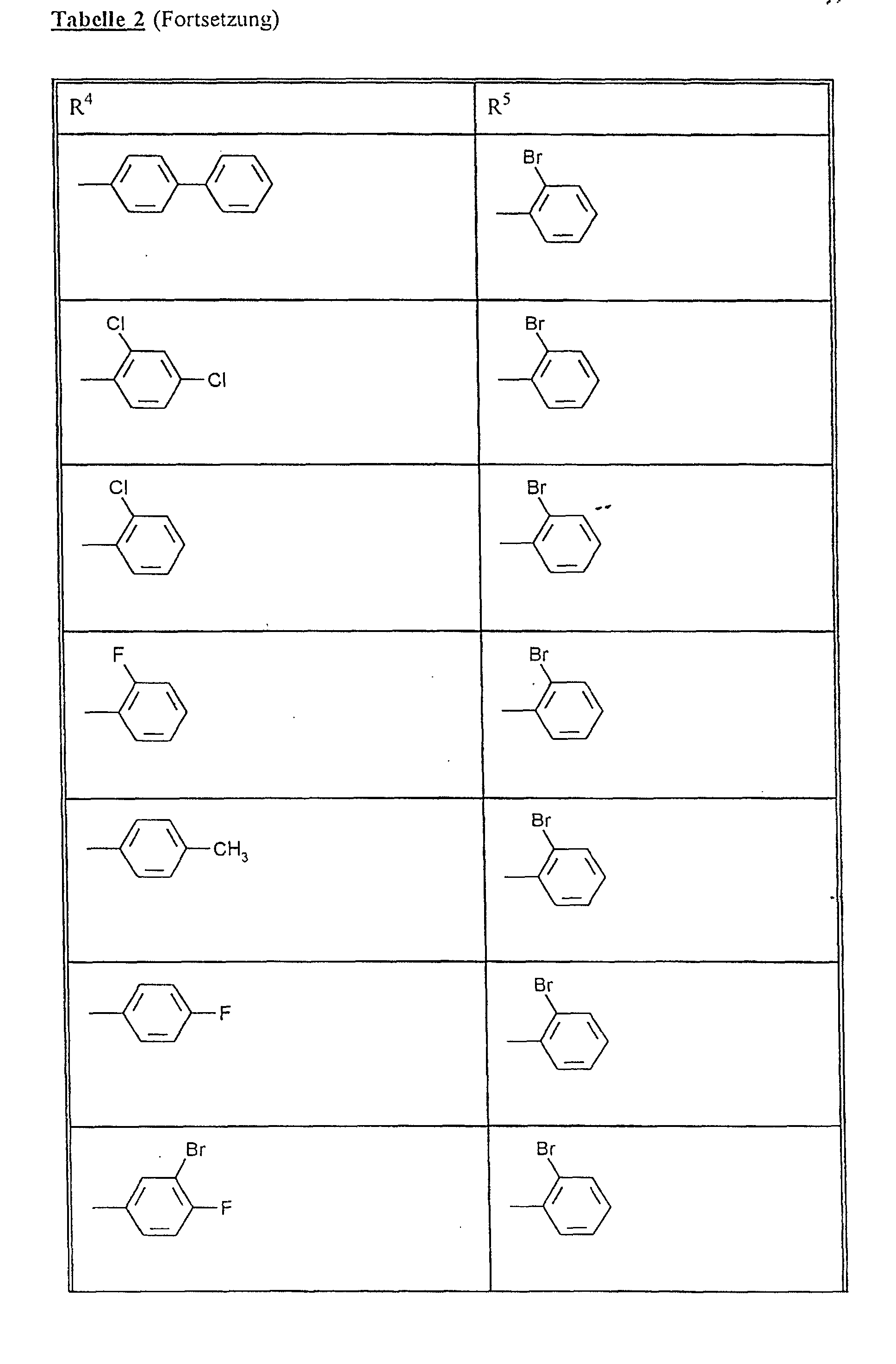

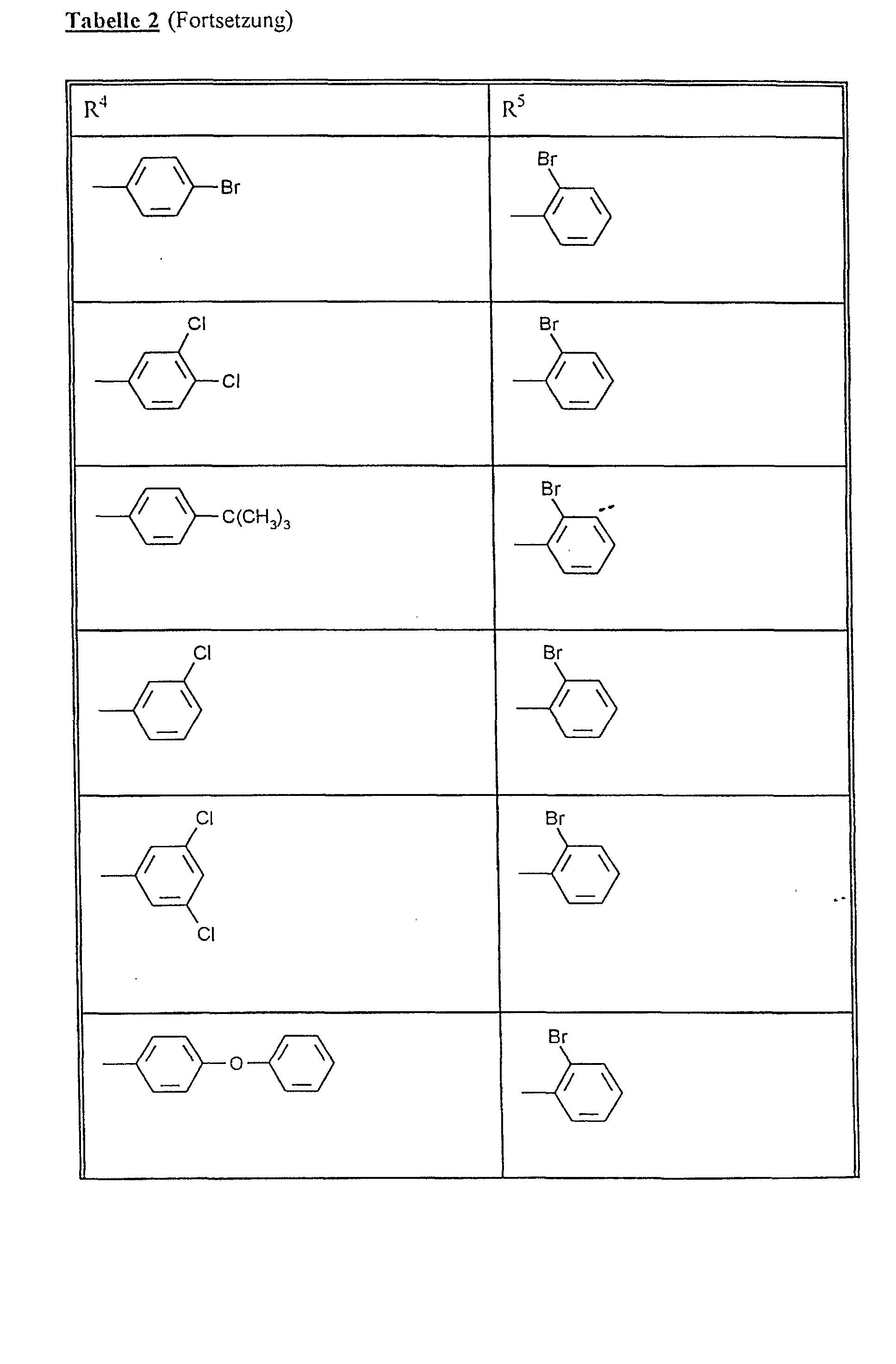

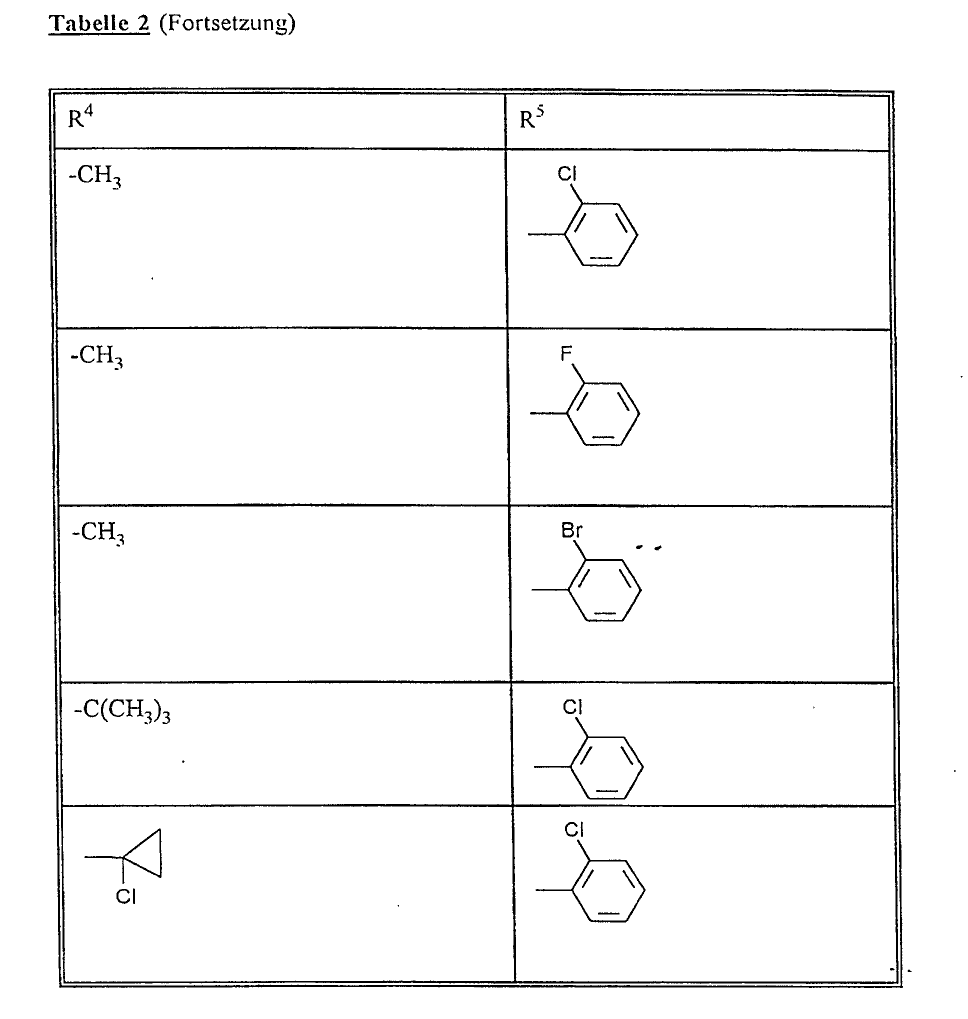

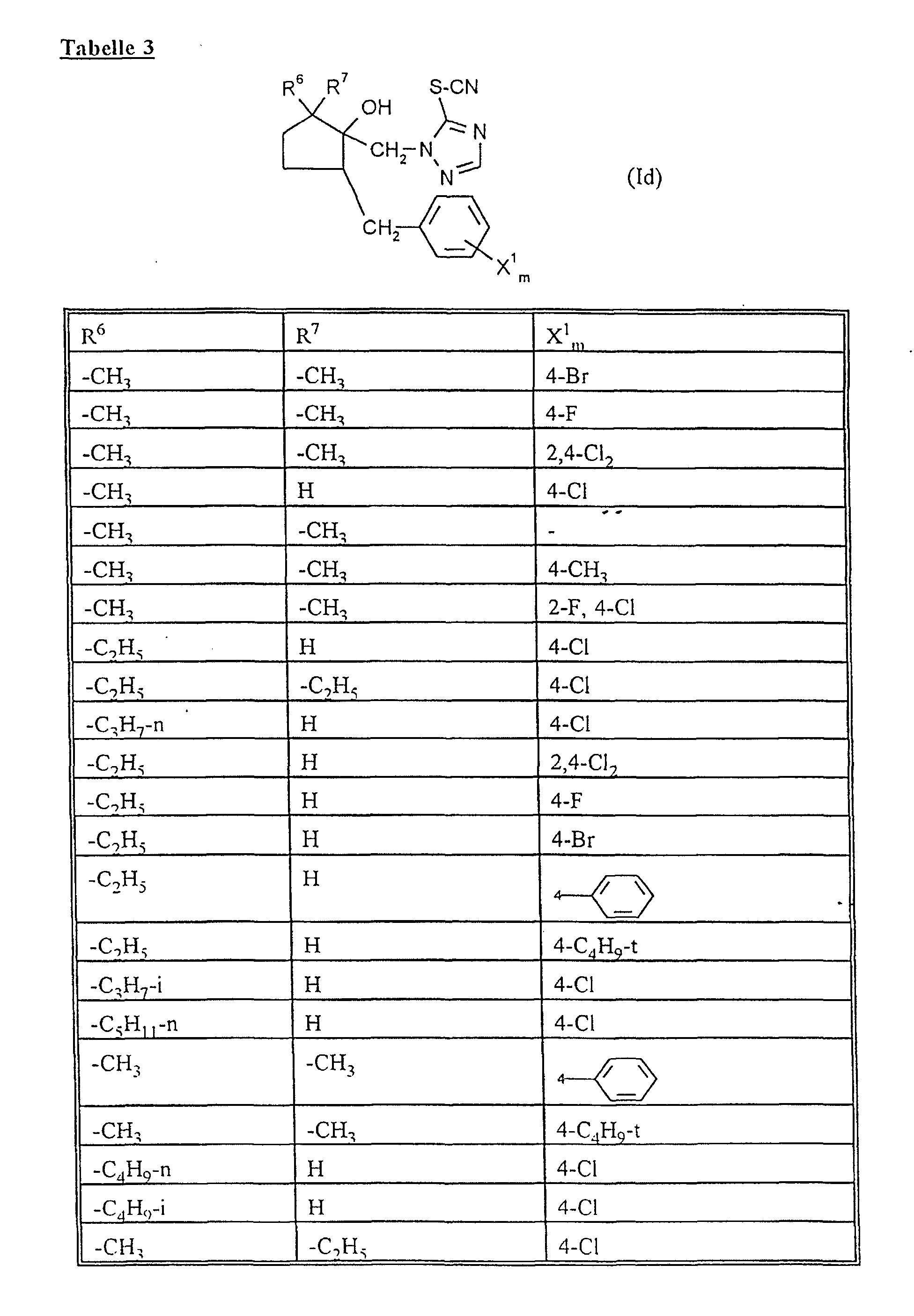

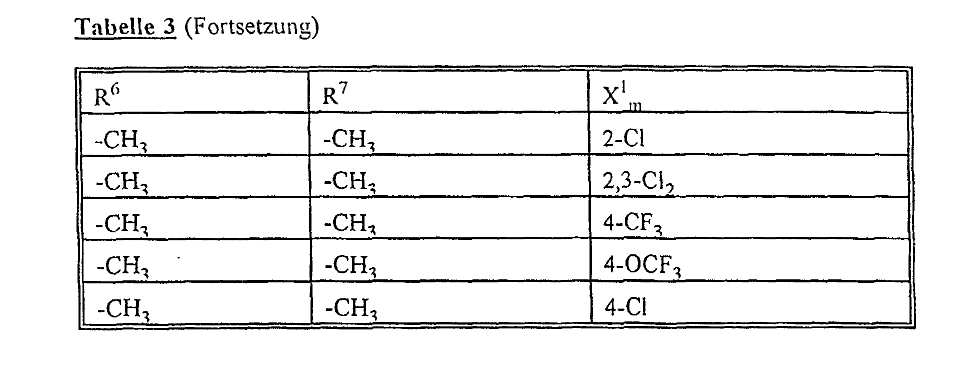

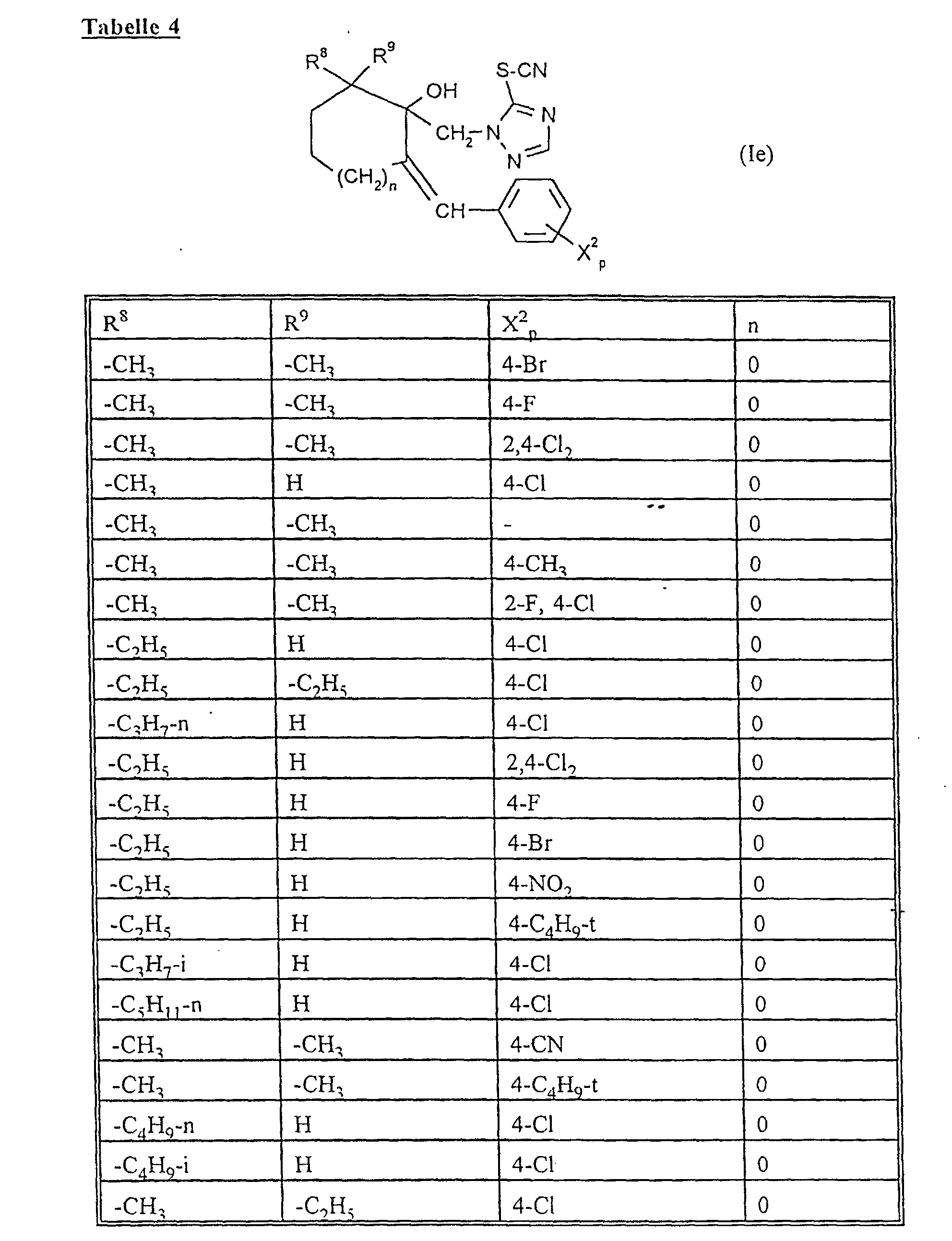

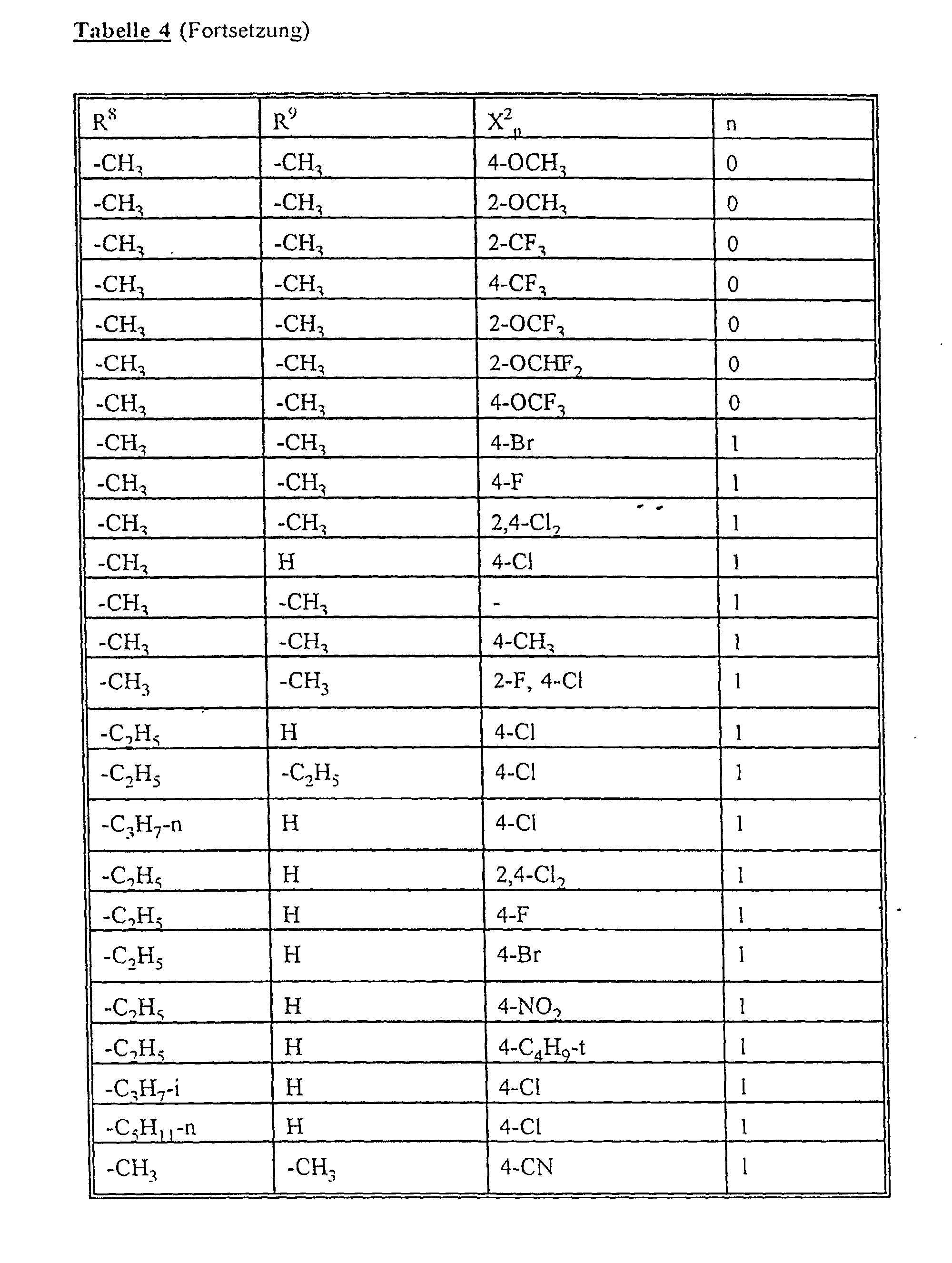

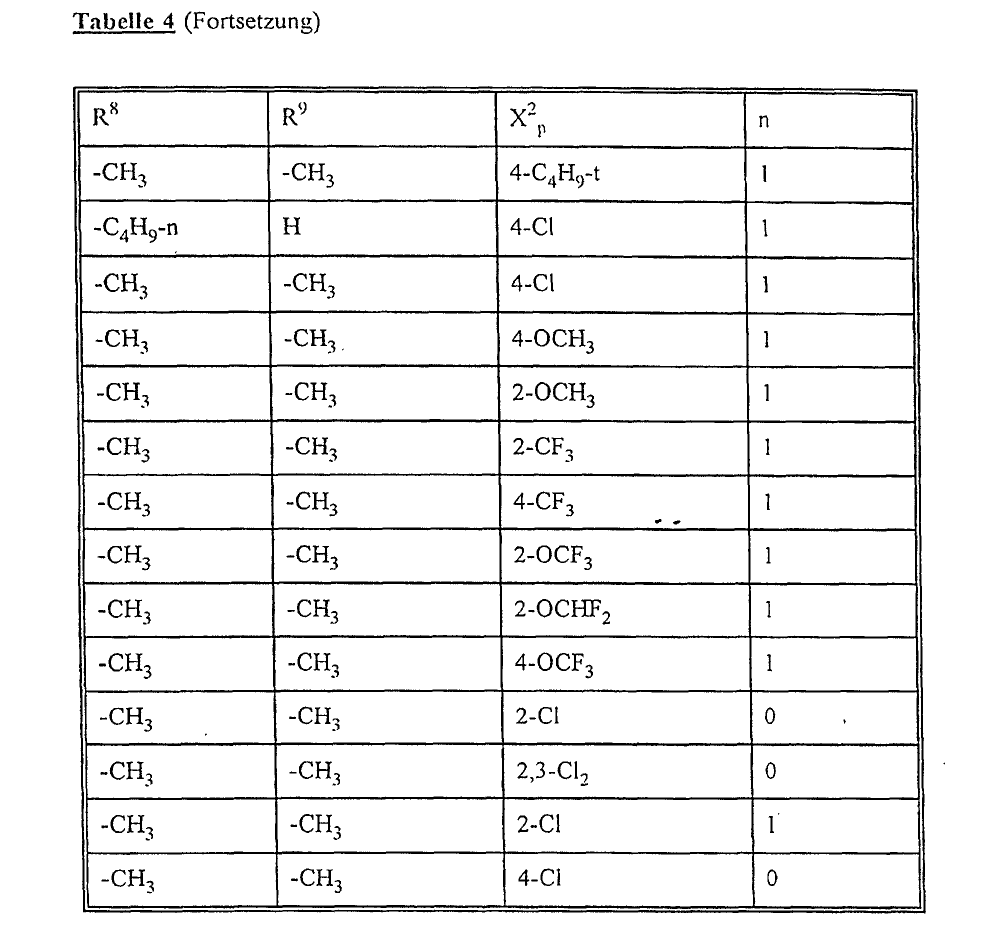

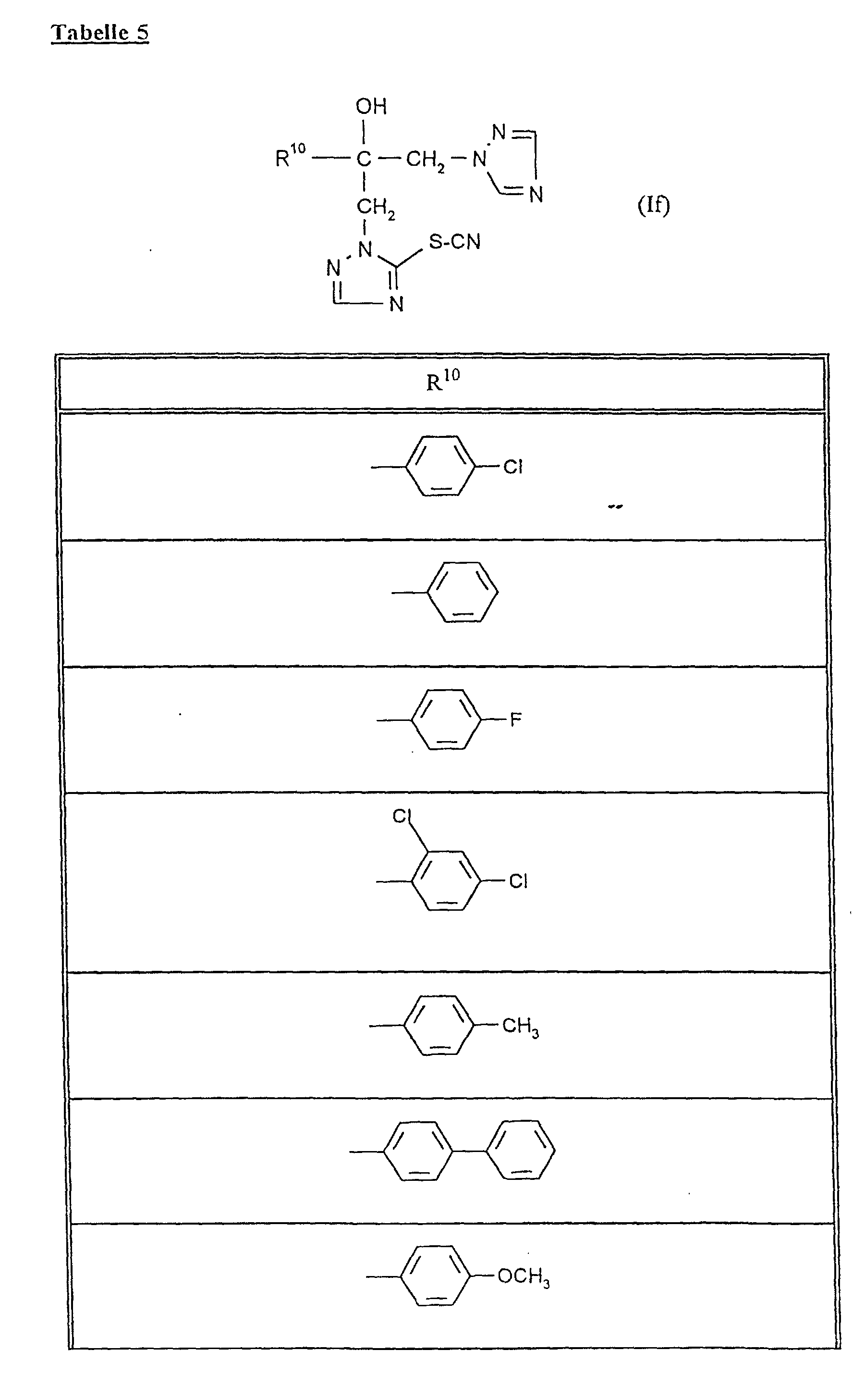

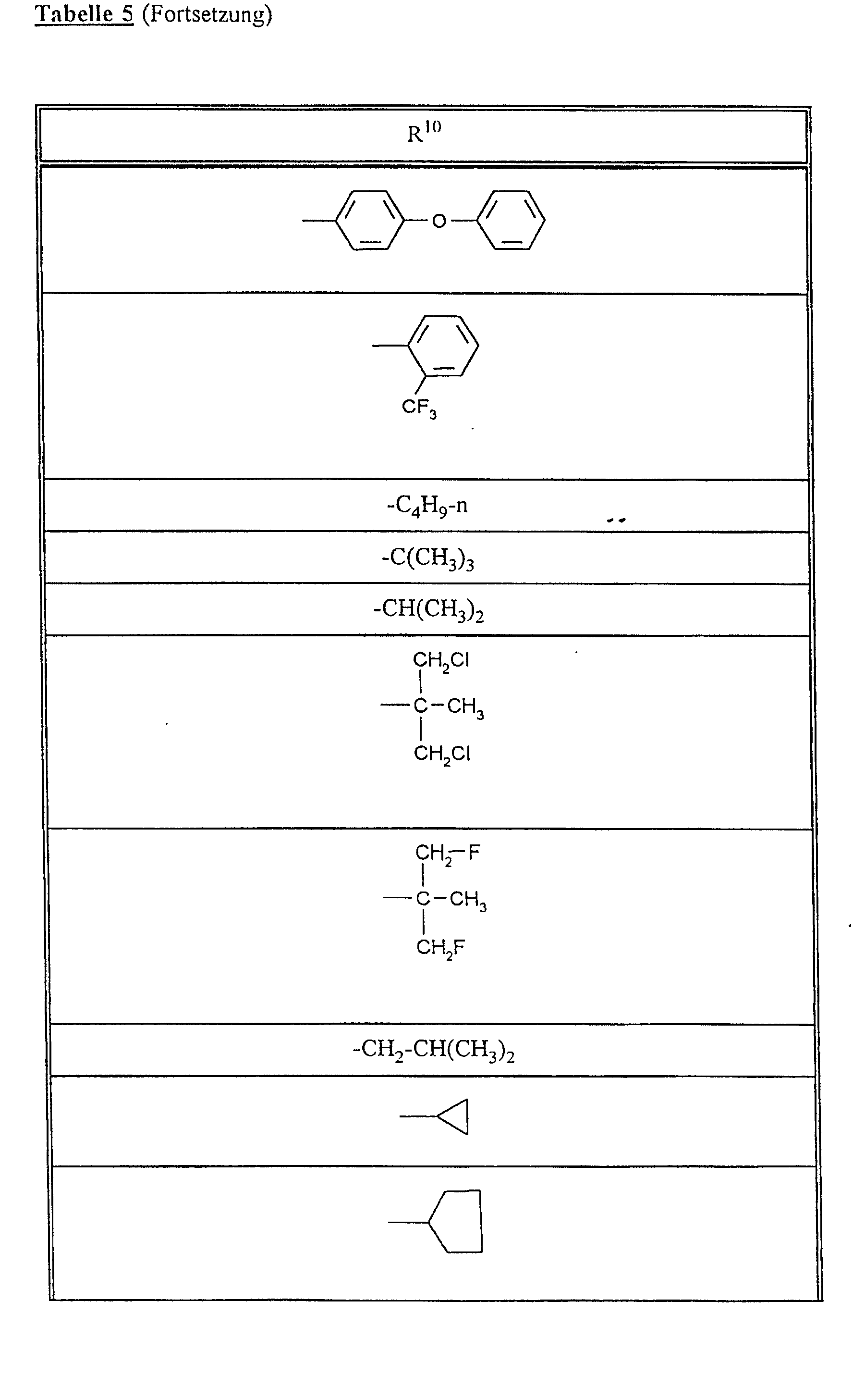

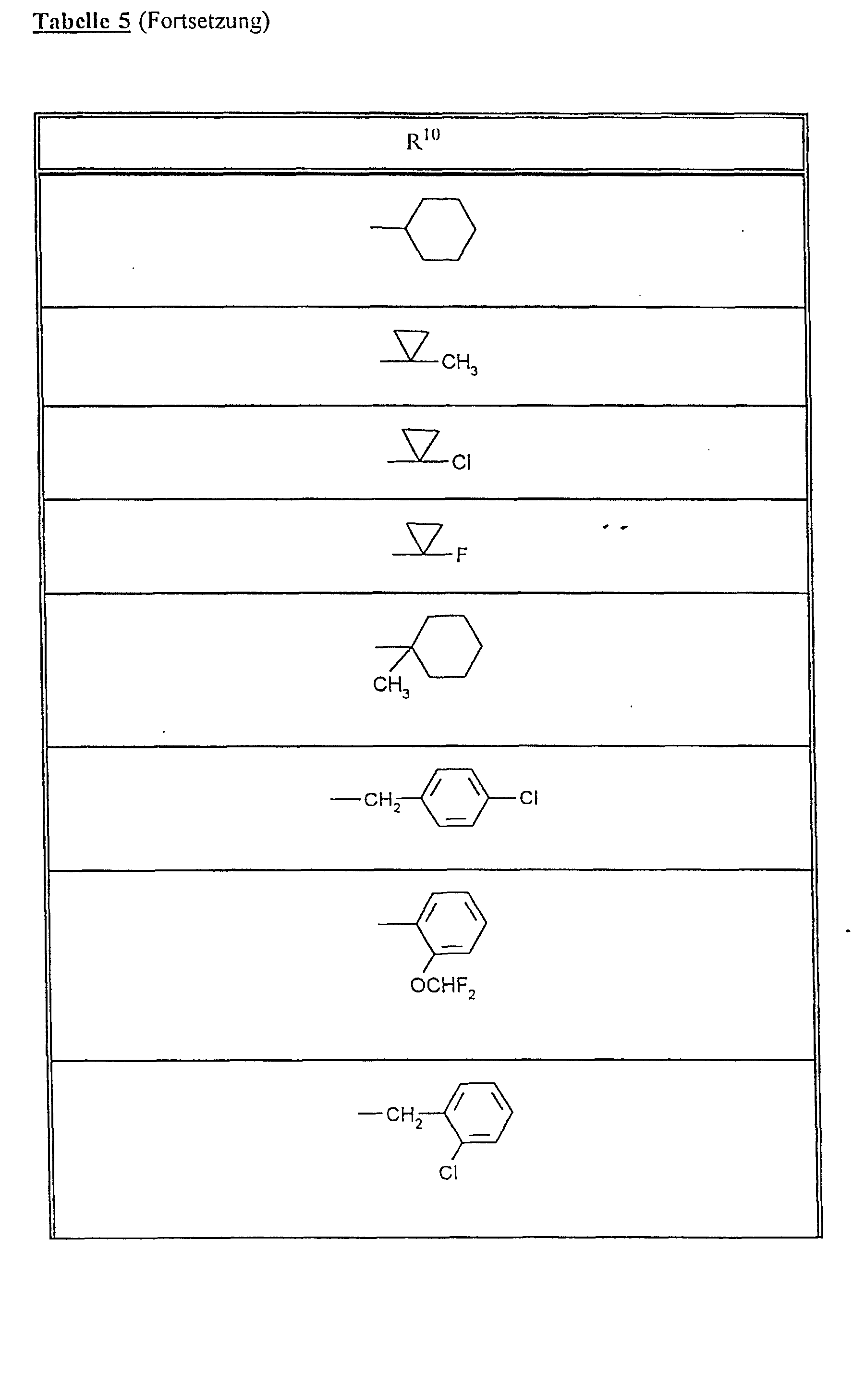

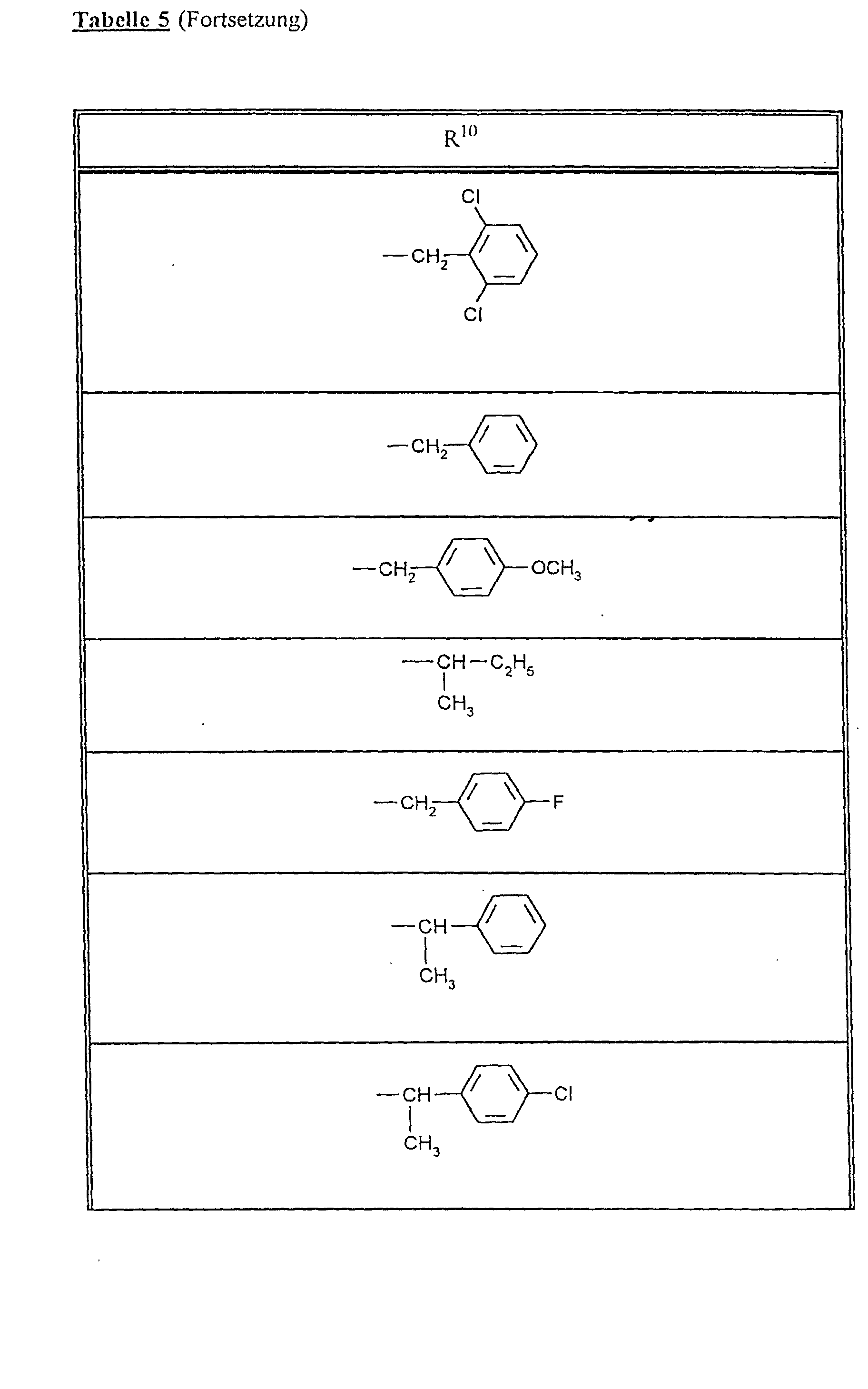

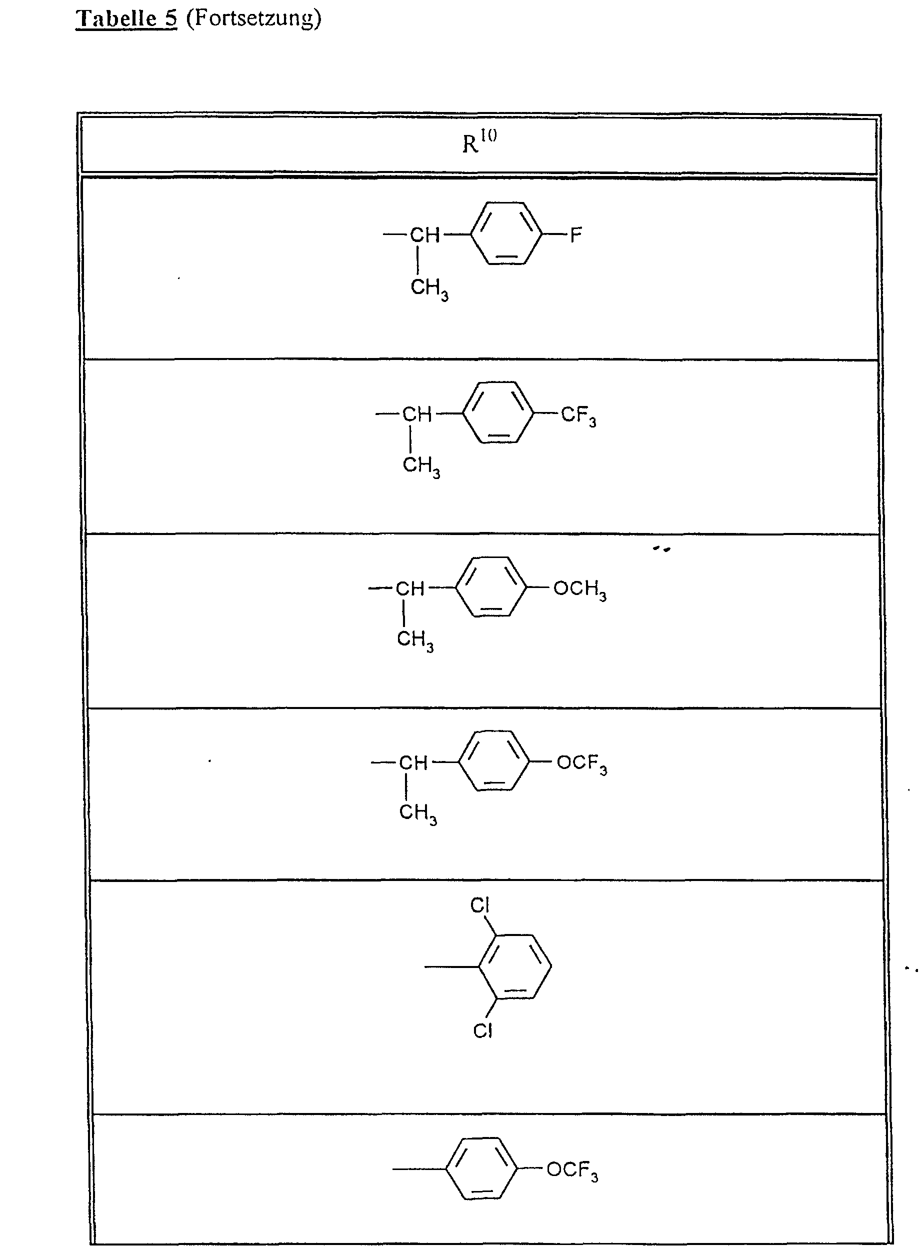

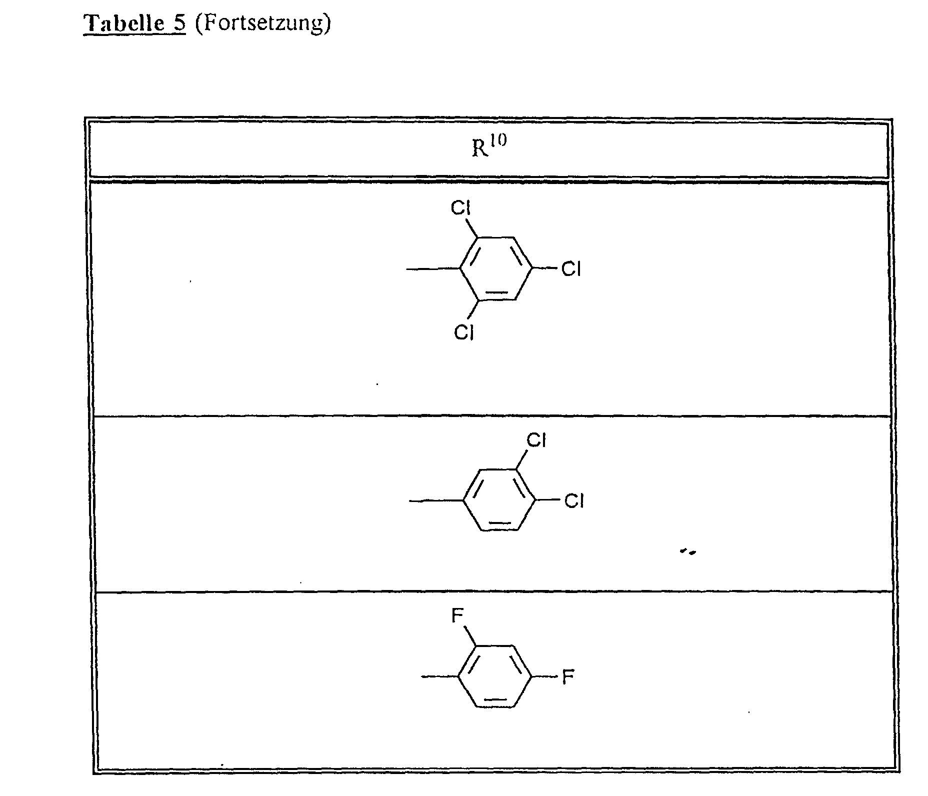

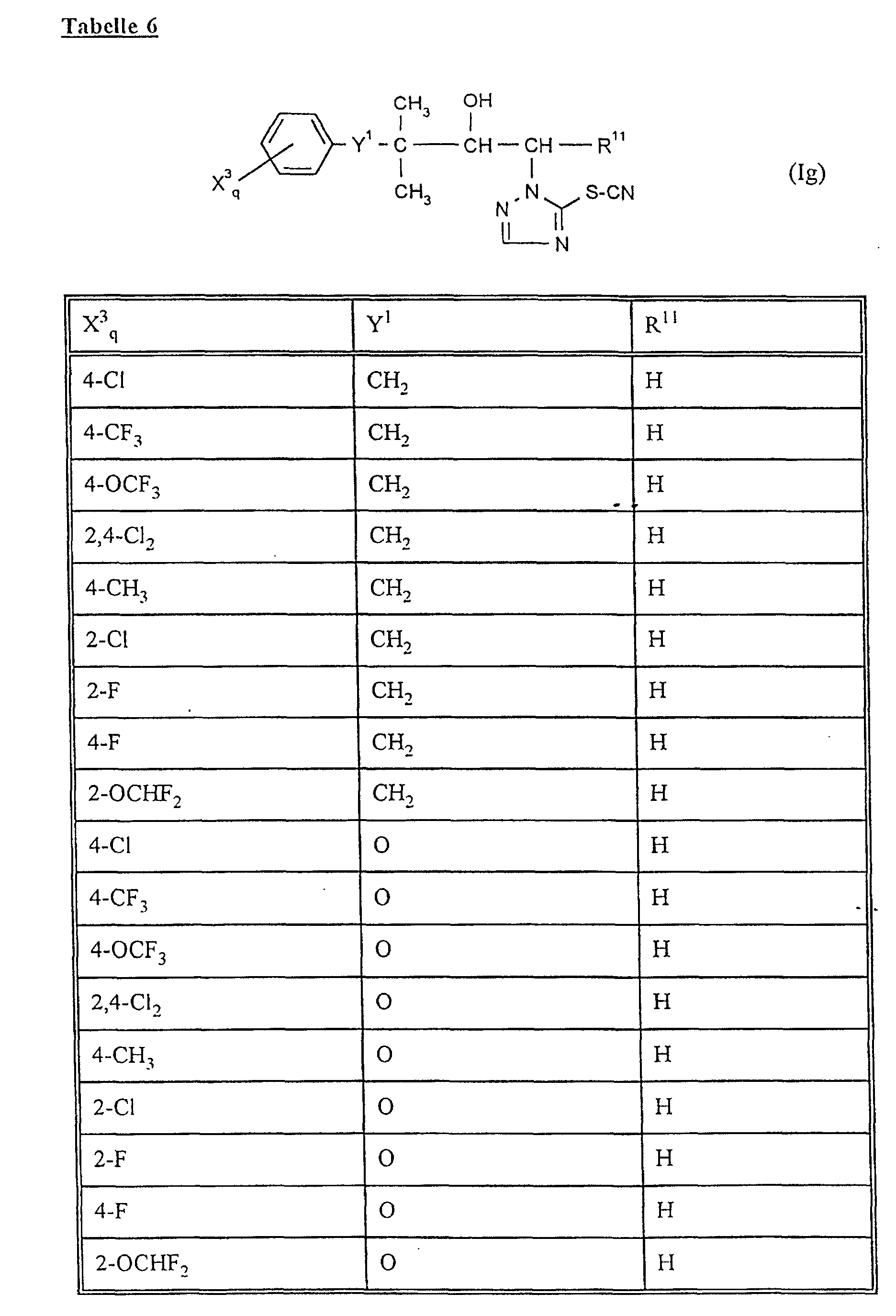

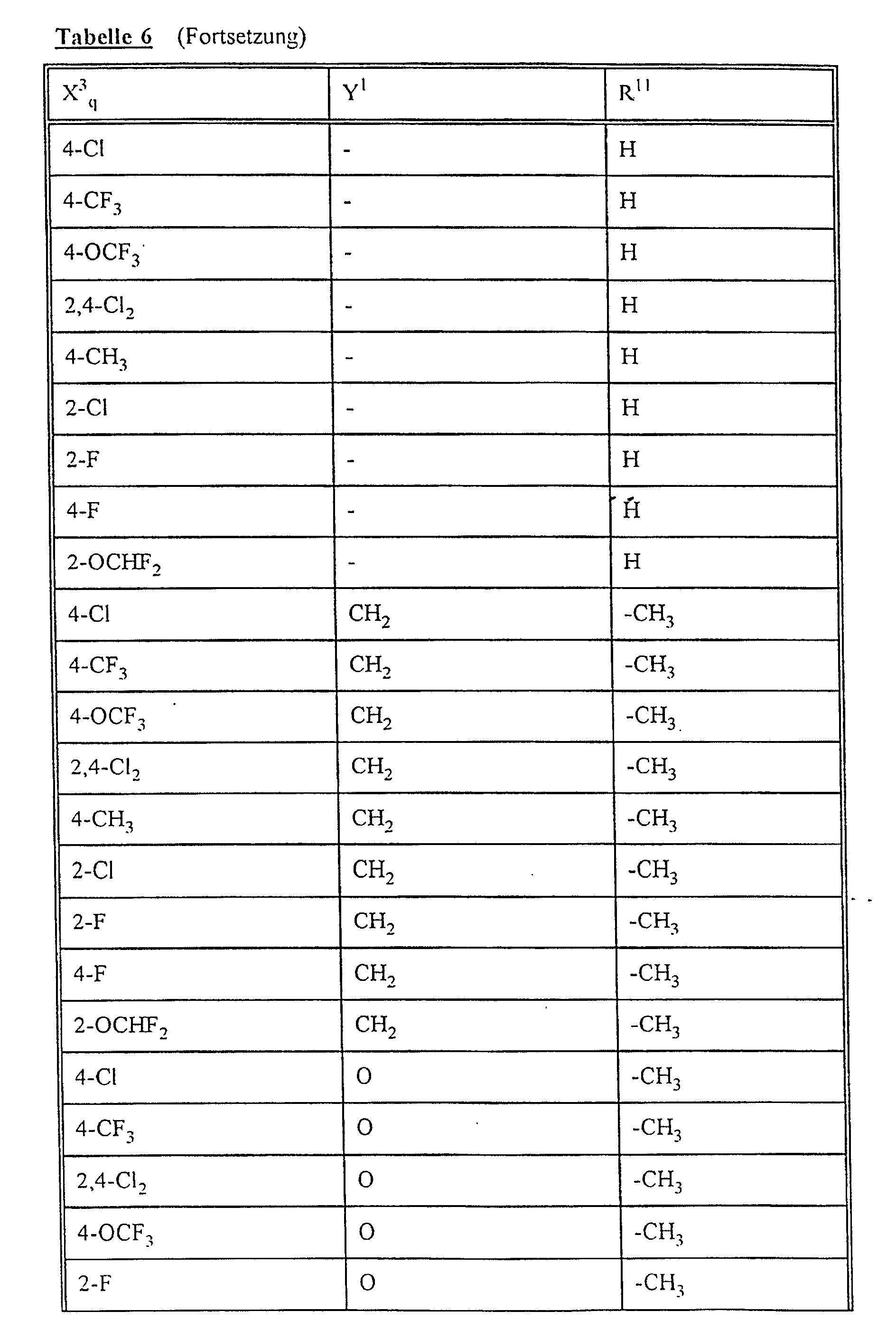

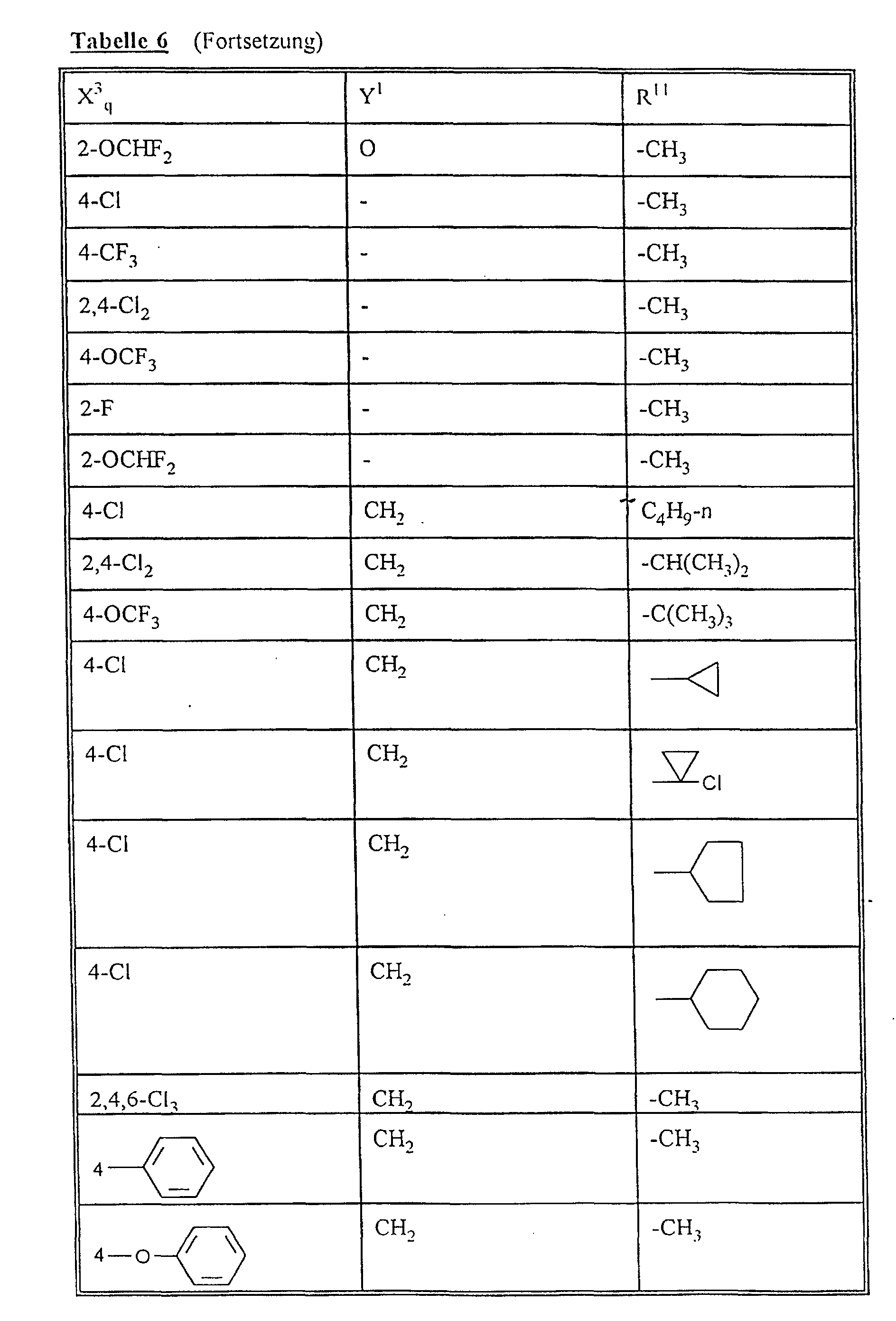

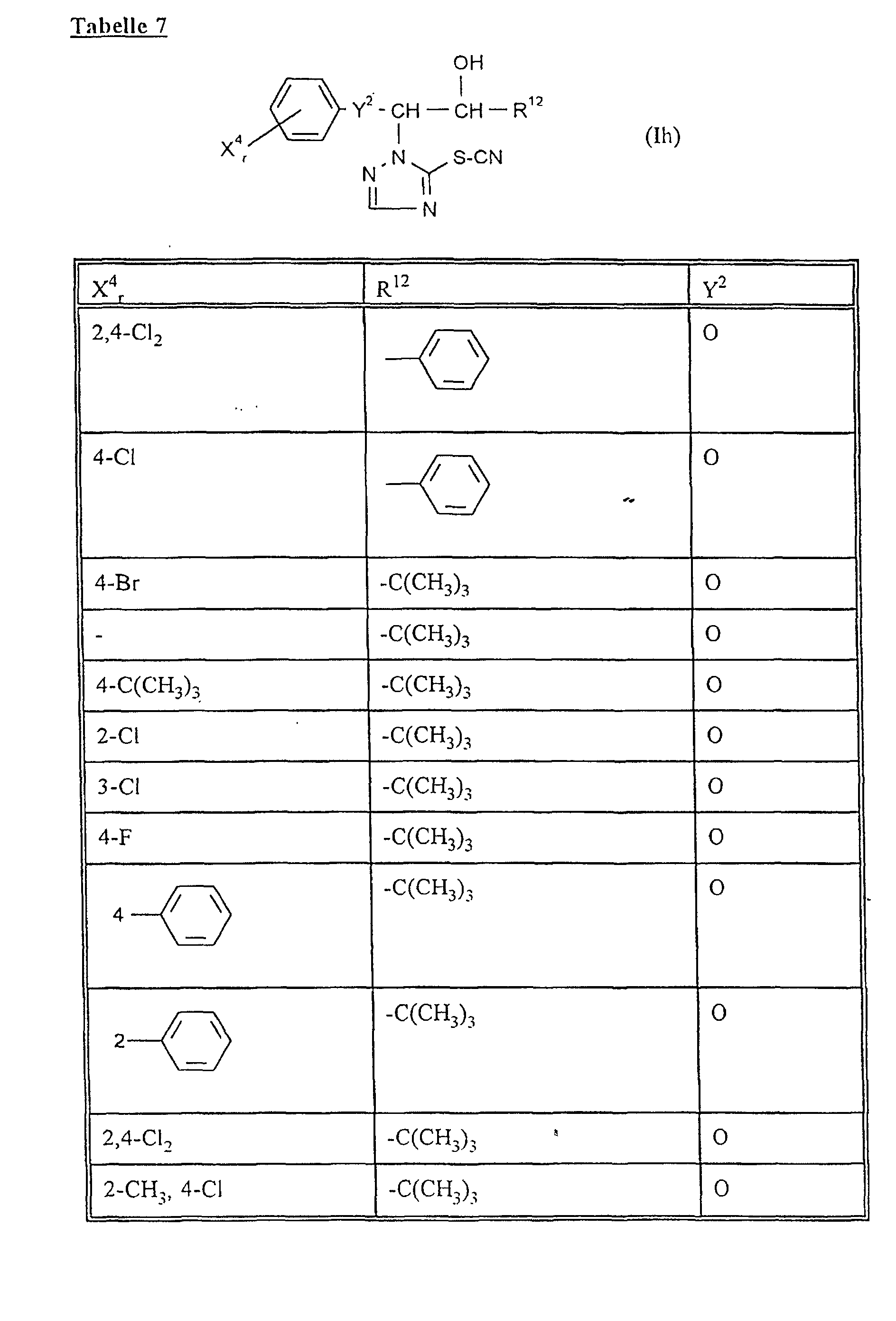

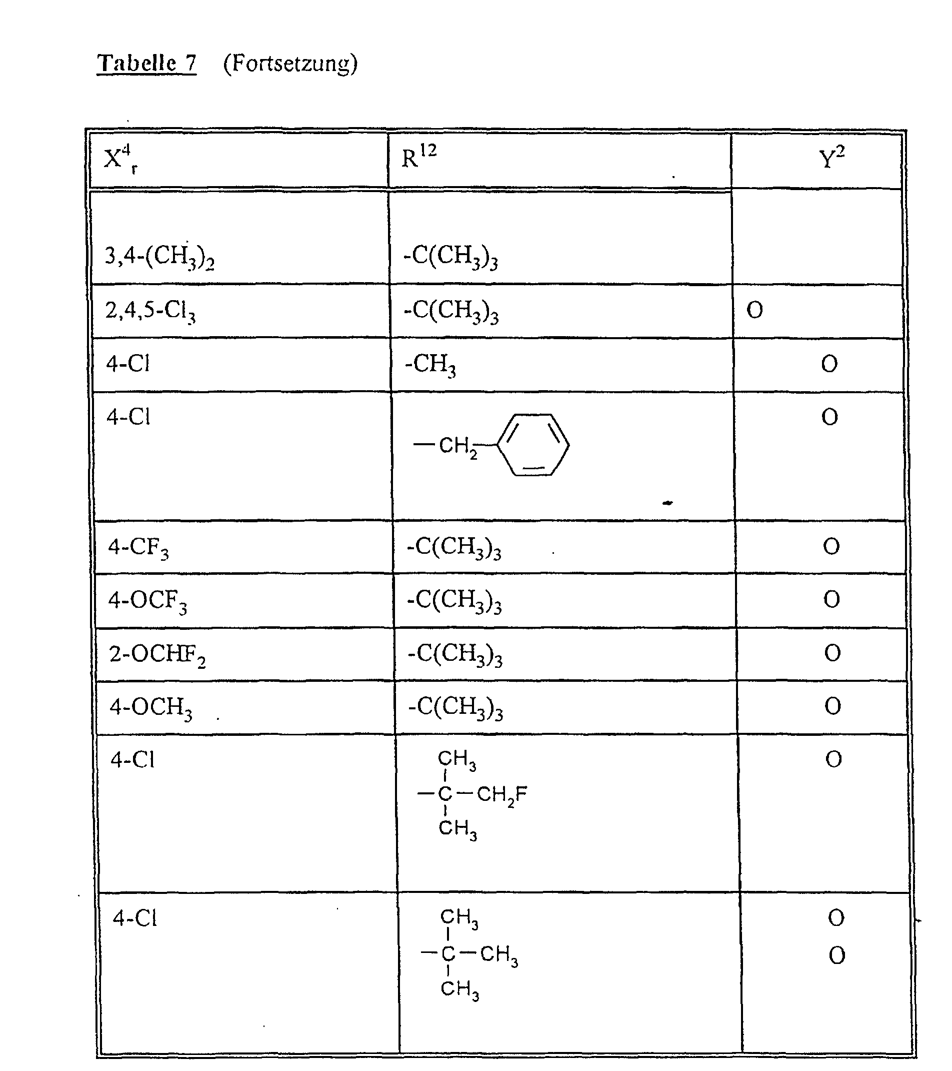

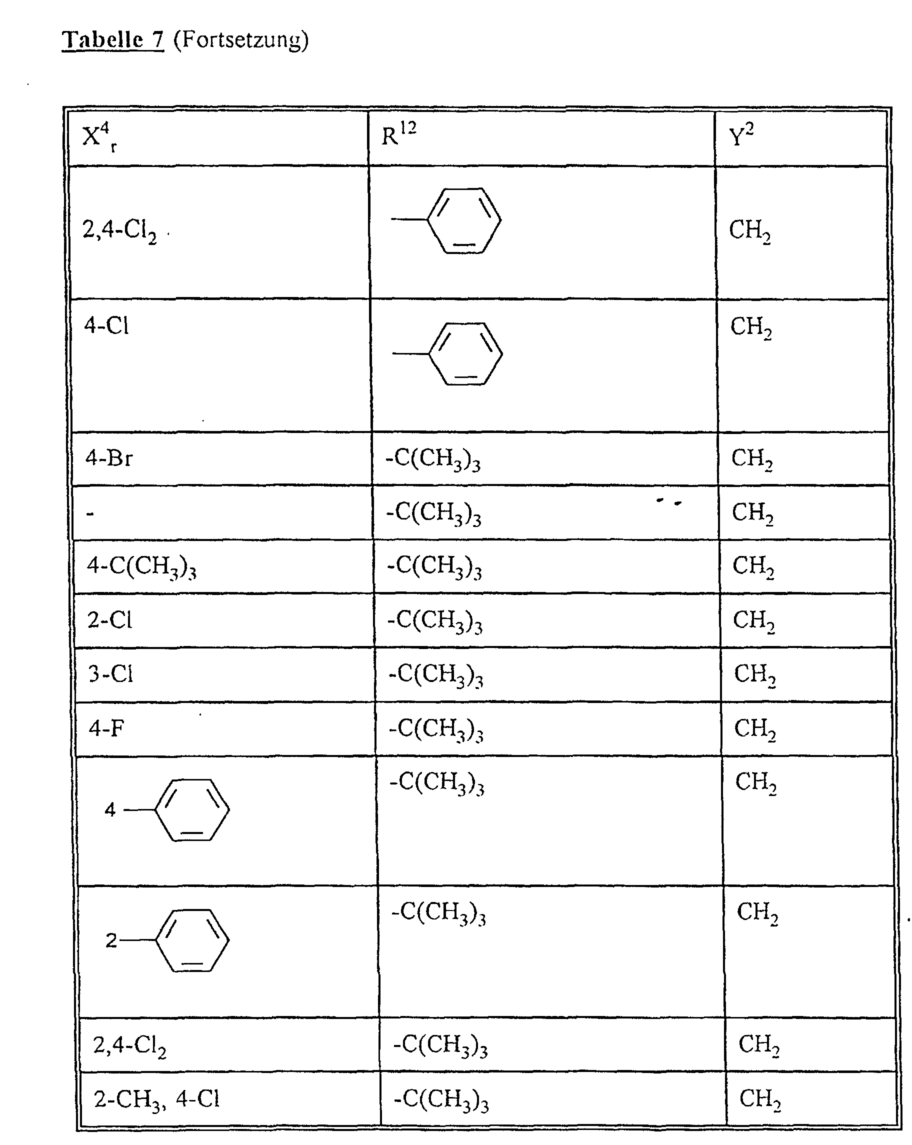

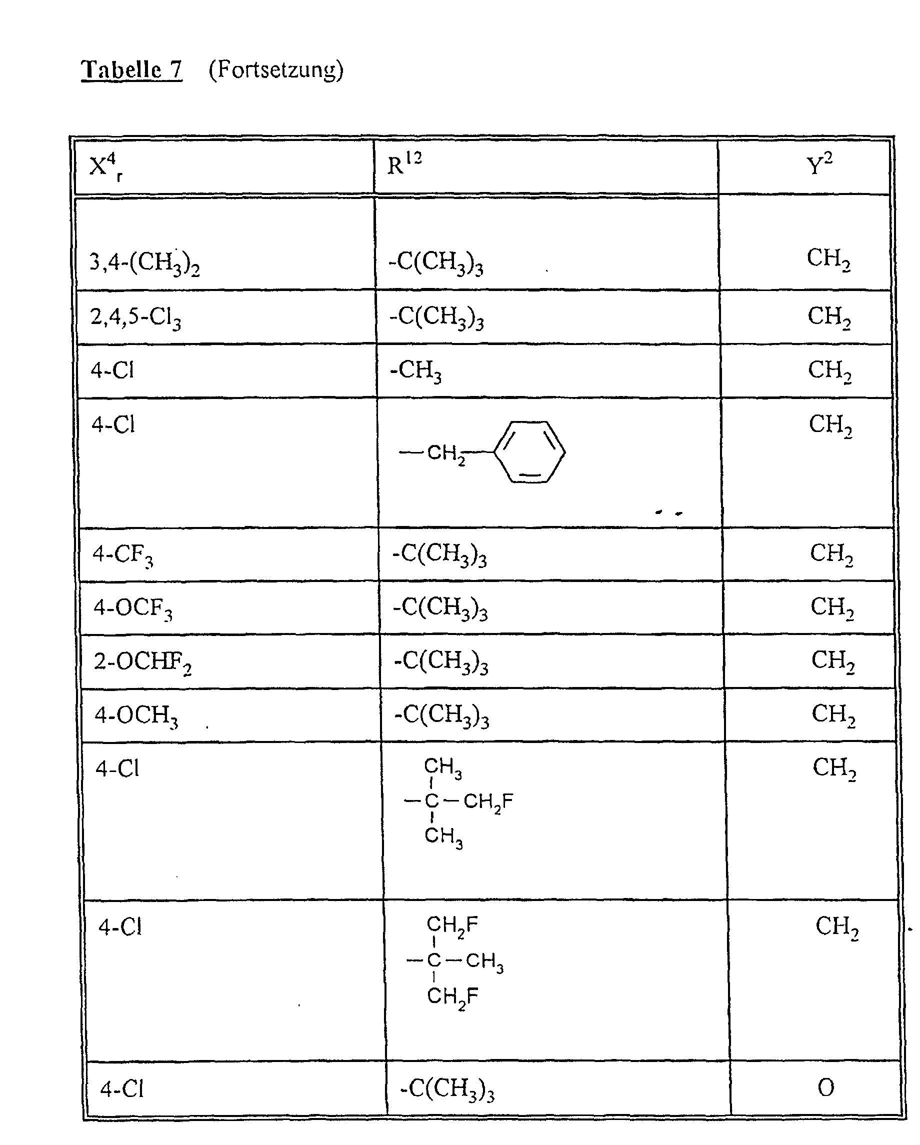

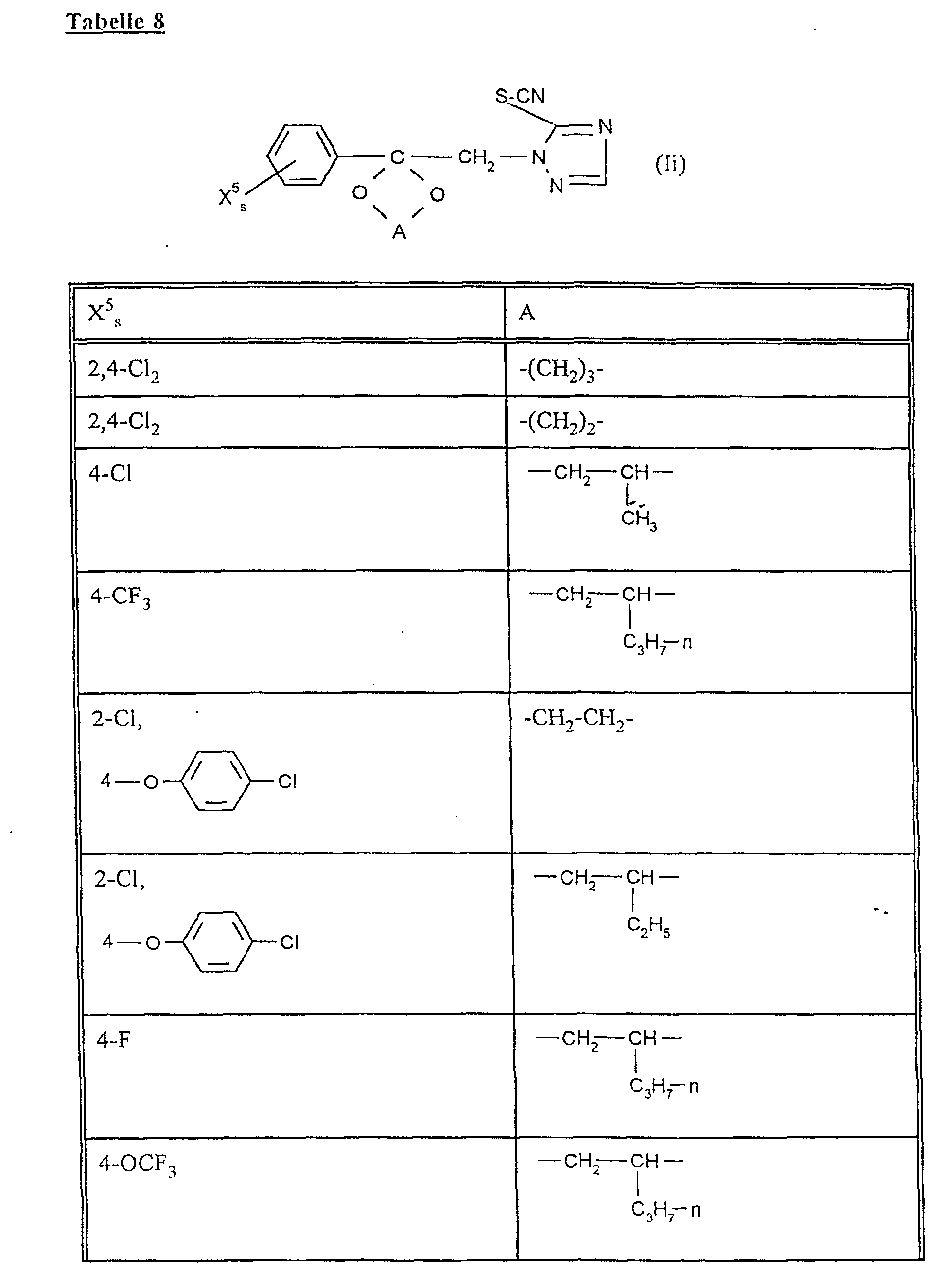

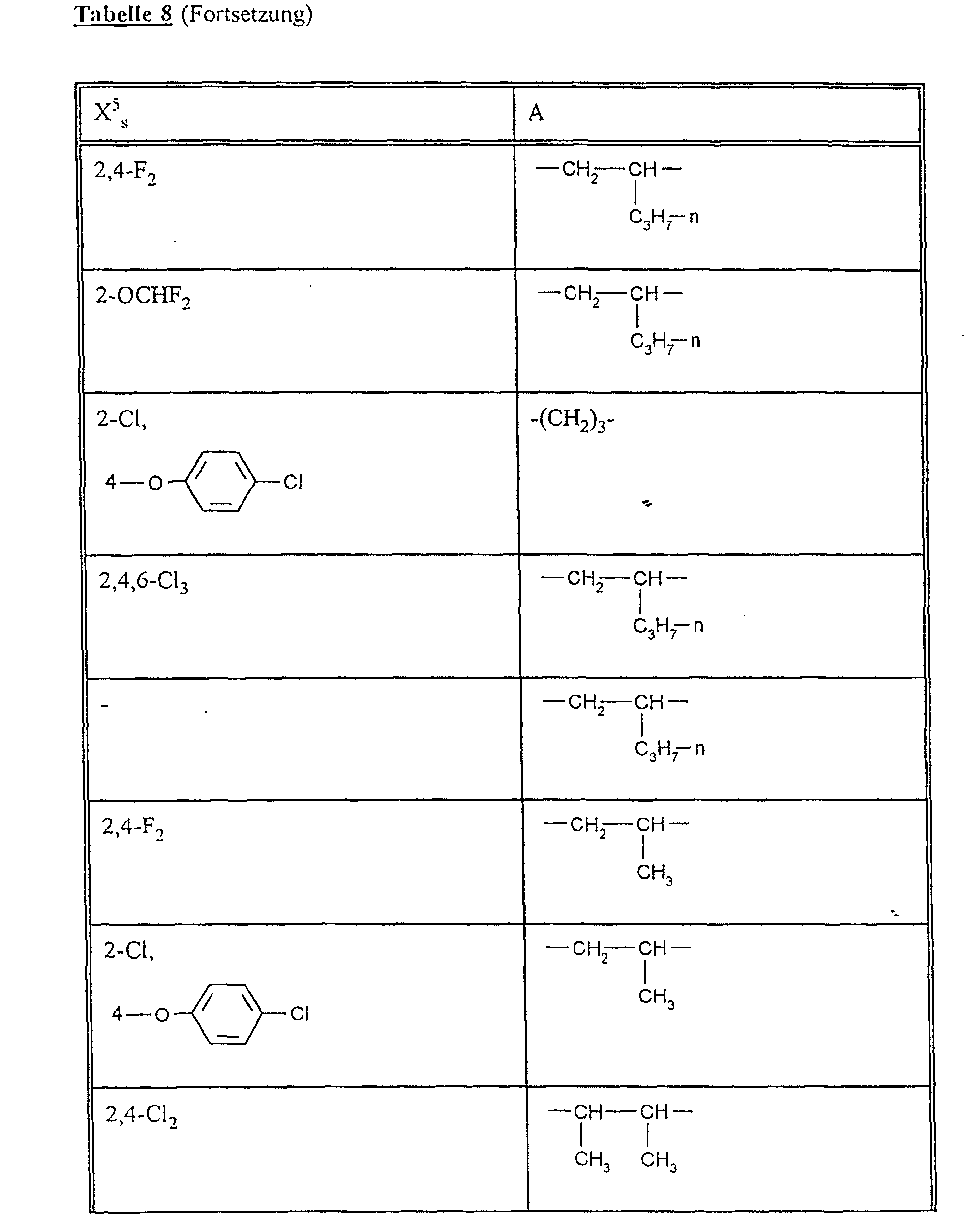

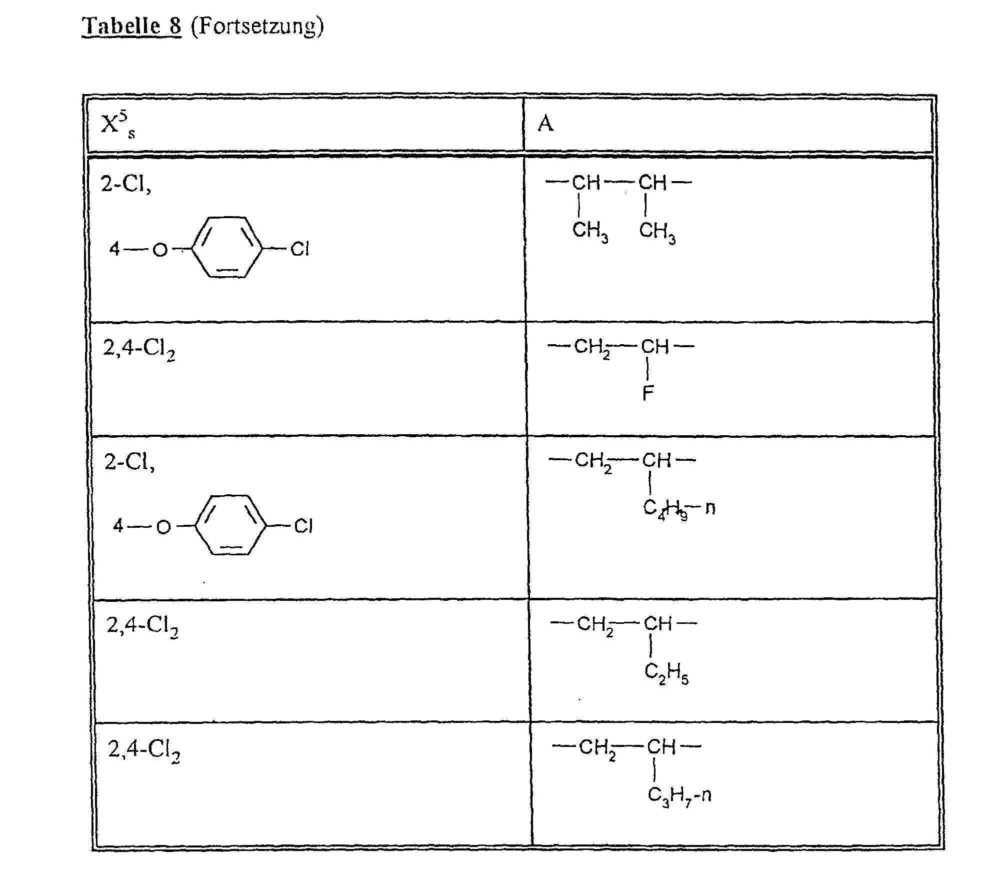

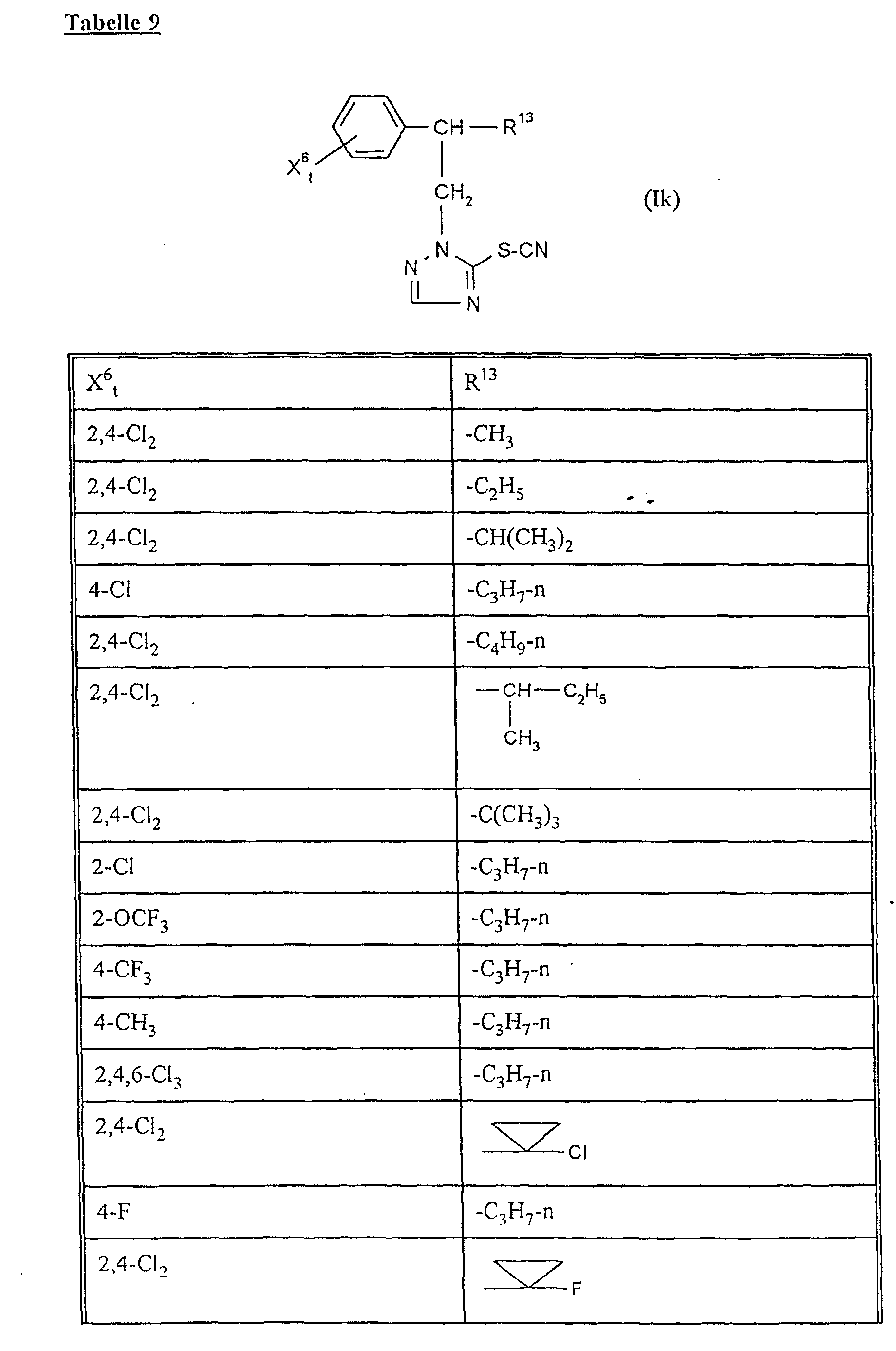

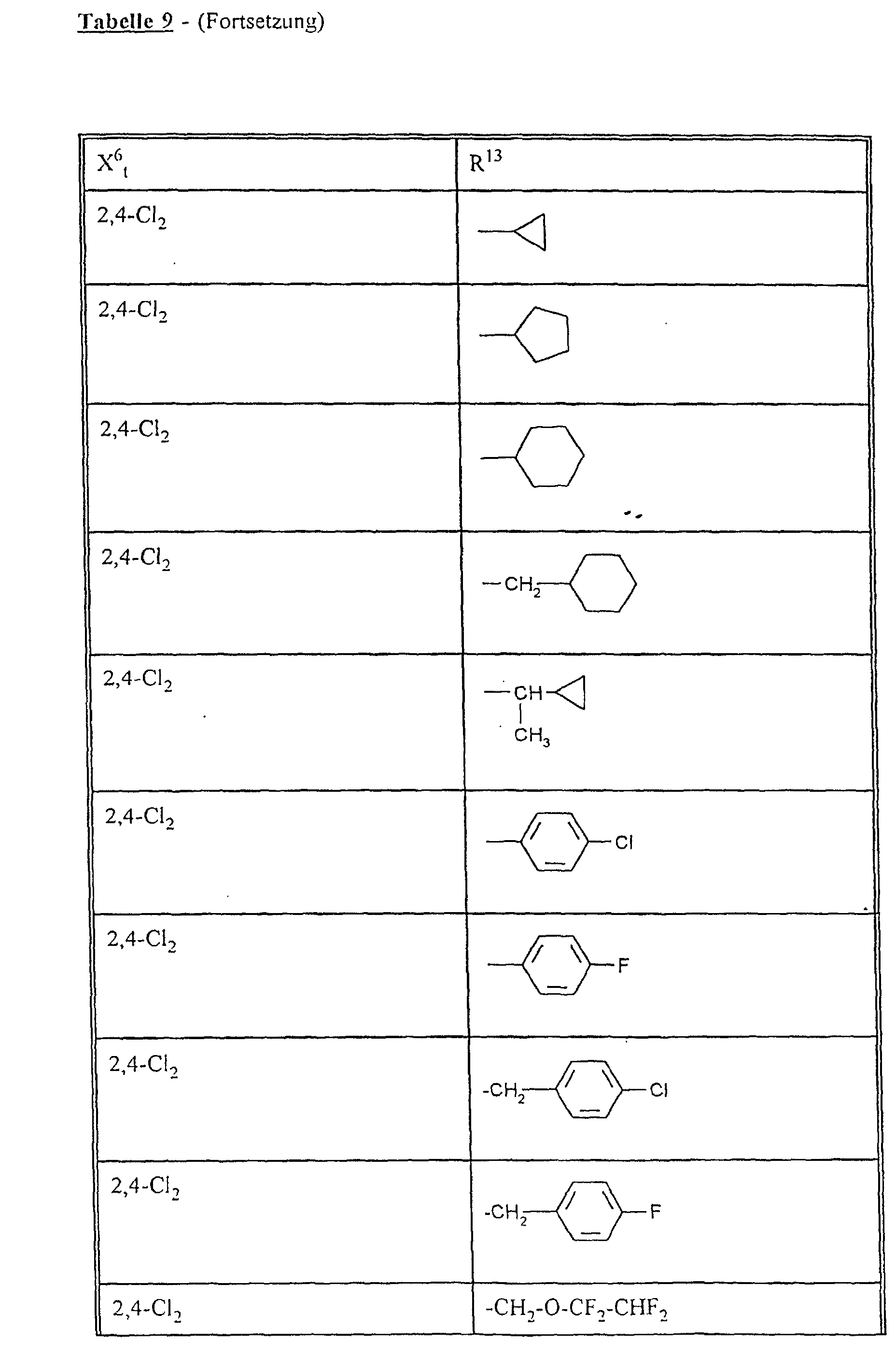

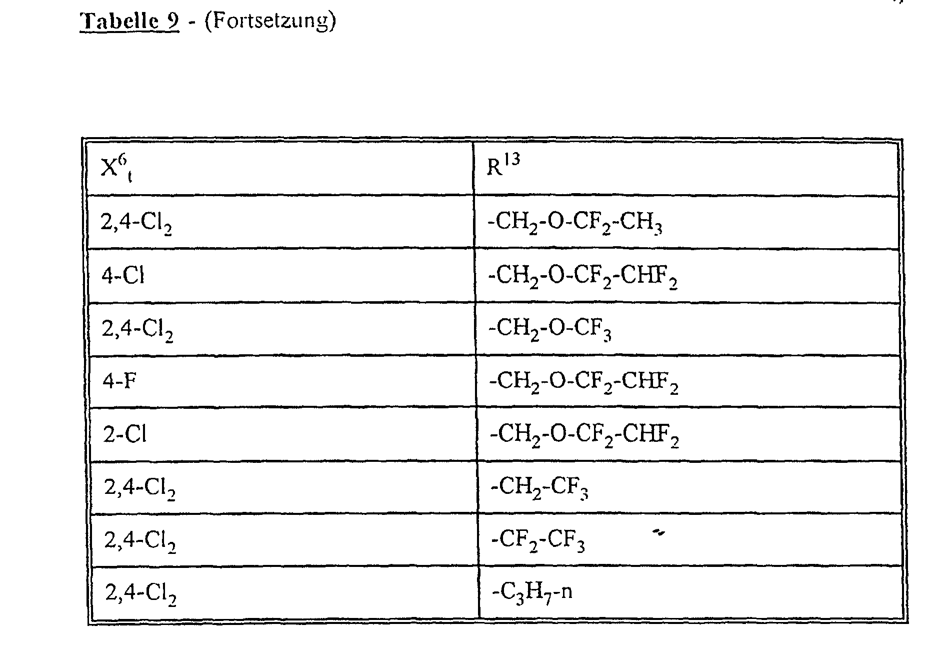

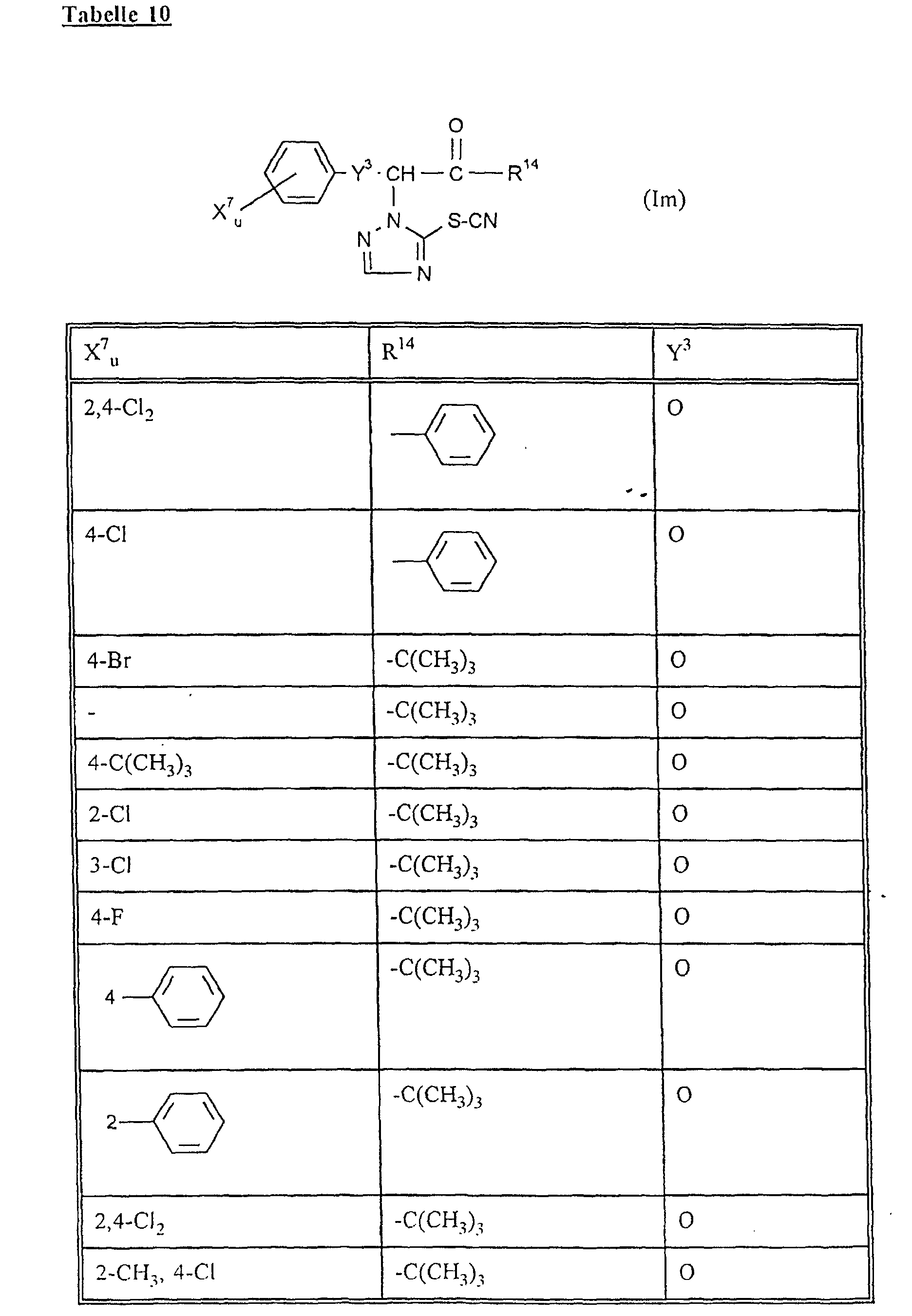

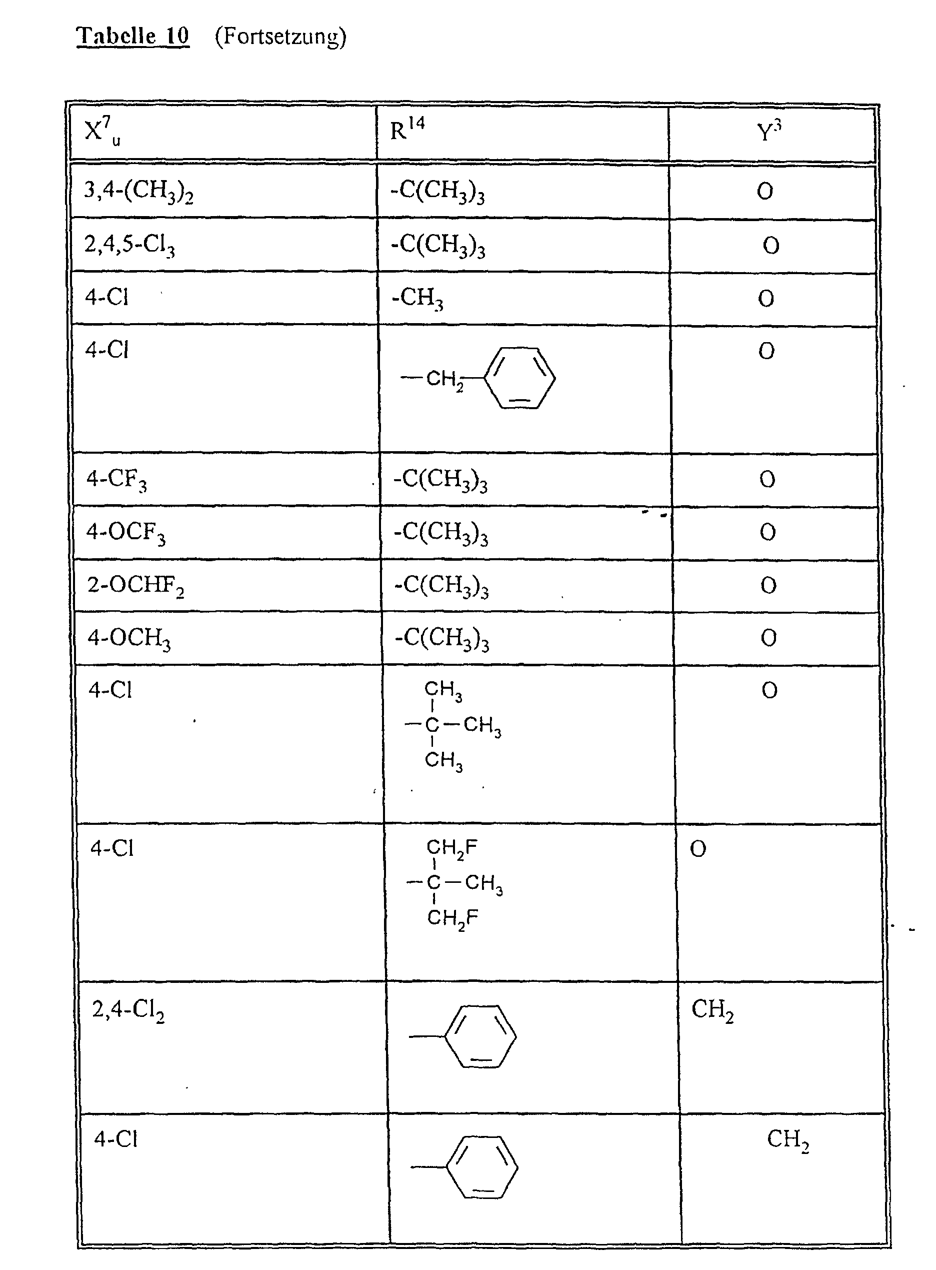

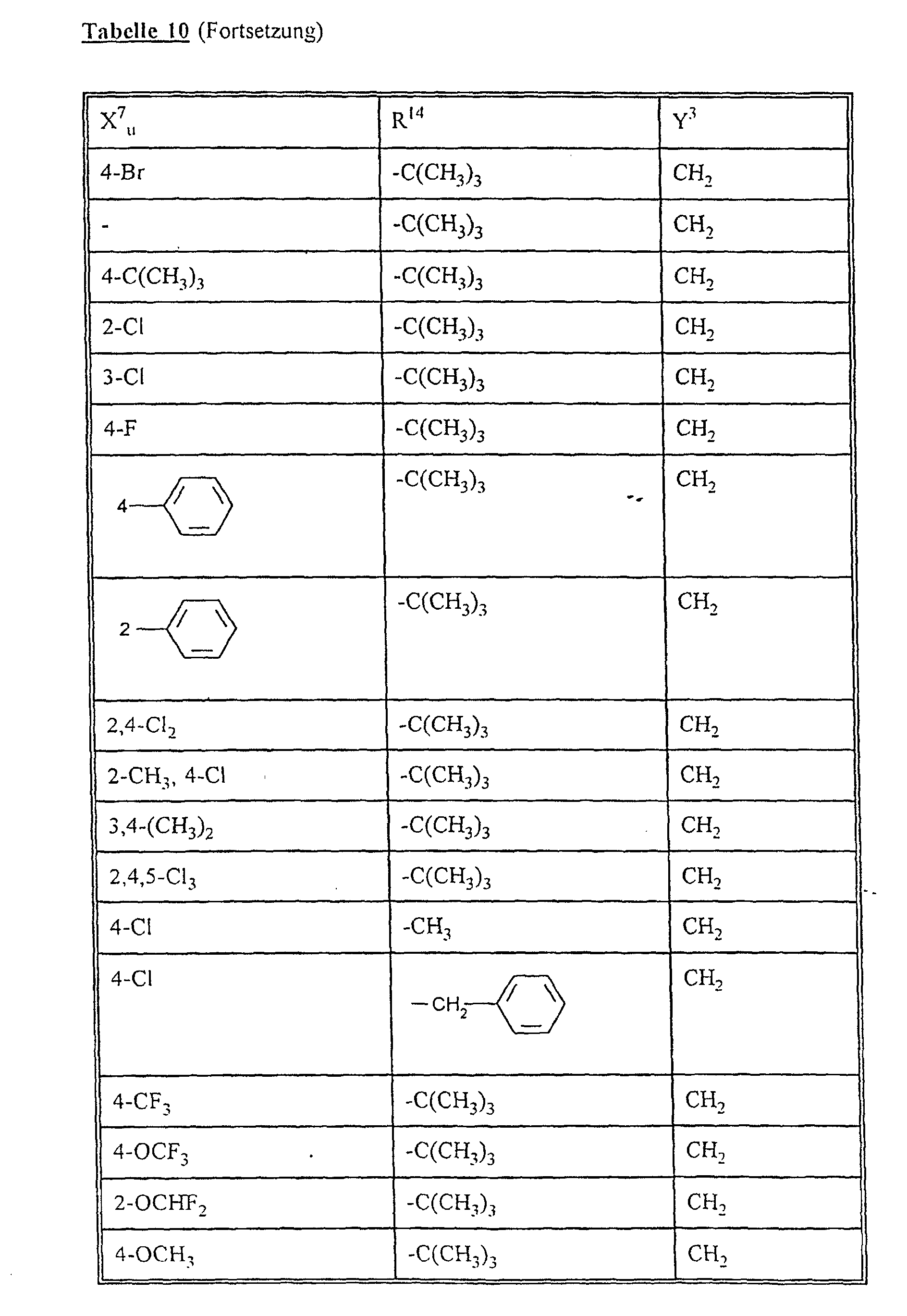

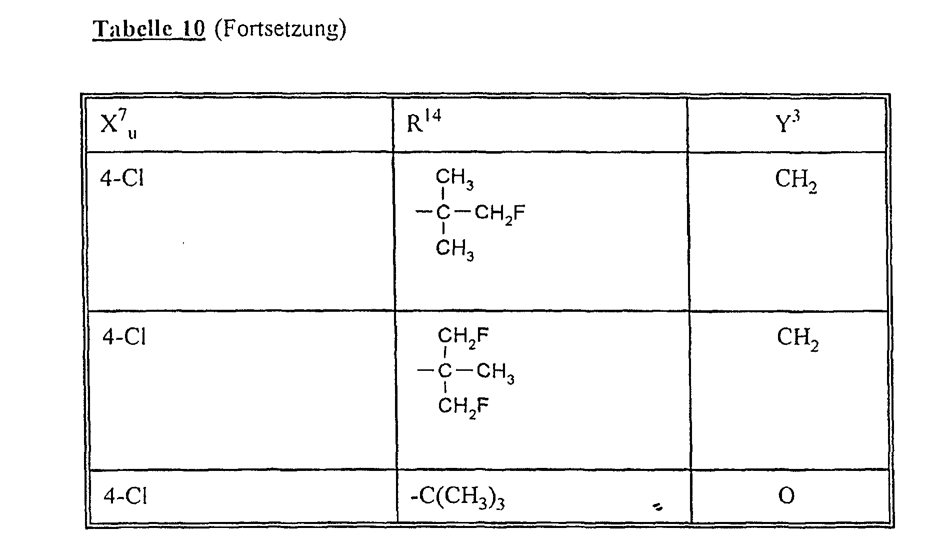

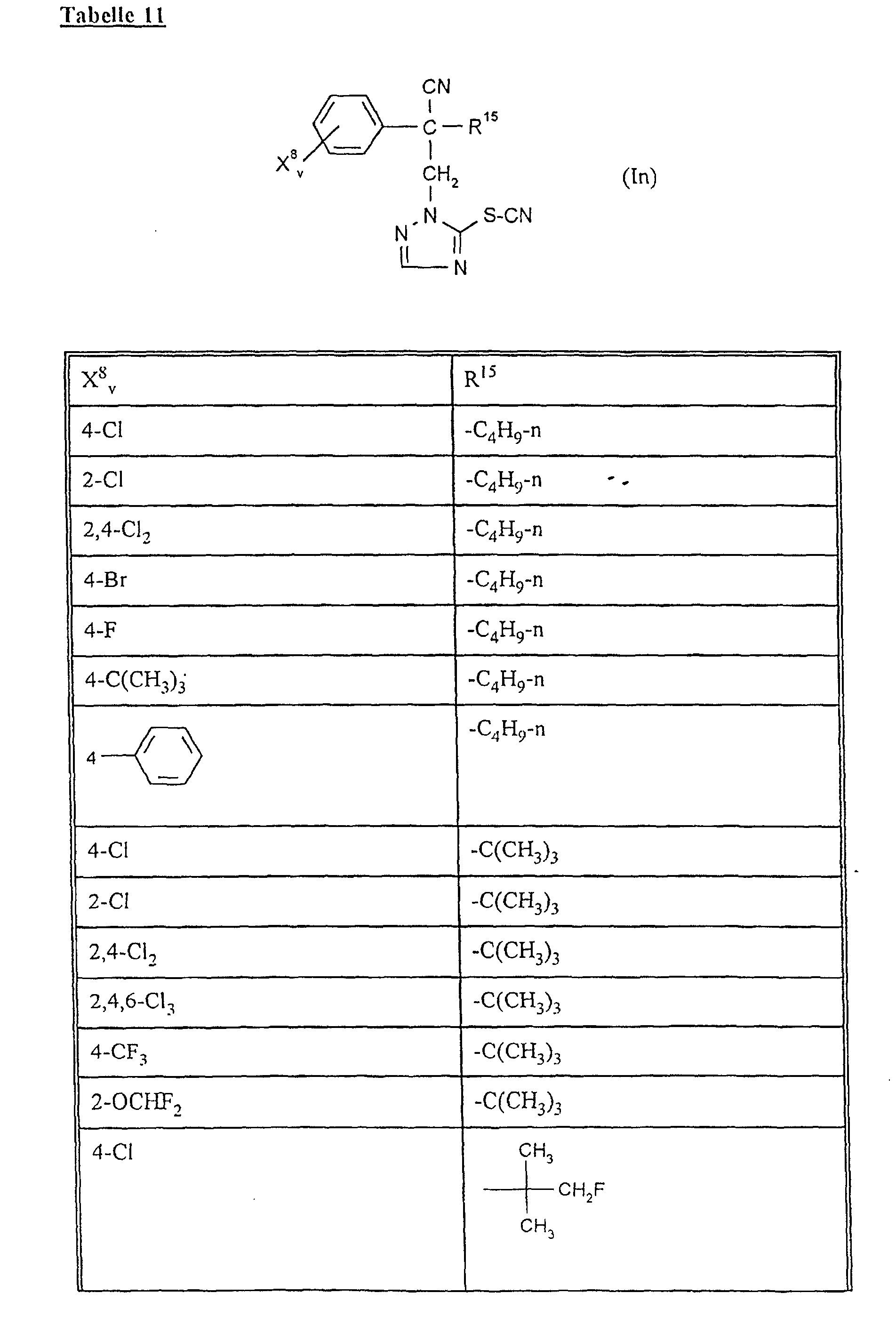

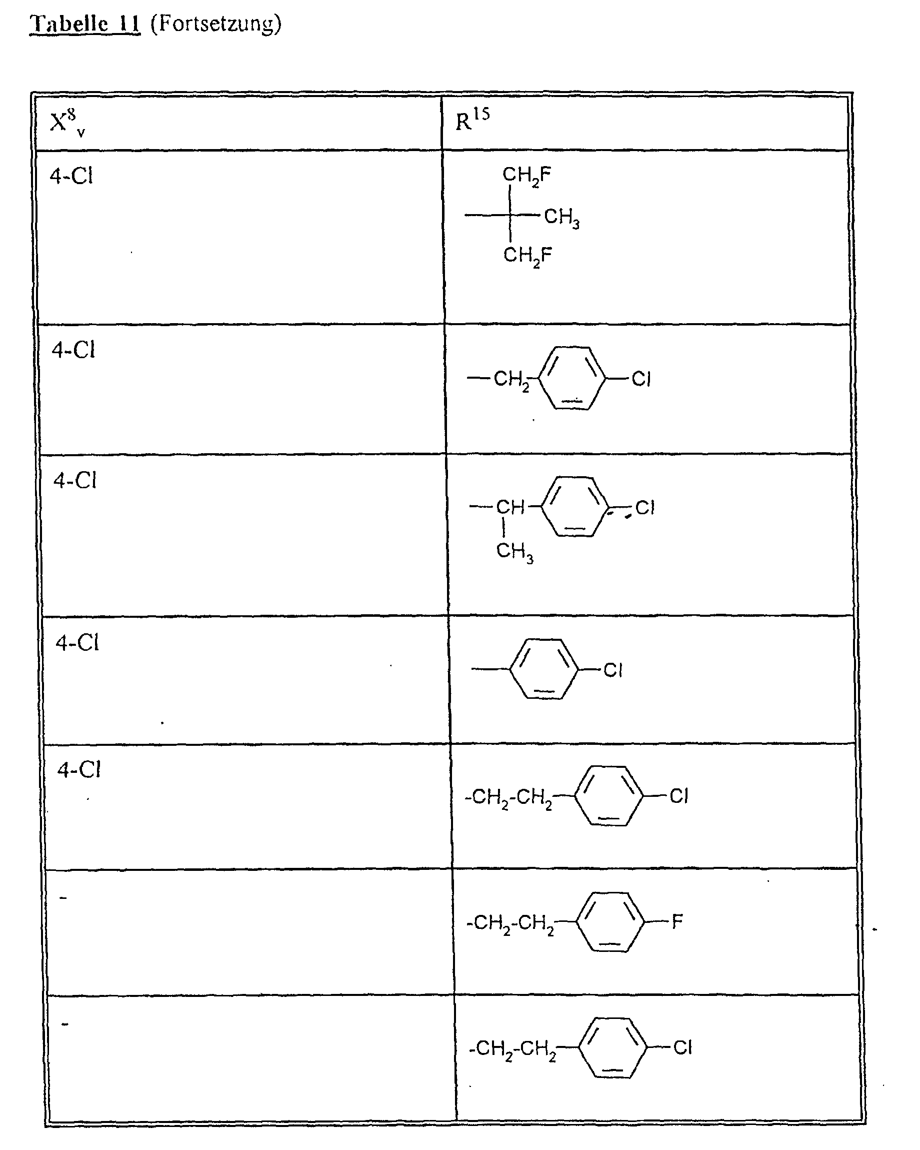

Verwendet man 2-(1-Chlor-cyclopropyl)-1-(2-chlor-phenyl)-3-(5-mercapto-1,2,4-triazol-1-yl)-propan-2-ol als Ausgangsstoff und frisch hergestelltes Chlorcyan als Reaktionskomponente, so kann der Verlauf des erfindungsgemäßen Verfahrens durch das folgende Formelschema veranschaulicht werden.

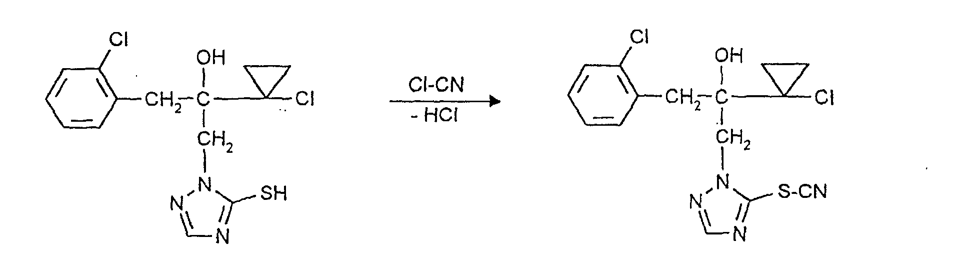

Die bei der Durchführung des erfindungsgemäßen Verfahrens als Ausgangsstoffe benötigten Mercapto-triazole sind durch die Formel (II) allgemein definiert. In dieser Formel hat R¹ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Mercapto-triazole der Formel (II) sind teilweise bekannt. Sie lassen sich herstellen, indem man Triazole der Formel 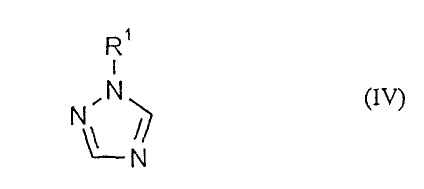 in welcher
- R¹: die oben angegebenen Bedeutungen hat,
entweder
α) nacheinander mit starken Basen und Schwefel in Gegenwart eines Verdünnungsmittels umsetzt und dann mit Wasser, gegebenenfalls in Gegenwart einer Säure hydrolysiert,
   oder
β) mit Schwefel in Gegenwart eines hoch siedenden Verdünnungsmittels umsetzt und dann gegebenenfalls mit Wasser sowie gegebenenfalls mit Säure behandelt.

Die bei der Durchführung des Verfahrens zur Herstellung von Mercapto-triazolen der Formel (II) als Ausgangsstoffe benötigten Triazole sind durch die Formel (IV) allgemein definiert. In dieser Formel hat R¹ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Triazole der Formel (IV) sind bekannt oder lassen sich nach bekannten Methoden herstellen (vgl. EP-A 0 015 756, EP-A 0 040 345, EP-A 0 052 424, EP-A 0 061 835, EP-A 0 297 345, EP-A 0 094 564, EP-A 0 196 038, EP-A 0 267 778, EP-A 0 378 953, EP-A 0 044 605, EP-A 0 069 442, EP-A 0 055 833, EP-A 0 301 393, DE-A 2 324 010, DE-A 2 737 489, DE-A 2 551 560, EP-A 0 065 485, DE-A 2 735 872, EP-A 0 234 242, DE-A 2 201 063, EP-A 0 145 294 und DE-A 3 721 786).

Als Basen kommen bei der Durchführung des obigen Verfahrens (α) zur Herstellung von Mercaptotriazolen der Formel (II) alle für derartige Reaktionen üblichen, starken Alkalimetall-Basen in Betracht. Vorzugsweise verwendbar sind n-Butyl-lithium, Lithium-diisopropyl-amid, Natriumhydrid, Natriumamid und auch Kalium-tert.-butylat im Gemisch mit Tetramethylethylen-diamin (= TMEDA).

Bei der Durchführung des obigen Verfahrens (α) zur Herstellung von Mercapto-triazolen der Formel (II) kommen alle für derartige Umsetzungen üblichen inerten organischen Solventien als Verdünnungsmittel in Betracht. Vorzugsweise verwendbar sind Ether, wie Tetrahydrofuran, Dioxan, Diethylether und 1,2-Dimethoxyethan, ferner flüssiger Ammoniak oder auch stark polare Solventien, wie Dimethylsulfoxid.

Schwefel wird sowohl bei der Durchführung des obigen Verfahrens (α) als auch des Verfahrens (β) vorzugsweise in Form von Pulver eingesetzt.

Zur Hydrolyse verwendet man bei der Durchführung des obigen Verfahrens (α) Wasser, gegebenenfalls in Gegenwart einer Säure. In Frage kommen hierbei alle für derartige Umsetzungen üblichen anorganischen oder organischen Säuren. Vorzugsweise verwendbar sind Essigsäure, verdünnte Schwefelsäure und verdünnte Salzsäure. Es ist jedoch auch möglich, die Hydrolyse mit wäßriger Ammoniumchlorid-Lösung durchzuführen.

Die Reaktionstemperaturen können bei der Durchführung des obigen Verfahrens (α) innerhalb eines bestimmten Bereiches variiert werden. Im allgemeinen arbeitet man bei Temperaturen zwischen -70°C und +20°C, vorzugsweise zwischen -70°C und 0°C.

Bei der Durchführung der obigen Verfahren (α) und (β) arbeitet man im allgemeinen unter Normaldruck. Es ist aber auch möglich, unter erhöhtem oder vermindertem Druck zu arbeiten. So kommt vor allem bei der Durchführung des Verfahrens (β) ein Arbeiten unter erhöhtem Druck in Frage.

Bei der Durchführung des obigen Verfahrens (α) setzt man auf 1 Mol an Triazol der Formel (IV) im allgemeinen 2 bis 3 Äquivalente, vorzugsweise 2,0 bis 2,5 Äquivalente, an starker Base und anschließend eine äquivalente Menge oder auch einen Überschuß an Schwefel ein. Die Umsetzung kann unter Schutzgasatmosphäre, z.B. unter Stickstoff oder Argon, vorgenommen werden. Die Aufarbeitung erfolgt nach üblichen Methoden. Im allgemeinen geht man so vor, daß man das Reaktionsgemisch mit einem in Wasser wenig löslichen organischen Solvens extrahiert, die vereinigten organischen Phasen trocknet und einengt und den verbleibenden Rückstand gegebenenfalls durch Umkristallisation und/oder Chromatographie reinigt.

Bei der Durchführung des obigen Verfahrens (β) kommen als Verdünnungsmittel alle für derartige Umsetzungen üblichen, hoch siedenden organischen Solventien in Betracht. Vorzugsweise verwendbar sind Amide, wie Dimethylformamid und Dimethylacetamid, außerdem heterocyclische Verbindungen, wie N-Methylpyrrolidon, und auch Ether, wie Diphenylether.

Bei der Durchführung des obigen Verfahrens (β) kann nach der Umsetzung gegebenenfalls eine Behandlung mit Wasser sowie gegebenenfalls mit Säure vorgenommen werden. Diese wird so durchgeführt, wie die Hydrolyse bei der Durchführung des Verfahrens (α).

Die Reaktionstemperaturen können auch bei der Durchführung des obigen Verfahrens (β) innerhalb eines größeren Bereiches variiert werden. Im allgemeinen arbeitet man bei Temperaturen zwischen 150°C und 300°C, vorzugsweise zwischen 180°C und 250°C.

Bei der Durchführung des obigen Verfahrens (β) setzt man auf 1 Mol Triazol der Formel (IV) im allgemeinen 1 bis 5 Mol, vorzugsweise 1,5 bis 3 Mol an Schwefel ein. Die Aufarbeitung erfolgt nach üblichen Methoden. Im allgemeinen geht man so vor, daß man das Reaktionsgemisch mit einem in Wasser nur wenig löslichen organischen Solvens extrahiert, die vereinigten organischen Phasen trocknet und einengt und den verbleibenden Rückstand gegebenenfalls nach üblichen Methoden, wie Umkristallisation oder Chromatographie, von eventuell vorhandenen Verunreinigungen befreit.

Bei der Durchführung des erfindungsgemäßen Verfahrens dient Chlorcyan der Formel (III) als Reaktionskomponente. Es wird zweckmäßigerweise in frisch hergestelltem Zustand eingesetzt. Dazu bereitet man es vorzugsweise in situ, indem man ein Salz der Cyanwasserstoffsäure, wie z.B. Kaliumcyanid, in Gegenwart einer Säure, wie z.B. Essigsäure, mit Chlorgas behandelt.

Als Verdünnungsmittel kommen bei der Durchführung des erfindungsgemäßen Verfahrens alle für derartige Umsetzungen üblichen Solventien in Betracht. Vorzugsweise verwendbar sind organische Säuren, wie z.B. Essigsäure.

Die Reaktionstemperaturen können bei der Durchführung des erfindungsgemäßen Verfahens innerhalb eines bestimmten Bereiches variiert werden. Im allgemeinen arbeitet man bei Temperaturen zwischen 0 und 60°C, vorzugsweise zwischen 10 und 40°C.

Bei der Durchführung des erfindungsgemäßen Verfahrens arbeitet man im allgemeinen unter Atmosphärendruck. Es ist aber auch möglich, unter erhöhtem Druck zu arbeiten.

Bei der Durchführung des erfindungsgemäßen Verfahrens setzt man auf 1 mol an Mercapto-Triazol der Formel (II) im allgemeinen 1 bis 3 mol an Chlorcyan der Formel (III) ein. Letzteres wird vorzugsweise in situ erzeugt, indem man ein Alkalimetallsalz der Cyanwasserstoffsäure, wie z.B. Natrium- oder Kaliumcyanid, in Gegenwart einer organischen Säure, wie z.B. Essigsäure, mit Chlorgas behandelt. Die Aufarbeitung erfolgt nach üblichen Methoden. Im allgemeinen verfährt man in der Weise, daß man das Reaktionsgemisch nach beendeter Umsetzung mit einem mit Wasser wenig mischbaren organischen Solvens verdünnt, mehrfach mit wäßrig alkalischer Lösung extrahiert, die organische Phase dann trocknet und unter vermindertem Druck einengt. Das erhaltene Produkt kann gegebenenfalls nach üblichen Methoden, z.B. durch Umkristallisation oder Chromatographie, von noch vorhandenen Verunreinigungen befreit werden.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Pseudocercosporella, Erysiphe- und Fusarium-Arten. Außerdem lassen sich die erfindungsgemäßen Stoffe sehr gut gegen Venturia und Sphaerotheca einsetzen. Sie besitzen darüber hinaus auch eine sehr gute in-vitro Wirkung.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder. schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Äluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Verwendung im Pflanzenschutz als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen beispielsweise die folgenden Stoffe infrage.

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino [alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Technazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
Quinalphos,
RH 5992,

Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung der erfindungsgemäßen Stoffe gehen aus den folgenden Beispielen hervor.

### Herstellungsbeispiele

### Beispiel 1

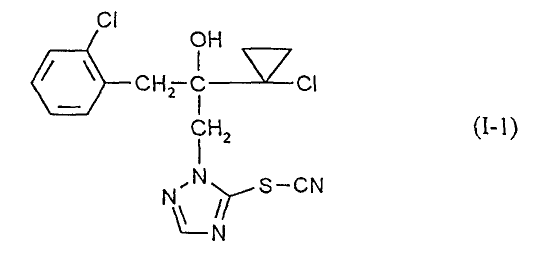

In ein Gemisch aus 1,72 g (5 mmol) 2-(1-Chlor-cyclopropyl)-1-(2-chlor-phenyl)-3-(5-mercapto-1,2,4-triazol-1-yl)-propan-2-ol und 0,65 g (10 mmol) Kaliumcyanid in 20 ml Essigsäure wird bei 20°C Chlorgas eingeleitet. Das Reaktionsgemisch wird 20 Stunden bei 20°C gerührt und dann mit Dichlormethan verdünnt. Danach schüttelt man mehrfach mit verdünnter, wäßriger Natronlauge aus, trocknet die organische Phase über Natriumsulfat und engt unter vermindertem Druck ein. Das verbleibende Produkt wird mit Petrolether/Essigsäureethylester = 1:1 als Laufmittel an Kieselgel gereinigt. Nach dem Einengen des Eluates erhält man 1,2 g (64 % der Theorie) an 2-(1-Chlor-cyclopropyl)-1-(2-chlor-phenyl)-3-(5-thiocyano-1,2,4-triazol-1-yl)-propan-2-ol in Form eines Öls.
¹H-NMR-Spektrum (400 MHz; CDCl₃; TMS):
δ = 0,5 - 0,7 (m,4H); 3,2 (d,1H); 3,6 (d,1H); 3,65 (OH); 4,2 (d,1H); 4,95 (d,1H); 7,2 - 7,55 (m,4H); 8,1 (s,1H) ppm.

### Herstellung von Ausgangssubstanzen:

### Beispiel 2

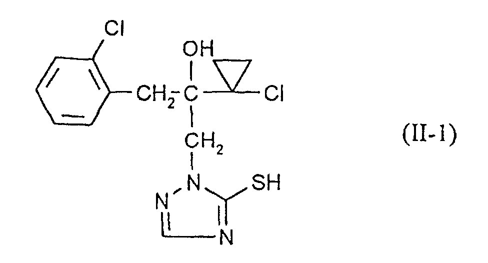

### Variante α:

Ein Gemisch aus 3,12 g (10 mMol) 2-(1-Chlor-cyclopropyl)-1-(2-chlorphenyl)-3-(1,2,4-triazol-1-yl)-propan-2-ol und 45 ml absolutem Tetrahydrofuran wird bei -20°C mit 8,4 ml (21 mMol) n-Butyl-lithium in Hexan versetzt und 30 Minuten bei 0°C gerührt. Danach wird das Reaktionsgemisch auf -70°C abgekühlt, mit 0,32 g (10 mMol) Schwefel-Pulver versetzt und 30 Minuten bei -70°C gerührt. Es wird auf -10°C erwärmt, mit Eiswasser versetzt und durch Zugabe von verdünnter Schwefelsäure auf einen pH-Wert von 5 eingestellt. Man extrahiert mehrfach mit Essigsäureethylester, trocknet die vereinigten organischen Phasen über Natriumsulfat und engt unter vermindertem Druck ein. Man erhält auf diese Weise 3,2 g (93 % der Theorie) an 2-(1-Chlor-cyclopropyl)-1-(2-chlorphenyl)-3-(5-mercapto-1,2,4-triazol-1-yl)-propan-2-ol in Form einer Festsubstanz, die nach Umkristallisation bei 138-139°C schmilzt.

### Variante β:

Ein Gemisch aus 3,12 g (10 mmol) 2-(1-Chlor-cyclopropyl)-l-(2-chlor-phenyl)-3-(1,2,4-triazol-1-yl)-propan-2-ol, 0,96 g (30 mmol) Schwefel-Pulver und 20 ml absolutem N-Methyl-pyrrolidon wird unter Rühren 44 Stunden auf 200°C erhitzt. Anschließend wird das Reaktionsgemisch unter vermindertem Druck (0,2 mbar) eingeengt. Das dabei anfallende Rohprodukt (3,1 g) wird aus Toluol umkristallisiert. Man erhält auf diese Weise 0,7 g (20 % der Theorie) an 2-(1-Chlor-cyclopropyl)-1-(2-chlorphenyl)-3-(5-mercapto-1,2,4-triazol-1-yl)-propan-2-ol in Form einer Festsubstanz vom Schmelzpunkt 138-139°C.

Nach der im Beispiel 2 angegebenen Methode werden auch die in der folgenden Tabelle 12 aufgeführten Verbindungen hergestellt.

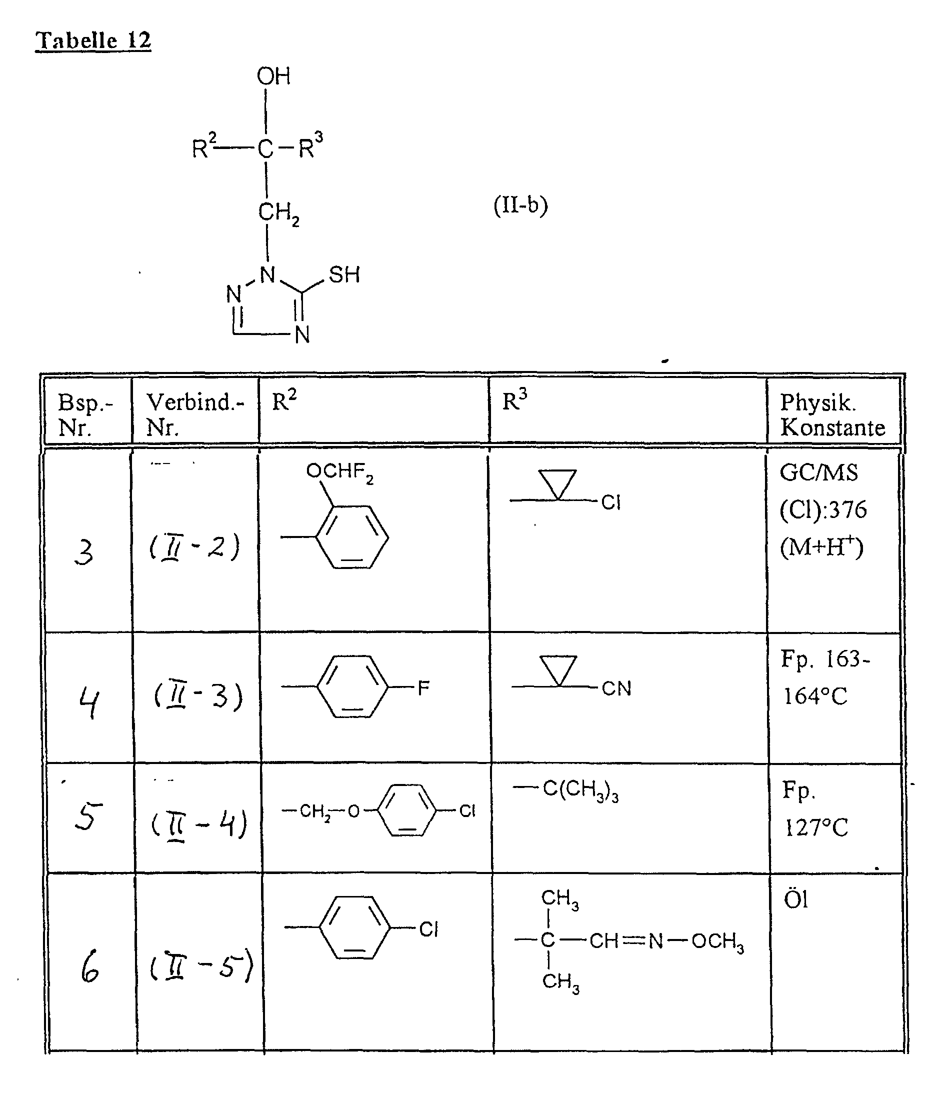

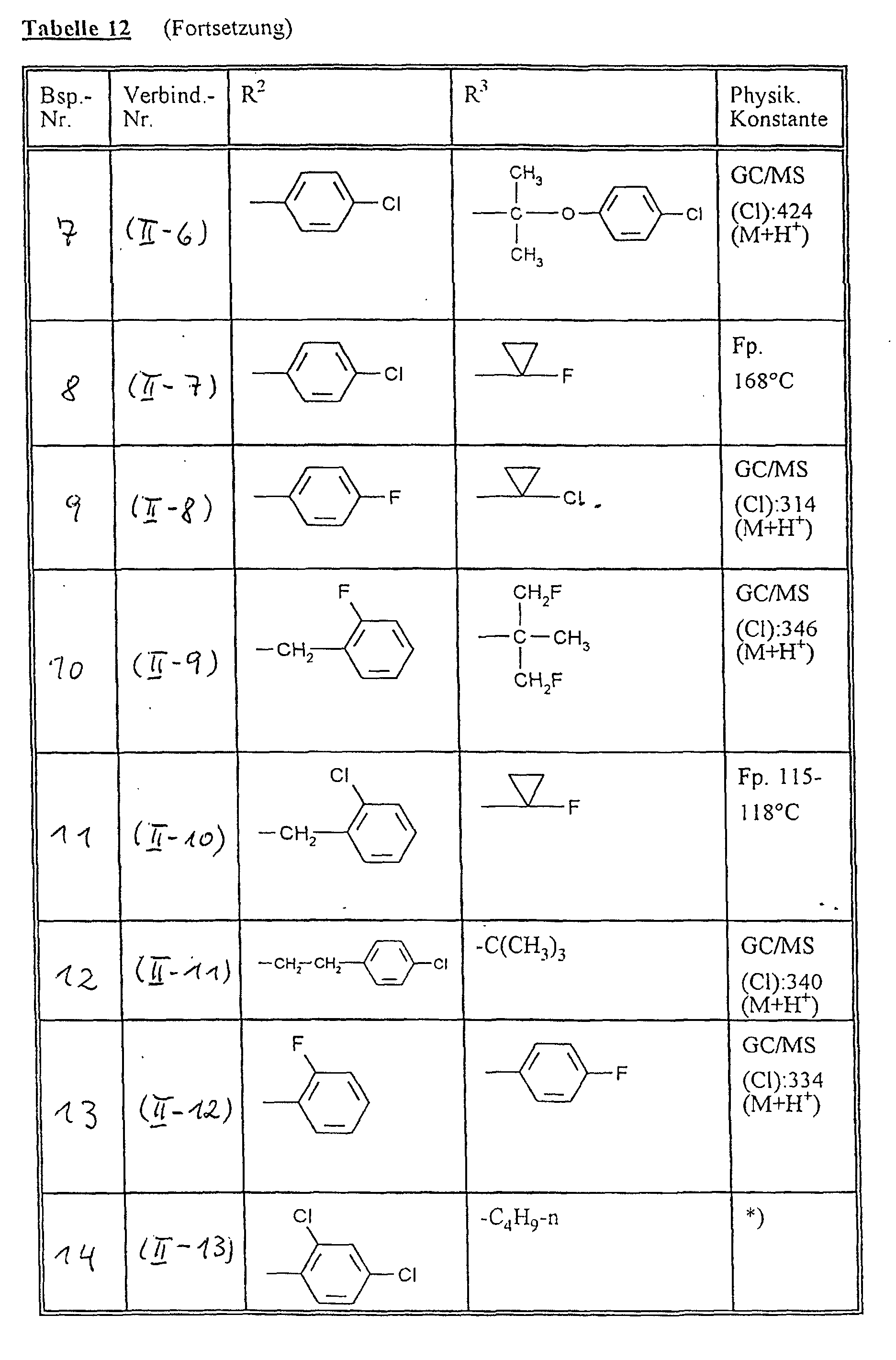

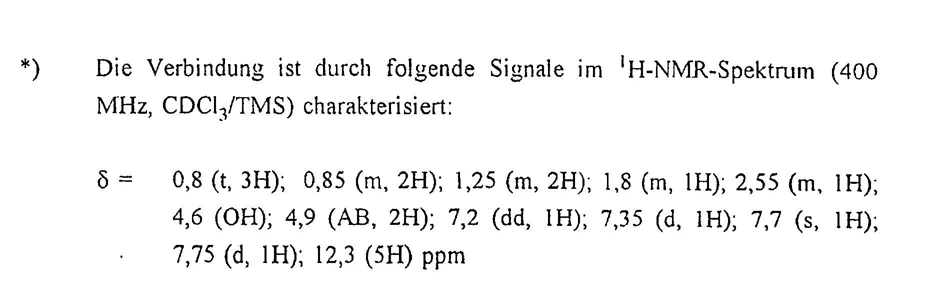

### Beispiel 15

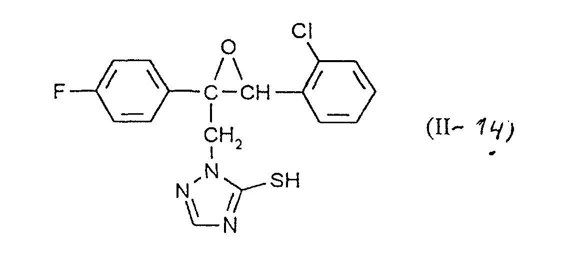

Ein Gemisch aus 1,3 g (4 mmol) 3-(2-Chlor-phenyl)-2-(4-fluor-phenyl)-2-(1,2,4-triazol-1-yl-methyl)-oxiran (Z-Form) und 25 ml absolutem Tetrahydrofuran wird bei -70°C mit 2,0 ml (5 mmol) n-Butyl-lithium in Hexan versetzt und 1 Stunde bei -70°C gerührt. Danach wird das Reaktionsgemisch mit 0,16 g (5 mmol) Schwefel-Pulver versetzt und 4 Stunden bei -70°C gerührt. Anschließend werden bei -70°C gleichzeitig 1 ml Methanol und 1 ml Essigsäure unter Rühren zugetropft. Man verdünnt das entstehende Gemisch mit Dichlormethan und schüttelt mehrfach mit gesättigter, wäßriger Ammoniumchlorid-Lösung aus. Die organische Phase wird über Natriumsulfat getrocknet und dann unter vermindertem Druck eingeengt. Das anfallende Rohprodukt (1,9 g), das gemäß Gaschromatogramm neben 20,7 % an Ausgangssubstanz 51,0 % an dem gewünschten Produkt enthält, wird aus Toluol umkristallisiert. Man erhält auf diese Weise 0,8 g (55 % der Theorie) an 3-(2-Chlorphenyl)-2-(4-fluor-phenyl)-2-(5-mercapto-1,2,4-triazol-1-yl-methyl)-oxiran (Z-Form) als Festsubstanz vom Schmelzpunkt 179 bis 180°C.
¹H-NMR-Spektrum (200 MHz, CDCl₃, TMS):
δ = 3,7 (d, J=15 Hz, 1H); 4,1 (s, 1H); 5,15 (d, J=15 Hz, 1H); 6,95-7,6 (m, 8H); 7,65 (s, 1H); 11,0 (s, 1H) ppm.
GC/MS (ci): 362 (M+H⁺)

### Beispiel 16

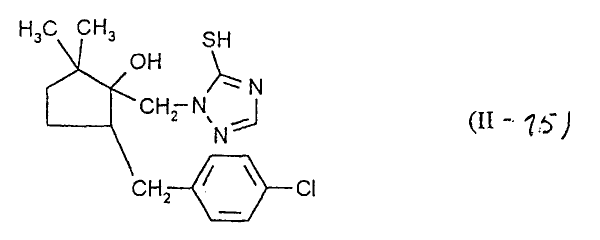

Ein Gemisch aus 1,6 g (5 mmol) 5-(4-Chlorbenzyl)-2,2-dimethyl-1-(1,2,4-triazol-1-yl-methyl)-cyclopentan-1-ol (Z-Form) und 30 ml absolutem Tetrahydrofuran wird bei -20°C mit 4 ml (10 mmol) n-Butyl-lithium in Hexan versetzt und 30 Minuten bei 0°C nachgerührt. Anschließend wird das Reaktionsgemisch auf -70°C abgekühlt, unter Rühren mit 0,19 g (6 mmol) Schwefel-Pulver versetzt, dann 1 Stunde bei -70°C und danach 2 Stunden bei 0°C gerührt, Man verdünnt das entstehende Gemisch mit Essigsäureethylester und schüttelt mehrfach mit gesättigter, wäßriger Ammoniumchlorid-Lösung aus. Die organische Phase wird über Natriumsulfat getrocknet und dann unter vermindertem Druck eingeengt. Das anfallende Rohprodukt (2,0 g) wird aus Toluol umkristallisiert. Man erhält auf diese Weise 1,1 g (63 % der Theorie) an 5-(4-Chlor-benzyl)-2,2-dimethyl-1-(5-mercapto-1,2,4-triazol-1-yl-methyl)-cyclopentan-1-ol (Z-Form) als Festsubstanz vom Schmelzpunkt 179 bis 180°C.
GC/MS (ci): 352 (M+H⁺)

### Beispiel 17

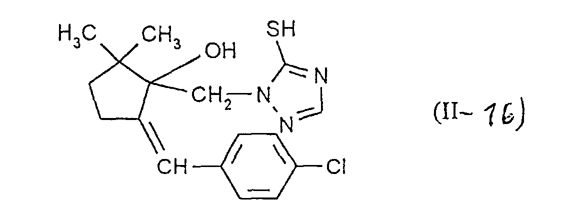

Ein Gemisch aus 1,59 g (5 mmol) 2-(4-Chlor-benzyliden)-5,5-dimethyl-1-(1,2,4-triazol-1-yl-methyl)-cyclopentan-1-ol und 30 ml absolutem Tetrahydrofuran wird bei -20°C mit 4,4 ml (11 mmol) n-Butyl-lithium in Hexan versetzt und 30 Minuten bei 0°C nachgerührt. Anschließend wird das Reaktionsgemisch auf -70°C abgekühlt, unter Rühren mit 0,19 g (6 mmol) Schwefel-Pulver versetzt, dann 1 Stunde bei -70°C und danach 2 Stunden bei 0°C gerührt. Man verdünnt das entstehende Gemisch mit Essigsäureethylester und schüttelt mehrfach mit gesättigter, wäßriger Ammoniumchlorid-Lösung aus. Die organische Phase wird über Natriumsulfat getrocknet und dann unter vermindertem Druck eingeengt. Das anfallende Rohprodukt (1,9 g) wird mit Essigsäureethylester an Kieselgel chromatographiert. Man erhält auf diese Weise 0,8 g (46 % der Theorie) an 2-(4-Chlor-benzyliden)-5,5-dimethyl-1-(5-mercapto-1,2,4-triazol-1-yl-methyl)-cyclopentan-1-ol.
¹H-NMR-Spektrum (200 MHz, CDCl₃; TMS):
δ = 0,9 (s,3H); 1,15 (s,3H); 1,6-1,95 (m,2H); 2,4-3,0 (m,2H); 4,25 (d,1H); 4,55 (d,1H); 5,9 (m,1H); 7,1-7,3 (m,4H); 7,6 (s,1H) ppm

### Beispiel 18

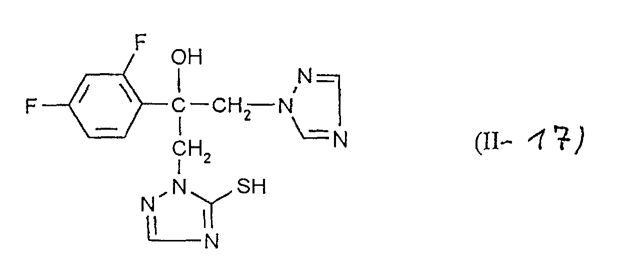

Ein Gemisch aus 1,53 g (5 mmol) 2-(2,4-Difluor-phenyl)-1,3-bis-(1,2,4-triazol-1-yl)-propan-2-ol und 30 ml absolutem Tetrahydrofuran wird bei -20°C mit 4,4 ml (11 mmol) n-Butyl-lithium in Hexan versetzt und 30 Minuten bei 0°C nachgerührt. Anschließend wird das Reaktionsgemisch auf -70°C abgekühlt, unter Rühren mit 0,19 g (6 mmol) Schwefel-Pulver versetzt, dann 1 Stunde bei -70°C und danach 2 Stunden bei 0°C gerührt. Man verdünnt das entstehende Gemisch mit Essigsäureethylester und schüttelt mehrfach mit gesättigter, wäßriger Ammoniumchlorid-Lösung aus. Die organische Phase wird über Natriumsulfat getrocknet und dann unter vermindertem Druck eingeengt. Das anfallende Rohprodukt (2,3 g) wird durch Chromatographie an Kieselgel mit einem Gemisch aus Ethylacetat und Ethanol = 9:1 als Laufmittel gereinigt. Man erhält auf diese Weise 1,0 g (59 % der Theorie) an 2-(2,4-Difluor-phenyl)-1-(5-mercapto-1,2,4-triazol-1-yl)-3-(1,2,4-triazol-1-yl)-propan-2-ol in Form einer Festsubstanz vom Schmelzpunkt 187°C.
GC/MS (ci): 339 (M+H⁺)

### Beispiel 19

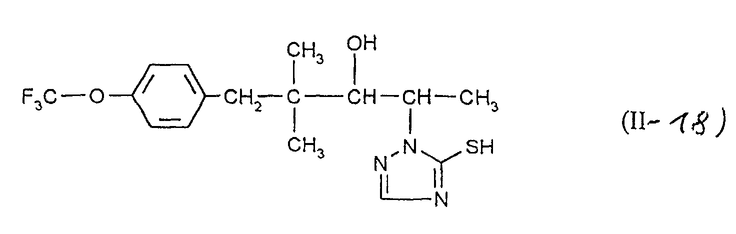

Ein Gemisch aus 1,72 g (5 mmol) 2,2-Dimethyl-3-hydroxy-4-(1,2,4-triazol-1-yl)-1-(4-trifluormethoxy-phenyl)-pentan und 30 ml absolutem Tetrahydrofuran wird bei -20°C mit 4,4 ml (11 mmol) n-Butyl-lithium in Hexan versetzt und 30 Minuten bei 0°C nachgerührt. Anschließend wird das Reaktionsgemisch auf -70°C abgekühlt, unter Rühren mit 0,19 g (6 mmol) Schwefel Pulver versetzt, dann 1 Stunde bei -70°C und danach 2 Stunden bei 0°C gerührt. Man verdünnt das entstehende Gemisch mit Ethylacetat und schüttelt mehrfach mit gesättigter, wäßriger Ammoniumchlorid-Lösung aus. Die organische Phase wird über Natriumsulfat getrocknet und dann unter vermindertem Druck eingeengt. Das anfallende Rohprodukt (2,2 g) wird durch Chromatographie an Kieselgel mit einem Gemisch aus Petrolether und Ethylacetat = 1:1 als Laufmittel gereinigt. Man erhält auf diese Weise 1,4 g (75 % der Theorie) an 2,2-Dimethyl-3-hydroxy-4-(5-mercapto-1,2,4-triazol-1-yl)-1-(4-trifluormethoxy-phenyl)-pentan in Form einer Festsubstanz vom Schmelzpunkt 125 bis 126°C.
GC/MS(ci): 376 (M + H⁺)

### Beispiel 20

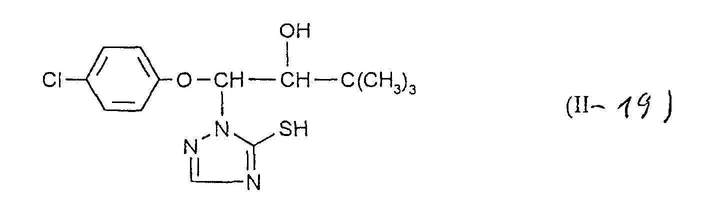

Ein Gemisch aus 1,48 g (5 mmol) 1-(4-Chlor-phenoxy)-1-(1,2,4-triazol-1-yl)-3,3-dimethyl-butan-2-ol und 30 ml absolutem Tetrahydrofuran wird bei -20°C mit 4 ml (10 mmol) n-Butyl-lithium in Hexan versetzt und 30 Minuten bei -20°C nachgerührt. Anschließend wird das Reaktionsgemisch bei -20°C unter Rühren mit 0,19 g (6 mmol) Schwefel-Pulver versetzt, dann 1 Stunde bei -20°C und danach 2 Stunden bei 0°C gerührt. Man verdünnt das entstehende Gemisch mit Ethylacetat und schüttelt mehrfach mit gesättigter, wäßriger Ammoniumchlorid-Lösung aus. Die organische Phase wird über Natriumsulfat getrocknet und dann unter vermindertem Druck eingeengt. Das anfallende Rohprodukt (1,9 g) wird durch Chromatographie an Kieselgel mit einem Gemisch aus Petrolether und Essigsäureethylester = 1:1 als Laufmittel gereinigt. Man erhält auf diese Weise 0,7 g (43 % der Theorie) an 1-(4-Chlorphenoxy)-1-(5-mercapto-1,2,4-triazol-1-yl)-3,3-dimethyl-butan-2-ol in Form einer Festsubstanz vom Schmelzpunkt 193 bis 194°C.
MS(ci): 328 (M + H⁺)

### Beispiel 21

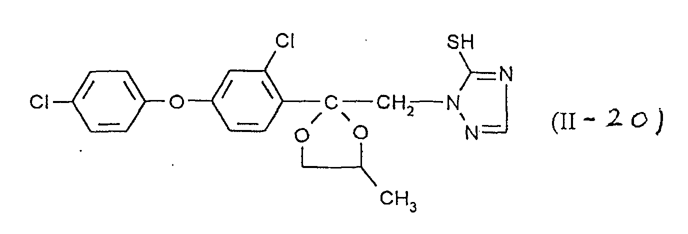

Ein Gemisch aus 2,0 g (5 mmol) 2-[2-Chlor-4-(4-chlor-phenoxy)-phenyl]-2-(1,2,4-triazol-1-yl-methyl)-4-methyl-1,3-dioxolan, 0,32 g (10 mmol) Schwefel-Pulver und 10 ml absolutem N-Methylpyrrolidon wird unter Rühren 22 Stunden auf 200°C erhitzt. Anschließend wird das Reaktionsgemisch unter vermindertem Druck (0,2 mbar) eingeengt. Der verbleibende Rückstand wird mit Essigsäureethylester versetzt, und das entstehende Gemisch wird mehrfach mit gesättigter, wäßriger Ammoniumchlorid-Lösung ausgeschüttelt. Die organische Phase wird über Natriumsulfat getrocknet und dann unter vermindertem Druck eingeengt. Das anfallende Rohprodukt (1,8 g) wird durch Chromatographie an Kieselgel mit einem Gemisch aus Petrolether und Ethylacetat = 1:1 als Laufmittel gereinigt. Man erhält auf diese Weise 0,9 g (41 % der Theorie) an 2-[2-Chlor-4-(4-chlorphenoxy)-phenyl]-2-[(5-mercapto-1,2,4-triazol-1-yl)-methyl]-4-methyl-1,3-dioxolan in Form eines Isomerengemisches.
MS (ci): 438 (M + H⁺, 100 %)

### Beispiel 22

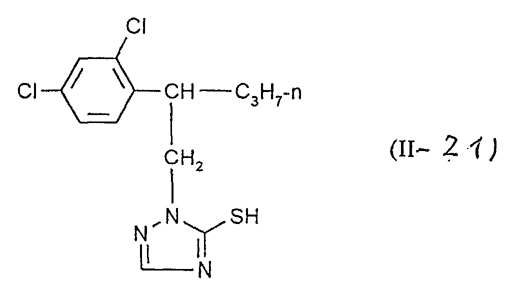

Ein Gemisch aus 1,42 g (5 mmol) 2-(2,4-Dichlor-phenyl)-1-(1,2,4-triazol-1-yl)-pentan, 0,32 g (10 mmol) Schwefel-Pulver und 10 ml absolutem N-Methylpyrrolidon wird unter Stickstoffatmosphäre und unter Rühren 3 Stunden auf 200°C erhitzt. Anschließend wird das Reaktionsgemisch unter vermindertem Druck eingeengt. Der verbleibende Rückstand wird mit Essigsäureethylester versetzt, und das entstehende Gemisch wird mehrfach mit gesättigter, wäßriger Ammoniumchlorid-Lösung ausgeschüttelt. Die organische Phase wird über Natriumsulfat getrocknet und dann unter vermindertem Druck eingeengt. Das anfallende Rohprodukt (2,1 g) wird durch Chromatographie an Kieselgel mit einem Gemisch aus Petrolether und Essigsäureethylester = 1:1 als Laufmittel gereinigt. Man erhält auf diese Weise 1,5 g (95 % der Theorie) an 2-(2,4-Dichlor-phenyl)-1-(5-mercapto-1,2,4-triazol-1-yl)-pentan in Form einer Festsubstanz vom Schmelzpunkt 103°C.

### Beispiel 23

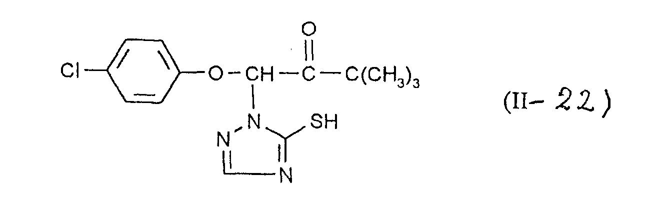

Ein Gemisch aus 2,93 g (10 mmol) l-(4-Chlor-phenoxy)-1-(1,2,4--triazol-1-yl)-3,3-dimethyl-butan-2-on, 0,64 g (20 mmol) Schwefel-Pulver und 10 ml absolutem N-Methylpyrrolidon wird unter Stickstoff-Atmosphäre und unter Rühren 8 Stunden auf 200°C erhitzt. Anschließend wird das Reaktionsgemisch unter vermindertem Druck eingeengt, und der verbleibende Rückstand wird in Dichlormethan gelöst.

Das entstehende Gemisch wird mehrfach mit gesättigter, wäßriger Ammoniumchlorid-Lösung ausgeschüttelt.

Die organische Phase wird über Natriumsulfat getrocknet und dann unter vermindertem Druck eingeengt. Das anfallende Rohprodukt (2,7 g) wird durch Chromatographie an Kieselgel mit einem Gemisch aus Petrolether und Essigsäureethylester = 1:1 als Laufmittel gereinigt. Man erhält auf diese Weise 2,0 g (62% der Theorie) an 1-(4-Chlorphenoxy)-1-(5-mercapto-1,2,4-triazol-1-yl)-3,3-dimethylbutan-2-on in Form einer Festsubstanz vom Schmelzpunkt 134 bis 136°C.

### Beispiel 24

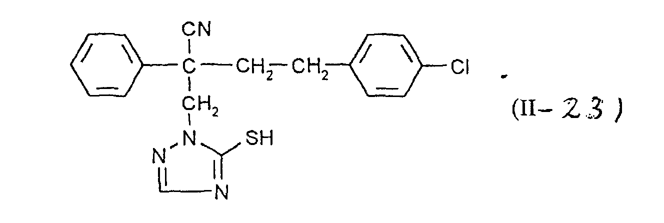

Ein Gemisch aus 1,68 g (5 mmol) 4-(4-Chlor-phenyl)-2-cyano-2-phenyl-1-(1,2,4-triazol-1-yl)-butan, 0,32 g (10 mmol) Schwefel-Pulver und 10 ml absolutem N-Methylpyrrolidon wird unter Stickstoff-Atmosphäre und unter Rühren 47 Stunden auf 200°C erhitzt. Anschließend wird das Reaktionsgemisch unter vermindertem Druck eingeengt, und der verbleibende Rückstand wird in Essigsäure-ethylester gelöst. Das entstehende Gemisch wird mehrfach mit gesättigter, wäßriger Ammoniumchlorid-Lösung ausgeschüttelt. Die organische Phase wird über Natriumsulfat getrocknet und dann unter vermindertem Druck eingeengt. Das anfallende Rohprodukt (1,9 g) wird durch Chromatographie an Kieselgel mit einem Gemisch aus Petrolether und Essigsäureethylester = 1:1 als Laufmittel gereinigt. Man erhält auf diese Weise 0,7 g (38 % der Theorie) an 4-(4-Chlorphenyl)-2-cyano-2-phenyl-1-(5-mercapto-1,2,4-triazol-1-yl)-butan in Form eines Öles.
¹H-NMR-Spektrum (400 MHz, CDCl₃, TMS):
δ = 2,4 (m, 3H); 2,75 (m, 1H); 4,5 (AB, 2H); 7,0 (d, 2H); 7,2 (d, 2H); 7,4 (m, 3H); 7,55 (m, 2H); 7,8 (s, 1H); 11,7 (1H) ppm.

### Verwendungsbeispiele

### Beispiel A

Pseudocercosporella herpotrichoides-Test; W-Stamm (Weizen) / protektiv
- Lösungsmittel:: 1,0 Gew.-Teile N-Methyl-pyyrolidon
- Emulgator:: 0,6 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen an der Halmbasis mit Sporen des W-Stammes von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

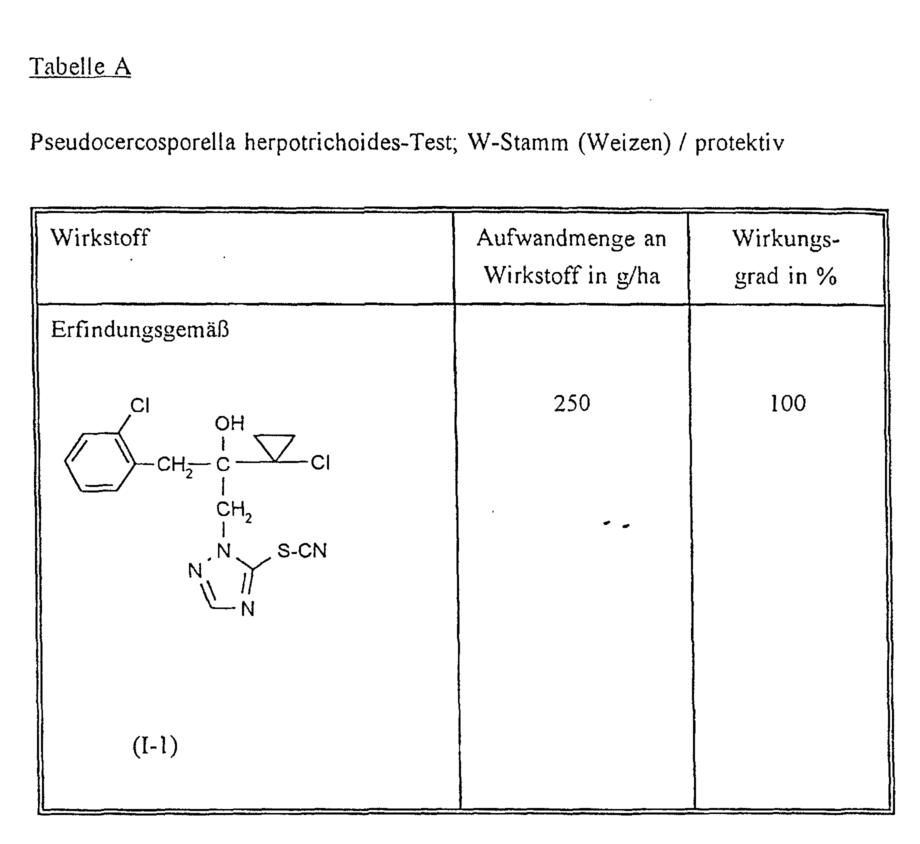

### Beispiel B

### Pyricularia-Test (Reis) / protektiv

- Lösungsmittel:: 12,5 Gew.-Teile Aceton
- Emulgator:: 0,3 Gew.-Teile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit besprüht man junge Reispflanzen mit der Wirkstoffzubereitung taufeucht. 1 Tag nach dem Antrocknen des Spritzbelages werden die Pflanzen mit einer wäßrigen Sporensuspension von Pyricularia oryzae inokuliert. Anschließend werden die Pflanzen werden in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

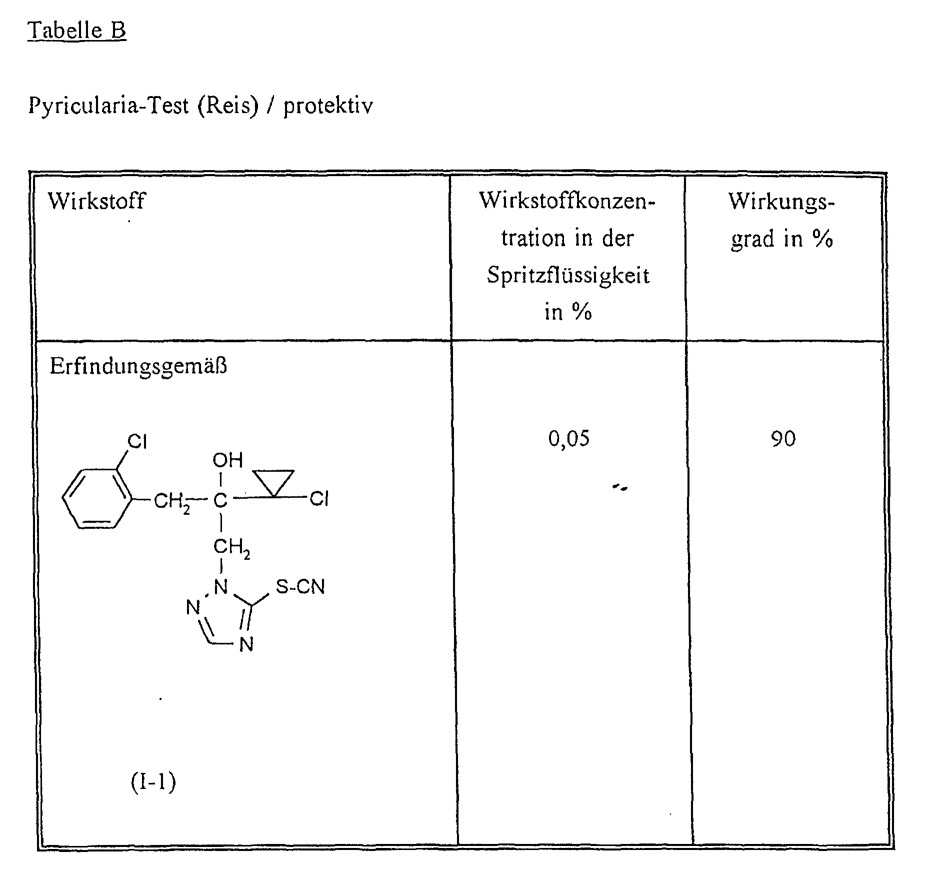

## Patentansprüche

1. Thiocyano-triazolyl-Derivate der Formel 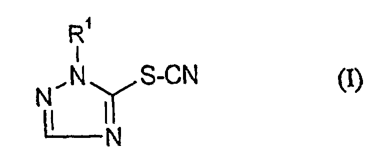 in welcher
R¹ für einen Rest der Formel 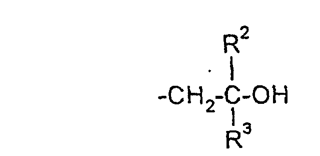 steht, in welcher
R² für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig
oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten, fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano, und
R³ für geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten, fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,
oder
R¹ für einen Rest der Formel 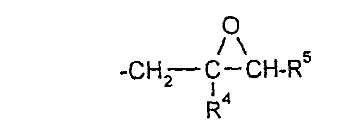 steht, in welcher
R⁴ für Methyl, Ethyl, n-Propyl, Isopropyl, n-Butyl, sek.-Butyl, iso-Butyl, tert.-Butyl, Fluor-tert.-butyl, Difluor-tert.-butyl, gegebenenfalls einfach bis dreifach, gleichartig oder verschieden durch Fluor, Chlor und/oder Brom substituiertes Cycloalkyl mit 3 bis 6 Kohlenstoffatomen, für Naphthyl oder für Phenyl steht, das einfach bis dreifach, gleichartig oder verschieden substituiert sein kann durch Fluor, Chlor, Brom, Nitro, Phenyl, Phenoxy, Methyl, Ethyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio, und
R⁵ für Phenyl steht, das einfach bis dreifach, gleichartig oder verschieden substituiert sein kann durch Fluor, Chlor, Brom, Methyl, Ethyl, Isopropyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio,
oder
R¹ für einen Rest der Formel 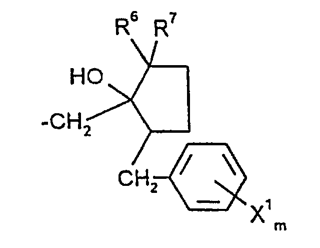 steht, in welcher
R⁶ für Wasserstoff, Methyl, Ethyl, n-Propyl, Isopropyl, n-Butyl, iso-Butyl, sek.-Butyl, tert.-Butyl oder n-Pentyl steht,
R⁷ für Wasserstoff, Methyl, Ethyl, n-Propyl, Isopropyl, n-Butyl, iso-Butyl, sek.-Butyl, tert.-Butyl oder n-Pentyl steht,
X¹ für Fluor, Chlor, Brom, Methyl, Ethyl, n-Propyl, Isopropyl, n-Butyl, iso-Butyl, sek.-Butyl, tert.-Butyl, Methoxy, Ethoxy, Phenyl, Phenoxy, Trichlormethyl, Trifluormethyl, Trifluormethoxy, Difluormethoxy oder Trifluormethylthio steht und
m für die Zahlen 0, 1 oder 2 steht,
wobei X¹ für gleiche oder verschiedene Reste stehen kann, wenn m für 2 steht, oder
R¹ für einen Rest der Formel 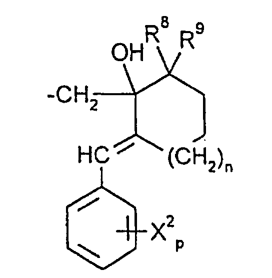 steht, in welcher
R⁸ für Wasserstoff, Methyl, Ethyl, n-Propyl, Isopropyl, n-Butyl, iso-Butyl, sek.-Butyl, tert.-Butyl oder n-Pentyl steht,
R⁹ für Wasserstoff, Methyl, Ethyl, n-Propyl, Isopropyl, n-Butyl, iso-Butyl, sek.-Butyl, tert.-Butyl oder n-Pentyl steht,
X² für Fluor, Chlor, Brom, Cyano, Nitro, Methyl, Ethyl, n-Propyl, Isopropyl, n-Butyl, iso-Butyl, sek.-Butyl, tert.-Butyl, Methoxy, Ethoxy, Trifluormethyl, Trichlormethyl, Difluormethyl, Trichlormethoxy, Trifluormethoxy, Difluormethoxy, Difluorchlormethoxy oder Phenyl steht,
n die Zahlen 0 oder 1 steht und
p für die Zahlen 0, 1 oder 2 steht,
wobei X² für gleiche oder verschiedene Reste stehen kann, wenn p für 2 steht,
oder
R¹ für einen Rest der Formel 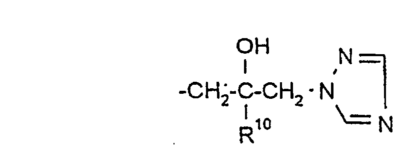 steht, in welcher
R¹⁰ für geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 5 Fluor-, Chlor- und/oder Brom-Atomen, für gegebenenfalls einfach bis dreifach, gleichartig oder verschieden durch Fluor, Chlor, Brom, Methyl und/oder Ethyl substituiertes Cycloalkyl mit 3 bis 6 Kohlenstoffatomen, für Phenyl, Benzyl oder Phenethyl steht, wobei jeder der drei zuletzt genannten Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen, Halogenalkylthio mit 1 bis 4 Kohlenstoffatomen und 1 bis 5 Halogenatomen, gegebenenfalls durch Halogen und/oder Alkyl mit 1 bis 4 Kohlenstoffatomen substituiertes Phenyl oder gegebenenfalls durch Halogen und/oder Alkyl mit 1 bis 4 Kohlenstoffatomen substituiertes Phenoxy,
oder
R¹ für einen Rest der Formel 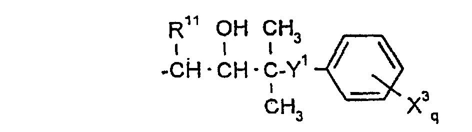 steht, in welcher
R¹¹ für Wasserstoff, geradkettiges oder verzweigtes Alkyl mit 1 bis 12 Kohlenstoffatomen oder für gegebenenfalls einfach bis dreifach, gleichartig oder verschieden durch Halogen und/oder Alkyl mit 1 bis 4 Kohlenstoffatomen substituiertes Cycloalkyl mit 3 bis 7 Kohlenstoffatomen steht,
X³ für Fluor, Chlor, Brom, Methyl, Ethyl, Methoxy, Methylthio, Trichlormethyl, Trifluormethyl, Trifluormethoxy, Difluormethoxy, Trifluormethylthio, Phenyl oder Phenoxy steht,
q für die Zahlen 0, 1, 2 oder 3 steht, wobei X³ für gleiche oder verschiedene Reste steht, wenn q für 2 oder 3 steht, und
Y¹ für ein Sauerstoffatom, eine CH₂-Gruppe oder eine direkte Bindung steht,
oder
R¹ für einen Rest der Formel 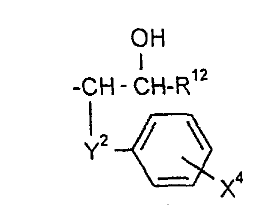 steht, in welcher
R¹² für geradkettiges oder verzweigtes Alkyl mit 1 bis 4 Kohlenstoffatomen, Fluoralkyl mit 1 bis 4 Kohlenstoffatomen und 1 bis 5 Fluoratomen, gegebenenfalls einfach bis dreifach, gleichartig oder verschieden durch Fluor, Chlor, Brom, Methyl und/oder Ethyl substituiertes Cycloalkyl mit 3 bis 6 Kohlenstoffatomen, Cycloalkylalkyl mit 3 bis 6 Kohlenstoffatomen im Cycloalkylteil und 1 bis 3 Kohlenstoffatomen im Alkylteil, gegebenenfalls einfach bis dreifach, gleichartig oder verschieden durch Fluor, Chlor und/oder Brom substituiertes Phenyl oder für gegebenenfalls einfach bis dreifach, gleichartig oder verschieden durch Fluor, Chlor und/oder Brom substituiertes Benzyl steht,
X⁴ für Fluor, Chlor, Brom, Nitro, Methyl, Ethyl, tert.-Butyl, Methoxy, Methylthio, Trichlormethyl, Trifluormethyl, Trifluormethoxy, Difluormethoxy, Trifluormethylthio, gegebenenfalls einfach oder zweifach, gleichartig oder verschieden durch Fluor, Chlor, Brom und/oder Methyl substituiertes Phenyl oder für gegebenenfalls einfach oder zweifach, gleichartig oder verschieden durch Fluor, Chlor, Brom und/oder Methyl substituiertes Phenoxy steht,
r für die Zahlen 0, 1, 2 oder 3 steht, wobei X⁴ für gleiche oder verschiedene Reste steht, wenn r für 2 oder 3 steht und
Y² für ein Sauerstoffatom oder für eine CH₂-Gruppe steht,
oder
R¹ für einen Rest der Formel 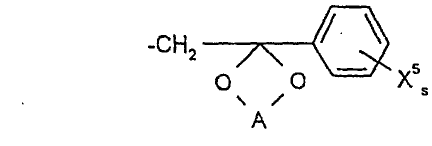 steht, in welcher
A für gegebenenfalls einfach bis dreifach, gleichartig oder verschieden durch Fluor, Chlor, Brom, Methyl, Ethyl, n-Propyl, Isopropyl, n-Butyl, i-Butyl, sek.-Butyl und/oder tert.-Butyl substituiertes Alkandiyl mit 2 oder 3 Kohlenstoffatomen steht,
X⁵ für Fluor, Chlor, Brom, Methyl, Ethyl, Methoxy, Methylthio, Trichlormethyl, Trifluormethyl, Trifluormethoxy, Trifluormethylthio, Difluormethoxy, gegebenenfalls einfach bis dreifach, gleichartig oder verschieden durch Fluor, Chlor und/oder Methyl substituiertes Phenyl und/oder für gegebenenfalls einfach bis dreifach, gleichartig oder verschieden durch Fluor, Chlor und/oder Methyl substituiertes Phenoxy steht und
s für die Zahlen 0, 1, 2 oder 3 steht, wobei X⁵ für gleiche oder verschiedene Reste steht, wenn s für 2 oder 3 steht,
oder
R¹ für einen Rest der Formel 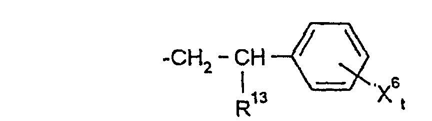 steht, in welcher
R¹³ für geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 5 Fluor-, Chlor- und/oder Bromatomen, Fluoralkoxyalkyl mit 1 bis 3 Kohlenstoffatomen und 1 bis 5 Fluoratomen im Fluoralkoxyteil und 1 bis 3 Kohlenstoffatomen im Alkylteil, gegebenenfalls einfach bis dreifach, gleichartig oder verschieden durch Fluor, Chlor, Brom, Methyl und/oder Ethyl substituiertes Cycloalkyl mit 3 bis 6 Kohlenstoffatomen, Cycloalkylalkyl mit 3 bis 6 Kohlenstoffatomen im Cycloalkylteil und 1 bis 3 Kohlenstoffatomen im Alkylteil, gegebenenfalls einfach bis dreifach, gleichartig oder verschieden durch Fluor, Chlor und/oder Brom substituiertes Phenyl oder für gegebenenfalls einfach bis dreifach, gleichartig oder verschieden durch Fluor, Chlor und/oder Brom substituiertes Phenylalkyl mit 1 oder 2 Kohlenstoffatomen im Alkylteil steht,
X⁶ für Fluor, Chlor, Brom, Methyl, Ethyl, tert.-Butyl, Methoxy, Methylthio, Trichlormethyl, Trifluormethyl, Trifluormethoxy, Difluormethoxy, Trifluormethylthio, gegebenenfalls einfach oder zweifach, gleichartig oder verschieden durch Fluor, Chlor, Brom und/oder Methyl substituiertes Phenyl oder für gegebenenfalls einfach oder zweifach, gleichartig oder verschieden durch Fluor, Chlor, Brom und/oder Methyl substituiertes Phenoxy steht und
t für die Zahlen 0, 1, 2 oder 3 steht, wobei X⁶ für gleiche oder verschiedene Reste steht, wenn t für 2 oder 3 steht,
oder
R¹ für einen Rest der Formel 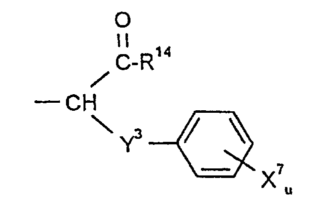 steht, in welcher
R¹⁴ für geradkettiges oder verzweigtes Alkyl mit 1 bis 4 Kohlenstoffatomen, Fluoralkyl mit 1 bis 4 Kohlenstoffatomen und 1 bis 5 Fluoratomen, gegebenenfalls einfach bis dreifach, gleichartig oder verschieden durch Fluor, Chlor, Brom, Methyl und/oder Ethyl substituiertes Cycloalkyl mit 3 bis 6 Kohlenstoffatomen, Cycloalkylalkyl mit 3 bis 6 Kohlenstoffatomen im Cycloalkylteil und 1 bis 3 Kohlenstoffatomen im Alkylteil, gegebenenfalls einfach bis dreifach, gleichartig oder verschieden durch Fluor, Chlor und/oder Brom substituiertes Phenyl oder für gegebenenfalls einfach bis dreifach, gleichartig oder verschieden durch Fluor, Chlor und/oder Brom substituiertes Benzyl steht,
X⁷ für Fluor, Chlor, Brom, Methyl, Ethyl, tert.-Butyl, Methoxy, Methylthio, Trichlormethyl, Trifluormethyl, Trifluormethoxy, Difluormethoxy, Trifluormethylthio, gegebenenfalls einfach oder zweifach, gleichartig oder verschieden durch Fluor, Chlor, Brom und/oder Methyl substituiertes Phenyl oder für gegebenenfalls einfach oder zweifach, gleichartig oder verschieden durch Fluor, Chlor, Brom und/oder Methyl substituiertes Phenoxy steht,
u für die Zahlen 0, 1, 2 oder 3 steht, wobei X⁷ für gleiche oder verschiedene Reste steht, wenn u für 2 oder 3 steht, und
Y³ für ein Sauerstoffatom oder für eine CH₂-Gruppe steht,
oder
R¹ für einen Rest der Formel 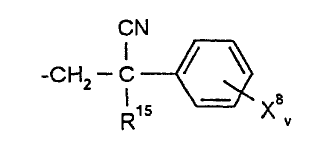 steht, in welcher
R¹⁵ für geradkettiges oder verzweigtes Alkyl mit 1 bis 4 Kohlenstoffatomen, Halogenalkyl mit 1 bis 4 Kohlenstoffatomen und 1 bis 5 Halogenatomen, gegebenenfalls einfach bis dreifach, gleichartig oder verschieden durch Halogen, Alkyl mit 1 bis 4 Kohlenstoffatomen, Halogenalkyl mit 1 oder 2 Kohlenstoffatomen mit 1 bis 5 Halogenatomen, Alkoxy mit 1 bis 4 Kohlenstoffatomen und/oder Halogenalkoxy mit 1 oder 2 Kohlenstoffatomen und 1 bis 5 Halogenatomen substituiertes Phenyl oder für gegebenenfalls einfach bis dreifach, gleichartig oder verschieden durch Halogen, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen und/oder Halogenalkoxy mit 1 oder 2 Kohlenstoffatomen und 1 bis 5 Halogenatomen substituiertes Phenylalkyl mit 1 bis 4 Kohlenstoffatomen im Alkylteil steht,
X⁸ für Fluor, Chlor, Brom, Methyl. Ethyl, tert.-Butyl, Methoxy, Methylthio, Trichlormethyl, Trifluormethyl, Trifluormethoxy, Difluormethoxy, Trifluormethylthio, gegebenenfalls einfach oder zweifach, gleichartig oder verschieden durch Fluor, Chlor, Brom und/oder Methyl substituiertes Phenyl oder für gegebenenfalls einfach oder zweifach, gleichartig oder verschieden durch Fluor, Chlor, Brom und/oder Methyl substituiertes Phenoxy steht und
v für die Zahlen 0, 1, 2 oder 3 steht, wobei X⁸ für gleiche oder verschiedene Reste steht, wenn v für 2 oder 3 steht.

2. Verfahren zur Herstellung von Thiocyano-triazolyl-Derivaten der Formel (I) gemäß Anspruch 1,
**dadurch gekennzeichnet, daß** man Mercapto-triazole der Formel 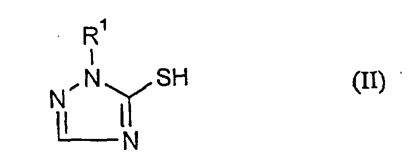 in welcher
R¹ die oben angegebenen Bedeutungen hat,
mit Chlorcyan der Formel
Cl-CN (III)
in Gegenwart eines Verdünnungsmittels umsetzt.

3. Mikrobizide Mittel, **gekennzeichnet durch** einen Gehalt an mindestens einem Thiocyano-triazolyl-Derivat der Formel (I) gemäß Anspruch 1 neben Streckmitteln und / oder oberflächenaktiven Stoffen.

4. Verwendung von Thiocyano-triazolyl-Derivaten der Formel (I) gemäß Anspruch 1 als Mikrobizide im Pflanzenschutz und im Materialschutz.

5. Verfahren zur Bekämpfung von unerwünschten Mikroorganismen im Pflanzenschutz und im Materialschutz, **dadurch gekennzeichnet, daß** man Thiocyano-triazolyl-Derivate der Formel (I) gemäß Anspruch 1 auf die Mikroorganismen und/oder deren Lebensraum ausbringt.

6. Verfahren zur Herstellung von mikrobiziden Mitteln, **dadurch gekennzeichnet, daß** man Thiocyano-triazolyl-Derivate der Formel (I) gemäß Anspruch 1 mit Streckmitteln und/oder oberflächenaktiven Stoffen vermischt.

## Claims

1. Thiocyanatotriazolyl derivatives of the formula 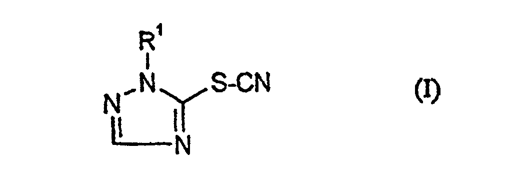 in which
R¹ represents a radical of the formula 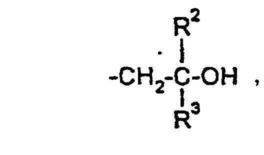 in which
R² represents straight-chain or branched alkyl having 1 to 6 carbon atoms, where these radicals may be mono- to tetrasubstituted by identical or different substituents from the group consisting of halogen, alkoxy having 1 to 4 carbon atoms, alkoximino having 1 to 4 carbon atoms in the alkoxy moiety and cycloalkyl having 3 to 7 carbon atoms,
or
represents cycloalkyl having 3 to 7 carbon atoms, where each of these radicals may be mono- to trisubstituted by identical or different substituents from the group consisting of halogen, cyano and alkyl having 1 to 4 carbon atoms,
or
represents aralkyl having 6 to 10 carbon atoms in the aryl moiety and 1 to 4 carbon atoms in the straight-chain or branched alkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, haloalkyl having 1 or 2 carbon atoms and 1 to 5 identical or different halogen atoms, haloalkoxy having 1 or 2 carbon atoms and 1 to 5 identical or different halogen atoms, haloalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aroxyalkyl having 6 to 10 carbon atoms in the aryl moiety and 1 to 4 carbon atoms in the straight-chain or branched oxyalkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, haloalkyl having 1 or 2 carbon atoms and 1 to 5 identical or different halogen atoms, haloalkoxy having 1 or 2 carbon atoms and 1 to 5 identical or different halogen atoms, haloalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aryl having 6 to 10 carbon atoms, where each of these radicals may be mono- to trisubstituted by identical or different substituents from the group consisting of halogen, alkyl having 1 to 4 carbon atoms, alkoxy having 1 to 4 carbon atoms, alkylthio having 1 to 4 carbon atoms, haloalkyl having 1 or 2 carbon atoms and 1 to 5 identical or different halogen atoms, haloalkoxy having 1 or 2 carbon atoms and 1 to 5 identical or different halogen atoms, haloalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents an optionally benzo-fused five- or six-membered heteroaromatic radical having 1 to 3 heteroatoms, such as nitrogen, sulphur and/or oxygen, where each of these radicals may be mono- to trisubstituted by identical or different substituents from the group consisting of halogen, alkyl having 1 to 4 carbon atoms, hydroxyalkyl having 1 to 4 carbon atoms, hydroxyalkynyl having 3 to 8 carbon atoms, alkoxy having 1 to 2 carbon atoms, alkylthio having 1 or 2 carbon atoms, haloalkyl, haloalkoxy and haloalkylthio having in each case 1 or 2 carbon atoms and 1 to 5 identical or different halogen atoms, such as fluorine or chlorine atoms, formyl, dialkoxymethyl having 1 or 2 carbon atoms in each alkoxy group, acyl having 2 to 4 carbon atoms, alkoxycarbonyl having 1 to 4 carbon atoms in the alkoxy moiety, alkoximinoalkyl having 1 to 4 carbon atoms in the alkoxy moiety and 1 to 3 carbon atoms in the alkyl moiety, nitro and cyano, and
R³ represents straight-chain or branched alkyl having 1 to 6 carbon atoms, where these radicals may be mono- to tetrasubstituted by identical or different substituents from the group consisting of halogen, alkoxy having 1 to 4 carbon atoms, alkoximino having 1 to 4 carbon atoms in the alkoxy moiety and cycloalkyl having 3 to 7 carbon atoms,
or
represents cycloalkyl having 3 to 7 carbon atoms, where each of these radicals may be mono- to trisubstituted by identical or different substituents from the group consisting of halogen, cyano and alkyl having 1 to 4 carbon atoms,
or
represents aralkyl having 6 to 10 carbon atoms in the aryl moiety and 1 to 4 carbon atoms in the straight-chain or branched alkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, haloalkyl having 1 or 2 carbon atoms and 1 to 5 identical or different halogen atoms, haloalkoxy having 1 or 2 carbon atoms and 1 to 5 identical or different halogen atoms, haloalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aroxyalkyl having 6 to 10 carbon atoms in the aryl moiety and 1 to 4 carbon atoms in the straight-chain or branched oxyalkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, haloalkyl having 1 or 2 carbon atoms and 1 to 5 identical or different halogen atoms, haloalkoxy having 1 or 2 carbon atoms and 1 to 5 identical or different halogen atoms, haloalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aryl having 6 to 10 carbon atoms, where each of these radicals may be mono- to trisubstituted by identical or different substituents from the group consisting of halogen, alkyl having 1 to 4 carbon atoms, alkoxy having 1 to 4 carbon atoms, alkylthio having 1 to 4 carbon atoms, haloalkyl having 1 or 2 carbon atoms and 1 to 5 identical or different halogen atoms, haloalkoxy having 1 or 2 carbon atoms and 1 to 5 identical or different halogen atoms, haloalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents an optionally benzo-fused five- or six-membered heteroaromatic radical having 1 to 3 heteroatoms, such as nitrogen, sulphur and/or oxygen, where each of these radicals may be mono- to trisubstituted by identical or different substituents from the group consisting of halogen, alkyl having 1 to 4 carbon atoms, hydroxyalkyl having 1 to 4 carbon atoms, hydroxyalkynyl having 3 to 8 carbon atoms, alkoxy having 1 to 2 carbon atoms, alkylthio having 1 or 2 carbon atoms, haloalkyl, haloalkoxy and haloalkylthio having in each case 1 or 2 carbon atoms and 1 to 5 identical or different halogen atoms, such as fluorine or chlorine atoms, formyl, dialkoxymethyl having 1 or 2 carbon atoms in each alkoxy group, acyl having 2 to 4 carbon atoms, alkoxycarbonyl having 1 to 4 carbon atoms in the alkoxy moiety, alkoximinoalkyl having 1 to 4 carbon atoms in the alkoxy moiety and 1 to 3 carbon atoms in the alkyl moiety, nitro and cyano,
or
R¹ represents a radical of the formula 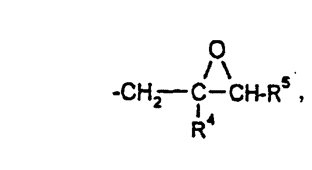 in which
R⁴ represents methyl, ethyl, n-propyl, isopropyl, n-butyl, sec-butyl, isobutyl, tert-butyl, fluoro-tert-butyl, difluoro-tert-butyl, cycloalkyl having 3 to 6 carbon atoms which is optionally mono- to trisubstituted by identical or different substituents from the group consisting of fluorine, chlorine and bromine, represents naphthyl or represents phenyl which may be mono- to trisubstituted by identical or different substituents from the group consisting of fluorine, chlorine, bromine, nitro, phenyl, phenoxy, methyl, ethyl, tert-butyl, methoxy, ethoxy, trifluoromethyl, trichloromethyl, difluoromethyl, difluorochloromethyl, trifluoromethoxy, difluoromethoxy and trifluoromethylthio, and
R⁵ represents phenyl which may be mono- to trisubstituted by identical or different substituents from the group consisting of fluorine, chlorine, bromine, methyl, ethyl, isopropyl, tert-butyl, methoxy, ethoxy, trifluoromethyl, trichloromethyl, difluoromethyl, difluorochloromethyl, trifluoromethoxy, difluoromethoxy and trifluoromethylthio,
or
R¹ represents a radical of the formula 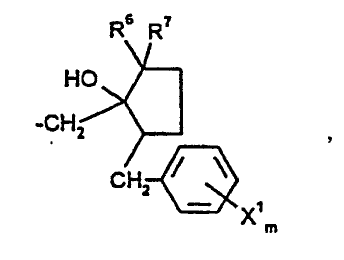 in which
R⁶ represents hydrogen, methyl, ethyl, n-propyl, isopropyl, n-butyl, isobutyl, sec-butyl, tert-butyl or n-pentyl,
R⁷ represents hydrogen, methyl, ethyl, n-propyl, isopropyl, n-butyl, isobutyl, sec-butyl, tert-butyl or n-pentyl,
X¹ represents fluorine, chlorine, bromine, methyl, ethyl, n-propyl, isopropyl, n-butyl, isobutyl, sec-butyl, tent-butyl, methoxy, ethoxy, phenyl, phenoxy, trichloromethyl, trifluoromethyl, trifluoromethoxy, difluoromethoxy or trifluoromethylthio and
m represents the number 0, 1 or 2,
where X¹ may represent identical or different radicals if m is 2,
or
R¹ represents a radical of the formula 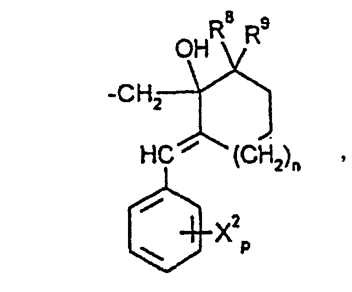 in which
R⁸ represents hydrogen, methyl, ethyl, n-propyl, isopropyl, n-butyl, isobutyl, sec-butyl, tert-butyl or n-pentyl,
R⁹ represents hydrogen, methyl, ethyl, n-propyl, isopropyl, n-butyl, isobutyl, sec-butyl, tert-butyl or n-pentyl,
X² represents fluorine, chlorine, bromine, cyano, nitro, methyl, ethyl, n-propyl, isopropyl, n-butyl, isobutyl, sec-butyl, tert-butyl, methoxy, ethoxy, trifluoromethyl, trichloromethyl, difluoromethyl, trichloromethoxy, trifluoromethoxy, difluoromethoxy, difluorochloromethoxy or phenyl,
n represents the number 0 or 1 and
p represents the number 0, 1 or 2,
where X² may represent identical or different radicals if p is 2,
or
R¹ represents a radical of the formula 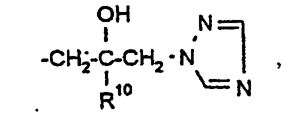 in which
R¹⁰ represents straight-chain or branched alkyl having 1 to 4 carbon atoms, straight-chain or branched haloalkyl having 1 to 4 carbon atoms and 1 to 5 fluorine, chlorine and/or bromine atoms, represents cycloalkyl having 3 to 6 carbon atoms which is optionally mono- to trisubstituted by identical or different substituents from the group consisting of fluorine, chlorine, bromine, methyl and/or ethyl, represents phenyl, benzyl or phenethyl, where each of the three last mentioned radicals may be mono- to trisubstituted by identical or different substituents from the group consisting of halogen, alkyl having 1 to 4 carbon atoms, alkoxy having 1 to 4 carbon atoms, alkylthio having 1 to 4 carbon atoms, haloalkyl having 1 to 4 carbon atoms and 1 to 5 halogen atoms, haloalkoxy having 1 to 4 carbon atoms and 1 to 5 halogen atoms, haloalkylthio having 1 to 4 carbon atoms and 1 to 5 halogen atoms, phenyl which is optionally substituted by halogen and/or alkyl having 1 to 4 carbon atoms or phenoxy which is optionally substituted by halogen and/or alkyl having 1 to 4 carbon atoms,
or
R¹ represents a radical of the formula 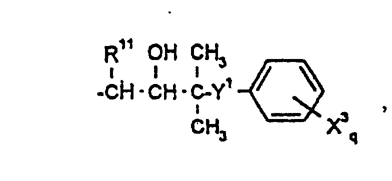 in which
R¹¹ represents hydrogen, straight-chain or branched alkyl having 1 to 12 carbon atoms or represents cycloalkyl having 3 to 7 carbon atoms which is optionally mono- to trisubstituted by identical or different substituents from the group consisting of halogen and alkyl having 1 to 4 carbon atoms,
X³ represents fluorine, chlorine, bromine, methyl, ethyl, methoxy, methylthio, trichloromethyl, trifluoromethyl, trifluoromethoxy, difluoromethoxy, trifluoromethylthio, phenyl or phenoxy,
q represents the number 0, 1, 2 or 3, where X³ represents identical or different radicals if q represents 2 or 3, and
Y¹ represents an oxygen atom, a CH₂ group or a direct bond,
or
R¹ represents a radical of the formula 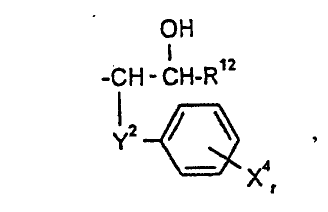 in which
R¹² represents straight-chain or branched alkyl having 1 to 4 carbon atoms, fluoroalkyl having 1 to 4 carbon atoms and 1 to 5 fluorine atoms, cycloalkyl having 3 to 6 carbon atoms which is optionally mono- to trisubtituted by identical or different substituents from the group consisting of fluorine, chlorine, bromine, methyl and ethyl, cycloalkylalkyl having 3 to 6 carbon atoms in the cycloalkyl moiety and 1 to 3 carbon atoms in the alkyl moiety, phenyl which is optionally mono- to trisubstituted by identical or different substituents from the group consisting of fluorine, chlorine and bromine or represents benzyl which is optionally mono- to trisubstituted by identical or different substituents from the group consisting of fluorine, chlorine and bromine,
X⁴ represents fluorine, chlorine, bromine, nitro, methyl, ethyl, tert-butyl, methoxy, methylthio, trichloromethyl, trifluoromethyl, trifluoromethoxy, difluoromethoxy, trifluoromethylthio, phenyl which is optionally mono- or disubstituted by identical or different substituents from the group consisting of fluorine, bromine and methyl or represents phenoxy which is optionally mono- or disubstituted by identical or different substituents from the group consisting of fluorine, chlorine, bromine and methyl,
r represents the number 0, 1, 2 or 3, where X⁴ represents identical or different radicals if r represents 2 or 3 and
Y² represents an oxygen atom or represents a CH₂ group,
or
R¹ represents a radical of the formula 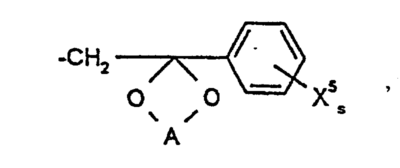 in which
A represents alkanediyl having 2 or 3 carbon atoms which is optionally mono- to trisubstituted by identical or different substituents from the group consisting of fluorine, chlorine, bromine, methyl, ethyl, n-propyl, isopropyl, n-butyl, isobutyl, sec-butyl and tert-butyl,
X⁵ represents fluorine, chlorine, bromine, methyl, ethyl, methoxy, methylthio, trichloromethyl, trifluoromethyl, trifluoromethoxy, trifluoromethylthio, difluoromethoxy, phenyl which is optionally mono- to trisubstituted by identical or different substituents from the group consisting of fluorine, chlorine and methyl or represents phenoxy which is optionally mono- to trisubstituted by identical or different substituents from the group consisting of fluorine, chlorine and methyl and
s represents the number 0, 1, 2 or 3, where X⁵ represents identical or different radicals if s represents 2 or 3,
or
R¹ represents a radical of the formula 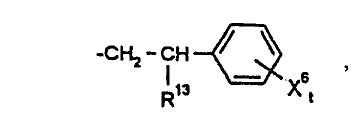 in which
R¹³ represents straight-chain or branched alkyl having 1 to 6 carbon atoms, straight-chain or branched haloalkyl having 1 to 6 carbon atoms and 1 to 5 fluorine, chlorine and/or bromine atoms, fluoroalkoxyalkyl having 1 to 3 carbon atoms and 1 to 5 fluorine atoms in the fluoroalkoxy moiety and 1 to 3 carbon atoms in the alkyl moiety, cycloalkyl having 3 to 6 carbon atoms which is optionally mono- to trisubstituted by identical or different substituents from the group consisting of fluorine, chlorine, bromine, methyl and ethyl, cycloalkylalkyl having 3 to 6 carbon atoms in the cycloalkyl moiety and 1 to 3 carbon atoms in the alkyl moiety, phenyl which is optionally mono- to trisubstituted by identical or different substituents from the group consisting of fluorine, chlorine and bromine or represents phenylalkyl which has 1 or 2 carbon atoms in the alkyl moiety and is optionally mono- to trisubstituted by identical or different substituents from the group consisting of fluorine, chlorine and bromine,
X⁶ represents fluorine, chlorine, bromine, methyl, ethyl, tert-butyl, methoxy, methylthio, trichloromethyl, trifluoromethyl, trifluoromethoxy, difluoromethoxy, trifluoromethylthio, phenyl which is optionally mono- or disubstituted by identical or different substituents from the group consisting of fluorine, chlorine, bromine and methyl or represents phenoxy which is optionally mono- or disubstituted by identical or different substituents from the group consisting of fluorine, chlorine, bromine and methyl and
t represents the number 0, 1, 2 or 3, where X⁶ represents identical or different radicals if t represents 2 or 3,
or
R¹ represents a radical of the formula 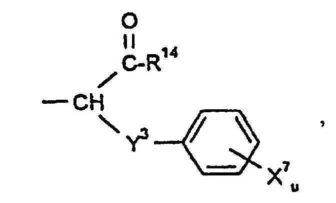 in which
R¹⁴ represents straight-chain or branched alkyl having 1 to 4 carbon atoms, fluoroalkyl having 1 to 4 carbon atoms and 1 to 5 fluorine atoms, cycloalkyl having 3 to 6 carbon atoms which is optionally mono- to trisubstituted by identical or different substituents from the group consisting of fluorine, chlorine, bromine, methyl and ethyl, cycloalkylalkyl having 3 to 6 carbon atoms in the cycloalkyl moiety and 1 to 3 carbon atoms in the alkyl moiety, phenyl which is optionally mono- to trisubstituted by identical or different substituents from the group consisting of fluorine, chlorine and bromine or represents benzyl which is optionally mono- to trisubstituted by identical or different substituents from the group consisting of fluorine, chlorine and bromine,
X⁷ represents fluorine, chlorine, bromine, methyl, ethyl, tert-butyl, methoxy, methylthio, trichloromethyl, trifluoromethyl, trifluoromethoxy, difluoromethoxy, trifluoromethylthio, phenyl which is optionally mono- or disubstituted by identical or different substituents from the group consisting of fluorine, chlorine, bromine and methyl or represents phenoxy which is optionally mono- or disubstituted by identical or different substituents from the group consisting of fluorine, chlorine, bromine and methyl,
u represents the number 0, 1, 2 or 3, where X⁷ represents identical or different radicals if u represents 2 or 3, and
Y³ represents an oxygen atom or represents a CH₂ group,
or
R¹ represents a radical of the formula 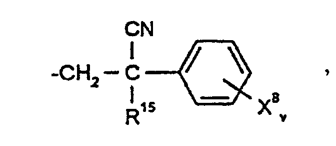 in which
R¹⁵ represents straight-chain or branched alkyl having 1 to 4 carbon atoms, haloalkyl having 1 to 4 carbon atoms and 1 to 5 halogen atoms, phenyl which is optionally mono- to trisubstituted by identical or different substituents from the group consisting of halogen, alkyl having 1 to 4 carbon atoms, haloalkyl having 1 or 2 carbon atoms and 1 to 5 halogen atoms, alkoxy having 1 to 4 carbon atoms and haloalkoxy having 1 or 2 carbon atoms and 1 to 5 halogen atoms or represents phenylalkyl which has 1 to 4 carbon atoms in the alkyl moiety and is optionally mono- to trisubstituted by identical or different substituents from the group consisting of halogen, alkyl having 1 to 4 carbon atoms, haloalkyl having 1 or 2 carbon atoms and 1 to 5 halogen atoms, alkoxy having 1 to 4 carbon atoms and haloalkoxy having 1 or 2 carbon atoms and 1 to 5 halogen atoms,
X⁸ represents fluorine, chlorine, bromine, methyl, ethyl, tert-butyl, methoxy, methylthio, trichloromethyl, trifluoromethyl, trifluoromethoxy, difluoromethoxy, trifluoromethylthio, phenyl which is optionally mono- or disubstituted by identical or different substituents from the group consisting of fluorine, chlorine, bromine and methyl or represents phenoxy which is optionally mono- or disubstituted by identical or different substituents from the group consisting of fluorine, chlorine, bromine and methyl and
v represents the number 0, 1, 2 or 3, where X⁸ represents identical or different radicals if v represents 2 or 3.

2. Process for preparing thiocyanatotriazolyl derivatives of the formula (I) according to Claim 1, **characterized in that** mercaptotriazoles of the formula 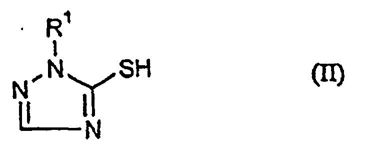 in which
R¹ is as defined above,
are reacted with cyanogen chloride of the formula
Cl-CN (III)
in the presence of a diluent.

3. Microbicidal compositions, **characterized in that** they comprise at least one thiocyanatotriazolyl derivative of the formula (I) according to Claim 1, in addition to extenders and/or surfactants.

4. Use of thiocyanatotriazolyl derivatives of the formula (I) according to Claim 1 as microbicides in crop protection and in the protection of materials.

5. Method for controlling undesirable microorganisms in crop protection and in the protection of materials, **characterized in that** thiocyanatotriazolyl derivatives of the formula (I) according to Claim 1 are applied to the microorganisms and/or their habitat.

6. Process for preparing microbicidal compositions, **characterized in that** thiocyanatotriazolyl derivatives of the formula (I) according to Claim 1 are mixed with extenders and/or surfactants.

## Revendications

1. Dérivés thiocyanotriazolyle de la formule : 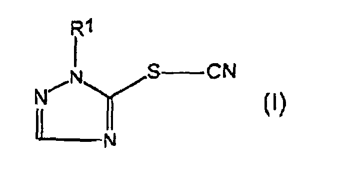 dans laquelle :
R¹ représente un reste de la formule : 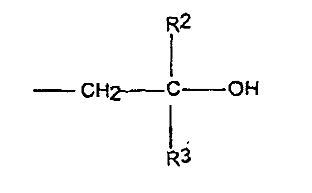
dans laquelle :
R² représente un radical alcoyle linéaire ou ramifié ayant 1 à 6 atomes de carbone, où ces restes peuvent être substitués une à quatre fois, de manière identique ou différente, par un atome d'halogène, un radical alcoxy ayant 1 à 4 atomes de carbone, un radical alcoximino ayant 1 à 4 atomes de carbone dans la partie alcoxy et/ou un radical cycloalcoyle ayant 3 à 7 atomes de carbone, ou
représente un radical cycloalcoyle ayant 3 à 7 atomes de carbone, où chacun de ces restes peut être substitué une à trois fois, de manière identique ou différente, par un atome d'halogène, le radical cyano et/ou un radical alcoyle ayant 1 à 4 atomes de carbone, ou
représente un radical aralcoyle ayant 6 à 10 atomes de carbone dans la partie aryle et 1 à 4 atomes de carbone dans la partie alcoyle linéaire ou ramifiée, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aroxyalcoyle ayant 6 à 10 atomes de carbone dans la partie aryle et 1 à 4 atomes de carbone dans la partie oxyalcoyle linéaire ou ramifiée, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aryle ayant 6 à 10 atomes de carbone, où chacun de ces restes peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un reste hétéroaromatique ayant cinq à six membres, le cas échéant condensé sur un cycle benzénique, ayant 1 à 3 hétéroatomes comme l'atome d'azote, de soufre et/ou d'oxygène, où chacun de ces restes peut être chaque fois substituée une à trois fois, de manière identique ou différente, par un atome d'halogène, un radical alcoyle ayant 1 à 4 atomes de carbone, un radical hydroxyalcoyle ayant 1 à 4 atomes de carbone, un radical hydroxyalcynyle ayant 3 à 8 atomes de carbone, un radical alcoxy ayant 1 ou 2 atomes de carbone, un radical alcoylthio ayant 1 ou 2 atomes de carbone, des radicaux halogénoalcoyle, halogénoalcoxy et halogénoalcoylthio ayant 1 ou 2 atomes de carbone et 1 à 5 atomes d'halogène identiques ou différents, comme l'atome de fluor ou de chlore, le radical formyle, un radical dialcoxyméthyle ayant 1 ou 2 atomes de carbone dans chaque radical alcoxy, un radical acyle ayant 2 à 4 atomes de carbone, un radical alcoxycarbonyle ayant 1 à 4 atomes de carbone dans la partie alcoxy, un radical alcoximinoalcoyle ayant 1 à 4 atomes de carbone dans la partie alcoxy et 1 à 3 atomes de carbone dans la partie alcoyle, le radical nitro et/ou le radical cyano, et
R³ représente un radical alcoyle linéaire ou ramifié ayant 1 à 6 atomes de carbone, où ces restes peuvent être substitués une à quatre fois, de manière identique ou différente, par un atome d'halogène, un radical alcoxy ayant 1 à 4 atomes de carbone, un radical alcoximino ayant 1 à 4 atomes de carbone dans la partie alcoxy et/ou un radical cycloalcoyle ayant 3 à 7 atomes de carbone, ou
représente un radical cycloalcoyle ayant 3 à 7 atomes de carbone, où chacun de ces restes peut être substitué une à trois fois, de manière identique ou différente, par un atome d'halogène, le radical cyano et/ou un radical alcoyle ayant 1 à 4 atomes de carbone, ou
représente un radical aralcoyle ayant 6 à 10 atomes de carbone dans la partie aryle et 1 à 4 atomes de carbone dans la partie alcoyle linéaire ou ramifiée, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoyithio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aroxyalcoyle ayant 6 à 10 atomes de carbone dans la partie aryle et 1 à 4 atomes de carbone dans la partie oxyalcoyle linéaire ou ramifiée, où la partie aryle peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un radical aryle ayant 6 à 10 atomes de carbone, où chacun de ces restes peut être chaque fois substituée une à trois fois, de manière identique ou différente, par un atome d'halogène, un radical alcoyle ayant 1 à 4 atomes de carbone, un radical alcoxy ayant 1 à 4 atomes de carbone, un radical alcoylthio ayant 1 à 4 atomes de carbone, un radical halogénoalcoyle ayant 1 ou 2 atomes de carbone et 1 à 5 atomes d'halogène identiques ou différents, un radical halogénoalcoxy ayant 1 ou 2 atomes de carbone et 1 à 5 atomes d'halogène identiques ou différents, un radical halogénoalcoylthio ayant 1 ou 2 atomes de carbone et 1 à 5 atomes d'halogène identiques ou différents, un radical cycloalcoyle ayant 3 à 7 atomes de carbone, le radical phényle, le radical phénoxy, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano, ou
représente un reste hétéroaromatique ayant cinq à six membres, le cas échéant condensé sur un cycle benzénique, ayant 1 à 3 hétéroatomes comme l'atome d'azote, de soufre et/ou d'oxygène, où chacun de ces restes peut être chaque fois substituée une à trois fois, de manière identique ou différente, par un atome d'halogène, un radical alcoyle ayant 1 à 4 atomes de carbone, un radical hydroxyalcoyle ayant 1 à 4 atomes de carbone, un radical hydroxyalcynyle ayant 3 à 8 atomes de carbone, un radical alcoxy ayant 1 ou 2 atomes de carbone, un radical alcoylthio ayant 1 ou 2 atomes de carbone, des radicaux haloqénoalcoyle, halogénoalcoxy et halogénoalcoylthio ayant 1 ou 2 atomes de carbone et 1 à 5 atomes d'halogène identiques ou différents, comme l'atome de fluor ou de chlore, le radical formyle, un radical dialcoxyméthyle ayant 1 ou 2 atomes de carbone dans chaque radical alcoxy, un radical acyle ayant 2 à 4 atomes de carbone, un radical alcoxycarbonyle ayant 1 à 4 atomes de carbone dans la partie alcoxy, un radical alcoximinoalcoyle ayant 1 à 4 atomes de carbone dans la partie alcoxy et 1 à 4 atomes de carbone dans la partie alcoyle, le radical nitro et/ou le radical cyano,
ou
R¹ représente un reste de la formule : 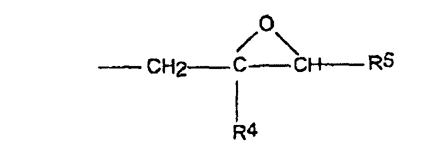 dans laquelle :
R⁴ représente le radical méthyle, éthyle, n-propyle, isopropyle, n-butyle, s-butyle, isobutyle, t-butyle, fluoro-t-butyle, difluoro-t-butyle, un radical cycloalcoyle ayant 3 à 6 atomes de carbone, le cas échéant substitué une à trois fois, de manière identique ou différente par l'atome de fluor, de chlore et/ou de brome, le radical naphtyle ou le radical phényle, qui peut être substitué une à trois fois, de manière identique ou différente, par l'atome de fluor, de chlore, de brome, le radical nitro, phényle, phénoxy, méthyle, éthyle, t-butyle, méthoxy, éthoxy, trifluorométhyle, trichlorométhyle, difluorométhyle, difluorochlorométhyle, trifluorométhoxy, difluorométhoxy et/ou trifluorométhylthio, et
R³ représente le radical phényle, qui peut être substitué une à trois fois, de manière identique ou différente, par l'atome de fluor, de chlore, de brome, le radical méthyle, éthyle, isopropyle, t-butyle, méthoxy, éthoxy, trifluorométhyle, trichlorométhyle, difluorométhyle, difluorochlorométhyle, trifluorométhoxy, difluorométhoxy et/ou trifluorométhylthio, ou
R¹ représente un reste de la formule : 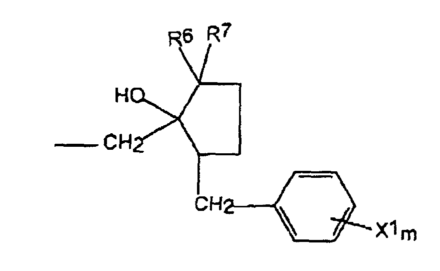
dans laquelle
R⁶ représente l'atome d'hydrogène, le radical méthyle, éthyle, n-propyle, isopropyle, n-butyle, isobutyle, s-butyle, t-butyle ou n-pentyle,
R⁷ représente l'atome d'hydrogène, le radical méthyle, éthyle, n-propyle, isopropyle, n-butyle, isobutyle, s-butyle, t-butyle ou n-pentyle,
X¹ représente l'atome de fluor, de chlore, de brome, le radical méthyle, éthyle, n-propyle, isopropyle, n-butyle, isobutyle, s-butyle, t-butyle, méthoxy, éthoxy, phényle, phénoxy, trichlorométhyle, trifluorométhyle, trifluorométhoxy, difluorométhoxy ou trifluorométhylthio, et
m représente les nombre 0, 1 ou 2,
où X¹ peut représenter des restes identiques ou différents lorsque m est 2, ou
R¹ représente un reste de la formule : 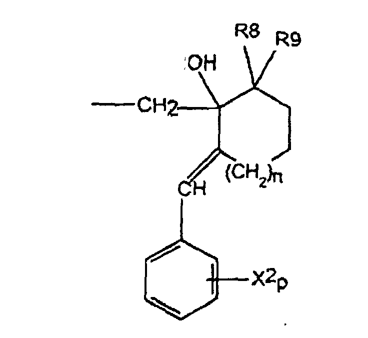 dans laquelle
R⁸ représente l'atome d'hydrogène, le radical méthyle, éthyle, n-propyle, isopropyle, n-butyle, isobutyle, s-butyle, t-butyle ou n-pentyle,
R⁹ représente l'atome d'hydrogène, le radical méthyle, éthyle, n-propyle, isopropyle, n-butyle, isobutyle, s-butyle, t-butyle ou n-pentyle,
X² représente l'atome de fluor, de chlore, de brome, le radical cyano, nitro, méthyle, éthyle, n-propyle, isopropyle, n-butyle, isobutyle, s-butyle, t-butyle, méthoxy, éthoxy, trichlorométhyle, trifluorométhyle, difluorométhyle, trichlorométhoxy, trifluorométhoxy, difluorométhoxy, difluorochlorométhoxy ou phényle,
n représente les nombre 0 ou 1, et
p représente les nombre 0, 1 ou 2,
où X² peut représenter des restes identiques ou différents lorsque p est 2, ou
R¹ représente un reste de la formule : 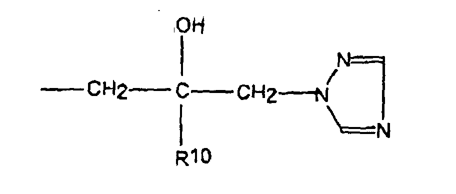 dans laquelle
R¹⁰ représente un radical alcoyle linéaire ou ramifié ayant 1 à 4 atomes de carbone, un radical halogénoalcoyle linéaire ou ramifié ayant 1 à 4 atomes de carbone et 1 à 5 atomes de fluor, de chlore et/ou de brome, un radical cycloalcoyle ayant 3 à 6 atomes de carbone, le cas échéant substitué une à trois fois, de manière identique ou différente par l'atome de fluor, de chlore, de brome, le radical méthyle et/ou éthyle, représente le radical phényle, benzyle ou phényléthyle, où chacun des trois restes cités en dernier lieu peut être substitué une à trois fois, de manière identique ou différente, par un atome d'halogène, un radical alcoyle ayant 1 à 4 atomes de carbone, alcoxy ayant 1 à 4 atomes de carbone, alcoylthio ayant 1 à 4 atomes de carbone, halogénoalcoyle ayant 1 à 4 atomes de carbone et 1 à 5 atomes d'halogène, halogénoalcoxy ayant 1 à 4 atomes de carbone et 1 à 5 atomes d'halogène, halogénoalcoylthio ayant 1 à 4 atomes de carbone et 1 à 5 atomes d'halogène, le radical phényle le cas échéant substitué par un atome d'halogène et/ou radical alcoyle ayant 1 à 4 atomes de carbone, ou le radical phénoxy le cas échéant substitué par un atome d'halogène et/ou radical alcoyle ayant 1 à 4 atomes de carbone, ou
R¹ représente un reste de la formule : 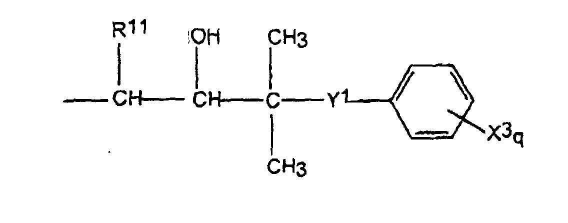 dans laquelle
R¹¹ représente l'atome d'hydrogène, un radical alcoyle linéaire ou ramifié ayant 1 à 12 atomes de carbone ou un radical cycloalcoyle ayant 3 à 7 atomes de carbone, le cas échéant substitué une à trois fois, de manière identique ou différente par un atome d'halogène et/ou un radical alcoyle ayant 1 à 4 atomes de carbone,
X³ représente l'atome de fluor, de chlore, de brome, le radical méthyle, éthyle, méthoxy, méthylthio, trichlorométhyle, trifluorométhyle, trifluorométhoxy, difluorométhoxy, trifluorométhylthio, phényle ou phénoxy,
q représente les nombre 0, 1, 2 ou 3, où X³ peut représenter des restes identiques ou différents lorsque q est 2 ou 3, et
Y¹ représente l'atome d'oxygène, le radical CH₂ ou une liaison directe, ou
R¹ représente un reste de la formule : 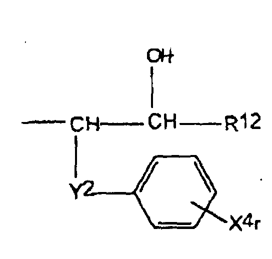 dans laquelle
R¹² représente un radical alcoyle linéaire ou ramifié ayant 1 à 4 atomes de carbone, un radical fluoroalcoyle ayant 1 à 4 atomes de carbone et 1 à 5 atomes de fluor, un radical cycloalcoyle ayant 3 à 6 atomes de carbone, le cas échéant substitué une à trois fois, de manière identique ou différente par l'atome de fluor, de chlore, de brome, le radical méthyle et/ou éthyle, un radical cycloalcoylalcoyle ayant 3 à 6 atomes de carbone dans la partie cycloalcoyle et 1 à 3 atomes de carbone dans la partie alcoyle, le radical phényle le cas échéant substitué une à trois fois, de manière identique ou différente par l'atome de fluor, de chlore et/ou de brome, ou le radical benzyle le cas échéant substitué une à trois fois, de manière identique ou différente par l'atome de fluor, de chlore et/ou de brome,
X⁴ représente l'atome de fluor, de chlore, de brome, le radical nitro, méthyle, éthyle, t-butyle, méthoxy, méthylthio, trichlorométhyle, trifluorométhyle, trifluorométhoxy, difluorométhoxy, trifluorométhylthio, le radical phényle le cas échéant substitué une ou deux fois, de manière identique ou différente par l'atome de fluor, de chlore, de brome et/ou le radical méthyle, ou le radical phénoxy le cas échéant substitué une ou deux fois, de manière identique ou différente par l'atome de fluor, de chlore, de brome et/ou le radical méthyle,
r représente les nombre 0, 1, 2 ou 3, où X⁴ peut représenter des restes identiques ou différents lorsque r est 2 ou 3, et
Y² représente l'atome d'oxygène ou le radical CH₂, ou
R¹ représente un reste de la formule : 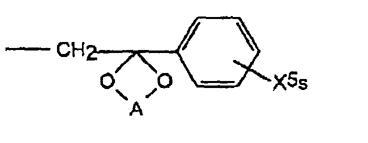 dans laquelle
A représente un radical alcanediyle ayant 2 ou 3 atomes de carbone, le cas échéant substitué une à trois fois, de manière identique ou différente par l'atome de fluor, de chlore, de brome, le radical méthyle, éthyle, n-propyle, isopropyle, n-butyle, i-butyle, s-butyle et/ou t-butyle,
X⁵ représente l'atome de fluor, de chlore, de brome, le radical méthyle, éthyle, méthoxy, méthylthio, trichlorométhyle, trifluorométhyle, trifluorométhoxy, trifluorométhylthio, difluorométhoxy, le radical phényle le cas échéant substitué une à trois fois, de manière identique ou différente par l'atome de fluor, de chlore et/ou le radical méthyle et/ou le radical phénoxy le cas échéant substitué une à trois fois, de manière identique ou différente par l'atome de fluor, de chlore et/ou le radical méthyle,
s représente les nombre 0, 1, 2 ou 3, où X³ peut représenter des restes identiques ou différents lorsque s est 2 ou 3, ou
R¹ représente un reste de la formule : 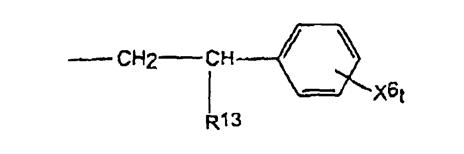 dans laquelle
R¹³ représente un radical alcoyle linéaire ou ramifié ayant 1 à 6 atomes de carbone, un radical halogénoalcoyle ayant 1 à 6 atomes de carbone et 1 à 5 atomes de fluor, de chlore et/ou de brome, un radical fluoroalcoxyalcoyle ayant 1 à 3 atomes de carbone et 1 à 5 atomes de fluor dans la partie fluoroalcoxy et 1 à 3 atomes de carbone dans la partie alcoyle, un radical cycloalcoyle ayant 3 à 6 atomes de carbone, le cas échéant substitué une à trois fois, de manière identique ou différente par l'atome de fluor, de chlore, de brome, le radical méthyle et/ou éthyle, un radical cycloalcoylalcoyle ayant 3 à 6 atomes de carbone dans la partie cycloalcoyle et 1 à 3 atomes de carbone dans la partie alcoyle, le radical phényle le cas échéant substitué une à trois fois, de manière identique ou différente par l'atome de fluor, de chlore et/ou de brome, ou le radical phénylalcoyle ayant 1 ou 2 atomes de carbone dans la partie alcoyle, le cas échéant substitué une à trois fois, de manière identique ou différente par l'atome de fluor, de chlore et/ou de brome,
X⁶ représente l'atome de fluor, de chlore, de brome, le radical méthyle, éthyle, t-butyle, méthoxy, méthylthio, trichlorométhyle, trifluorométhyle, trifluorométhoxy, difluorométhoxy, trifluorométhylthio, le radical phényle le cas échéant substitué une ou deux fois, de manière identique ou différente par l'atome de fluor, de chlore, de brome et/ou le radical méthyle ou le radical phénoxy le cas échéant substitué une ou deux fois, de manière identique ou différente par l'atome de fluor, de chlore, de brome et/ou le radical méthyle,
t représente les nombre 0, 1, 2 ou 3, où X⁶ peut représenter des restes identiques ou différents lorsque t est 2 ou 3, ou
R¹ représente un reste de la formule : 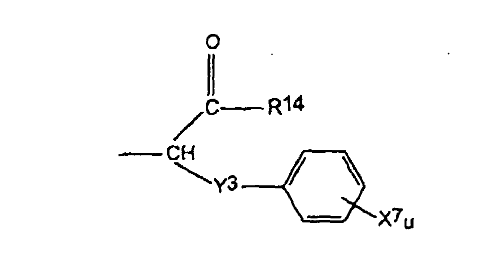 dans laquelle
R¹⁴ représente un radical alcoyle linéaire ou ramifié ayant 1 à 4 atomes de carbone, un radical fluoroalcoyle ayant 1 à 4 atomes de carbone et 1 à 5 atomes de fluor, un radical cycloalcoyle ayant 3 à 6 atomes de carbone, le cas échéant substitué une à trois fois, de manière identique ou différente par l'atome de fluor, de chlore, de brome, le radical méthyle et/ou éthyle, un radical cycloalcoylalcoyle ayant 3 à 6 atomes de carbone dans la partie cycloalcoyle et 1 à 3 atomes de carbone dans la partie alcoyle, le radical phényle le cas échéant substitué une à trois fois, de manière identique ou différente par l'atome de fluor, de chlore et/ou de brome, ou le radical benzyle le cas échéant substitué une à trois fois, de manière identique ou différente par l'atome de fluor, de chlore et/ou de brome,
X⁷ représente l'atome de fluor, de chlore, de brome, le radical méthyle, éthyle, t-butyle, méthoxy, méthylthio, trichlorométhyle, trifluorométhyle, trifluorométhoxy, difluorométhoxy, trifluorométhylthio, le radical phényle le cas échéant substitué une ou deux fois, de manière identique ou différente par l'atome de fluor, de chlore, de brome et/ou le radical méthyle ou le radical phénoxy le cas échéant substitué une ou deux fois, de manière identique ou différente par l'atome de fluor, de chlore, de brome et/ou le radical méthyle,
u représente les nombre 0, 1, 2 ou 3, où X⁷ peut représenter des restes identiques ou différents lorsque u est 2 ou 3, et
Y³ représente l'atome d'oxygène ou le radical CH₂, ou
R¹ représente un reste de la formule : 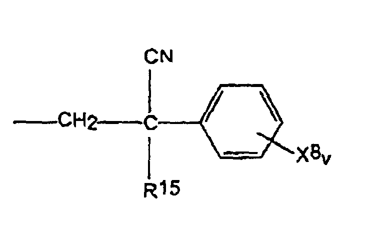 dans laquelle
R¹⁵ représente un radical alcoyle linéaire ou ramifié ayant 1 à 4 atomes de carbone, un radical halogénooalcoyie ayant 1 à 4 atomes de carbone et 1 à 5 atomes d'halogène, le radical phényle le cas échéant substitué une à trois fois, de manière identique ou différente par un atome d'halogène, un radical alcoyle ayant 1 à 4 atomes de carbone, halogénoalcoyle ayant 1 ou 2 atomes de carbone et 1 à 5 atomes d'halogène, alcoxy ayant 1 à 4 atomes de carbone et/ou halogénoalcoxy ayant 1 ou 2 atomes de carbone et 1 à 5 atomes d'halogène, ou le radical phénylalcoyle ayant 1 à 4 atomes de carbone dans la partie alcoyle, le cas échéant substitué une à trois fois, de manière identique ou différente par un atome d'halogène, un radical alcoyle ayant 1 à 4 atomes de carbone, halogénoalcoyle ayant 1 ou 2 atomes de carbone et 1 à 5 atomes d'halogène, alcoxy ayant 1 à 4 atomes de carbone et/ou halogénoalcoxy ayant 1 ou 2 atomes de carbone et 1 à 5 atomes d'halogène,
X⁸ représente l'atome de fluor, de chlore, de brome, le radical méthyle, éthyle, t-butyle, méthoxy, méthylthio, trichlorométhyle, trifluorométhyle, trifluorométhoxy, difluorométhoxy, trifluorométhylthio, le radical phényle le cas échéant substitué une ou deux fois, de manière identique ou différente par l'atome de fluor, de chlore, de brome et/ou le radical méthyle ou le radical phénoxy le cas échéant substitué une ou deux fois, de manière identique ou différente par l'atome de fluor, de chlore, de brome et/ou le radical méthyle,
v représente les nombre 0, 1, 2 ou 3, où X⁸ peut représenter des restes identiques ou différents lorsque v est 2 ou 3.

2. Procédé de préparation de dérivés thiocyanotriazolyle de la formule (I) suivant la revendication 1, **caractérisé en ce que** l'on fait réagir un mercaptotriazole de la formule : 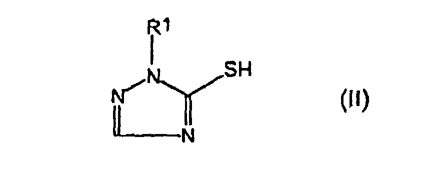 dans laquelle :
R¹ a la signification indiquée ci-dessus,
avec le chlorocyanure de la formule :
Cl-CN (III)
en présence d'un agent de dilution.

3. Agent microbicide, **caractérisé par** une teneur en au moins un dérivé thiocyanotriazolyle de la formule (I) suivant la revendication 1, avec un agent de dilution et/ou un tensioactif.

4. Utilisation de dérivés thiocyanotriazolyle de la formule (I) suivant la revendication 1, comme microbicide dans la protection des végétaux et dans la protection des matériaux.

5. Procédé pour lutter contre les microorganismes non souhaités dans la protection des végétaux et dans la protection des matériaux, **caractérisé en ce que** l'on applique des dérivés thiocyanotriazolyle de la formula (I) suivant la revendication 1, sur les microorganismes et/ou leur biotope.

6. Procédé de préparation d'agents microbicides, **caractérisé en ce que** l'on mélange des dérivés thiocyanotriazolyle de la formule (I) suivant la revendication 1, avec un agent de dilution et/ou un tensioactif.
